# EUROPEAN PATENT SPECIFICATION

(11) **EP 4 356 925 B1**
(45) Date of publication and mention of the grant of the patent: **10.09.2025**
(21) Application number: 23204288.7
(22) Date of filing: 18.10.2023
(51) Int. Cl.: A61K 39/12, A61K 9/19, A61K 39/295, A61K 47/00, A61K 47/02, A61K 47/10, A61K 47/16, A61K 47/18, A61K 47/26, A61K 47/64, A61K 39/00, A61K 39/39

(54) **DENGUE VACCINE FORMULATION**
DENGUE-IMPFSTOFFFORMULIERUNG
FORMULATION DE VACCIN CONTRE LA DENGUE

(30) Priority: 18.10.2022 EP 22202112
(43) Date of publication of application: 24.04.2024
(73) Proprietor: Takeda Vaccines, Inc., Cambridge, MA 02139 (US)
(72) Inventor: KOMMAREDDY, Sushma, Cambridge, 02139 (US); YAZDI, Sara, Cambridge, 02139 (US)
(74) Representative: Maiwald GmbH

(56) References cited:
- WO-A1-2020/051334
- US-A1- 2010 056 500
- US-A1- 2019 184 004
- US-A1- 2020 261 369
- US-A1- 2020 390 877
- US-A1- 2021 187 092
- KANESA-THASAN N ET AL: "Safety and immunogenicity of attenuated dengue virus vaccines (Aventis Pasteur) in human volunteers", VACCINE, ELSEVIER, AMSTERDAM, NL, vol. 19, no. 23-24, 30 April 2001 (2001-04-30), pages 3179 - 3188, XP004234190, ISSN: 0264-410X, DOI: 10.1016/S0264-410X(01)00020-2
- KITCHENER ET AL: "Immunogenicity and safety of two live-attenuated tetravalent dengue vaccine formulations in healthy Australian adults", VACCINE, ELSEVIER, AMSTERDAM, NL, vol. 24, no. 9, 27 February 2006 (2006-02-27), pages 1238 - 1241, XP005289614, ISSN: 0264-410X, DOI: 10.1016/J.VACCINE.2005.09.029
- DAYAN GUSTAVO H ET AL: "Safety and immunogenicity of three tetravalent dengue vaccine formulations in healthy adults in the USA", VACCINE, vol. 31, no. 44, 7 September 2013 (2013-09-07), pages 5047 - 5054, XP028738110, ISSN: 0264-410X, DOI: 10.1016/J.VACCINE.2013.08.088

## Description

### FIELD OF THE INVENTION

The present invention relates to a dengue vaccine formulation, unit doses of the dengue vaccine formulation, uses of the dengue vaccine formulation in vials, kits and in methods of preventing dengue disease, and methods for preparing the dengue vaccine formulation.

### BACKGROUND OF THE INVENTION

Dengue disease is the fastest-spreading mosquito-borne viral disease caused by infection with a dengue virus with an estimated 390 million cases and 500,000 hospitalizations per year globally. With limited options for prevention, dengue viruses are estimated to cause around 20,000 deaths globally each year. Dengue virus infections can lead to debilitating and painful symptoms, including a sudden high fever, headaches, joint and muscle pain, nausea, vomiting and skin rashes. To date, four serotypes of dengue virus have been identified: dengue-1 (DENV-1), dengue-2 (DENV-2), dengue-3 (DENV-3) and dengue-4 (DENV-4). Dengue virus serotypes 1-4 can also cause dengue hemorrhagic fever (DHF) and dengue shock syndrome (DSS). In the most severe cases, DHF and DSS can be life threatening. All four dengue virus serotypes are endemic throughout the tropical regions of the world and constitute the most significant mosquito-borne viral threat to humans there. Dengue viruses are transmitted to humans primarily by Aedes aegypti mosquitoes, but also by Aedes albopictus mosquitoes. Infection with one dengue virus serotype results in life-long protection from re-infection by that serotype, but does not prevent secondary infection by one of the other three dengue virus serotypes. In fact, previous infection with one dengue virus serotype may lead to an increased risk of severe disease (DHF/DSS) upon secondary infection with a different serotype.

Vaccines for protection against viral infections have been effectively used to reduce the incidence of human disease. One of the most successful technologies for viral vaccines is to immunize animals or humans with a weakened or attenuated virus strain (a "live attenuated virus"). Due to limited replication after immunization, the attenuated virus strain does not cause disease. However, the limited viral replication is sufficient to express the full repertoire of viral antigens and can generate potent and long-lasting immune responses to the virus. Thus, upon subsequent exposure to a pathogenic virus strain, the immunized individual is protected from the disease. These live attenuated viral vaccines are among the most successful vaccines used in public health.

In order for live attenuated viral vaccines to be effective, they must be capable of replicating after immunization, and the viruses themselves must be protected from degradation during preparation as well as transport prior to administration to a subject, in order to ensure for example, that the correct dosage concentration is delivered to the subject. Some vaccines are sensitive to temperature extremes; either excessive heat or accidental freezing can inactivate the vaccine. Maintaining this "cold chain" throughout distribution is particularly difficult in the developing world. Therefore, there remains a need for improving the stability of live attenuated viruses in vaccine formulations, such as in dengue vaccine formulations in order to improve manufacturing of vaccines, as well as, transport and delivery of the formulations.

Stable dengue vaccine formulations are described in US 2019/0184004 A1, US 2020/0390877 A1, and WO 2020/051334 A1. The primary driver for the development of a new excipient composition is to further improve the virus stability, which is also expected to reduce the amount of virus needed to ensure the required end of shelf life (ESL) potency for the vaccine formulation.

### OBJECTS AND SUMMARY

It is an object of the present invention to provide an improved dengue vaccine formulation for reducing degradation of live attenuated dengue virus (e.g. serotypes 1 to 4) during manufacturing, storage and transport of the dengue vaccine formulation.

It is an object of the present invention to provide an improved dengue vaccine formulation for reducing consumption of live attenuated dengue virus (e.g. serotypes 1 to 4) during the manufacturing of a dengue vaccine formulation.

It is an object of the present invention to provide an improved dengue vaccine formulation for reducing degradation of live attenuated dengue virus (e.g. serotypes 1 to 4) during lyophilization.

It is an object of the present invention to provide an improved dengue vaccine formulation for reducing the drying time.

It is an object of the present invention to provide an improved dengue vaccine formulation for reducing degradation of live attenuated dengue virus (e.g. serotypes 1 to 4) post lyophilization.

It is an object of the present invention to provide an improved dengue vaccine formulation for improving local physiological compatibility of the dengue vaccine formulation after reconstitution.

It is an object of the present invention to provide an improved dengue vaccine formulation which maintains low foaming characteristics upon reconstitution of the formulation.

It is an object of the present invention to provide a lyophilized dengue vaccine formulation that will retain its potential immunizing capacity during preparation and for the duration required for a pharmaceutically acceptable period of time and can be dissolved in diluent with minimum foaming.

A further object of the present invention is to provide an improved method of preparing a dengue vaccine formulation for reducing the consumption of live attenuated dengue virus.

Another object of the present invention to provide an improved method of preparing a dengue vaccine formulation for reducing the lyophilization cycle length.

Another object of the present invention to provide an improved method of preparing a dengue vaccine formulation for improving the product capacity.

A further object of the present invention is to provide a safe and effective vaccine against all serotypes of dengue virus for dengue-endemic and dengue non-endemic populations and for a broad range of ages and independent of previous exposure to dengue virus and corresponding seropositive or seronegative status before vaccination.

The invention is defined by the appended claims. Any part of the description which does not fall under the scope of the appended claims is for illustrative purposes only. In particular, any subject matter referred to as "disclosure" which is not falling under the scope of the appended claims does not form part of the invention.

Any reference in the description to methods of preventing dengue disease in a subject refers to the dengue vaccine formulation of the present invention for use in a method of preventing dengue disease in a subject.

The present disclosure is directed in part to a dengue vaccine formulation comprising:
a tetravalent dengue virus composition comprising a live attenuated dengue virus serotype 1, a live attenuated dengue virus serotype 2, a live attenuated dengue virus serotype 3, and a live attenuated dengue virus serotype 4 (e.g. "TAK-003") ;
at least one non-reducing disaccharide (e.g. trehalose and/or sucrose);
at least one poloxamer (e.g. poloxamer 407);
urea;
at least one amino acid having a positively charged side chain at neutral pH (e.g. arginine, lysine and/or histidine);
tromethamine; and
human serum albumin.

The present invention is directed in part to a dengue vaccine formulation comprising:
A. a tetravalent dengue virus composition comprising:
   (i) a live attenuated dengue virus serotype 1 (e.g. a chimeric dengue serotype 2/1 strain),
   (ii) a live attenuated dengue virus serotype 2 (e.g. a dengue serotype 2 strain),
   (iii) a live attenuated dengue virus serotype 3 (e.g. a chimeric dengue serotype 2/3 strain), and
   (iv) a live attenuated dengue virus serotype 4 (e.g. a chimeric dengue serotype 2/4 strain); and
B. an excipient composition comprising:
   a. trehalose,
   b. poloxamer,
   c. urea,
   d. arginine hydrochloride,
   e. tromethamine, and
   f. human serum albumin,
   wherein the dengue vaccine formulation comprises:
   a. the trehalose at an amount of 20 to 34 mg/dose,
   b. the poloxamer at an amount of 0.1 to 3.0 mg/dose,
   c. the urea at an amount of 0.1 to 3.0 mg/dose,
   d. the arginine hydrochloride at an amount of 0.1 to 2.0 mg/dose,
   e. the tromethamine at an amount of 0.05 to 1.0 mg/dose, and
   f. the human serum albumin at an amount of 0.05 to 0.5 mg/dose.

It was found that, in particular, the combination of trehalose, poloxamer, urea, arginine, tromethamine, and human serum albumin as described in the previous paragraph provides superior stability of the tetravalent dengue virus composition (e.g. "TAK-003"). In particular, the excipient composition as disclosed herein helps in improving the stability of the dengue vaccine formulation after drying, for example during storage and transport of the lyophilized dengue vaccine formulation as described herein. The excipient composition as disclosed herein also helps in improving the stability of the dengue vaccine formulation during manufacturing, for example during freezing when the dengue vaccine formulation as described herein is subject to a lyophilization process. In certain such embodiments, the excipient composition helps in avoiding pH changes of the dengue vaccine formulation during freezing and thereby reduces degradation of live attenuated dengue virus. In certain embodiments, the excipient composition helps in reducing the time for lyophilizing the dengue vaccine formulation as described herein. In certain embodiments, the excipient composition as disclosed herein helps in maintaining low foaming characteristics of the dengue vaccine formulation as described herein during reconstitution and withdrawal.

The present invention is in particular directed to such a dengue vaccine formulation comprising:
A. a tetravalent dengue virus composition comprising:
   (i) a live attenuated dengue virus serotype 1 (e.g. a chimeric dengue serotype 2/1 strain),
   (ii) a live attenuated dengue virus serotype 2 (e.g. a dengue serotype 2 strain),
   (iii) a live attenuated dengue virus serotype 3 (e.g. a chimeric dengue serotype 2/3 strain), and
   (iv) a live attenuated dengue virus serotype 4 (e.g. a chimeric dengue serotype 2/4 strain); and
B. an excipient composition comprising based on the dry weight of the vaccine formulation:
   a. about 65 to 95 % w/w trehalose, such as about 87.0 % w/w trehalose,
   b. about 0.05 to 5.0 % w/w poloxamer, such as about 2.9 % w/w poloxamer,
   c. about 0.1 to 6.0 % w/w urea, such as about 2.9 % w/w urea,
   d. about 0.1 to 5.0 % w/w arginine hydrochloride, such as about 2.4 % w/w arginine hydrochloride,
   e. about 0.05 to 5.0 % w/w tromethamine, such as about 1.2 % w/w tromethamine, and
   f. about 0.05 to 3.0 % w/w human serum albumin, such as about 0.96 % w/w human serum albumin,
   wherein the dengue vaccine formulation comprises:
   a. the trehalose at an amount of 20 to 34 mg/dose,
   b. the poloxamer at an amount of 0.1 to 3.0 mg/dose,
   c. the urea at an amount of 0.1 to 3.0 mg/dose,
   d. the arginine hydrochloride at an amount of 0.1 to 2.0 mg/dose,
   e. the tromethamine at an amount of 0.05 to 1.0 mg/dose, and
   f. the human serum albumin at an amount of 0.05 to 0.5 mg/dose.

The present invention is in particular directed to such a dengue vaccine formulation comprising:
A. a tetravalent dengue virus composition comprising:
   (i) live attenuated dengue virus serotype 1 (e.g. a chimeric dengue serotype 2/1 strain),
   (ii) live attenuated dengue virus serotype 2 (e.g. a dengue serotype 2 strain),
   (iii) live attenuated dengue virus serotype 3 (e.g. a chimeric dengue serotype 2/3 strain), and
   (iv) live attenuated dengue virus serotype 4 (e.g. a chimeric dengue serotype 2/4 strain); and
B. an excipient composition comprising:
   a. trehalose,
   b. poloxamer,
   c. urea,
   d. arginine hydrochloride,
   e. tromethamine, and
   f. human serum albumin;

wherein the ratio of the amount of arginine hydrochloride to the amount of human serum albumin contained in the dengue vaccine formulation is about 1:1 to 4:1;
wherein chloride salts are contained in the dengue vaccine formulation at an amount of less than 4.0 % w/w based on the dry weight of the vaccine formulation;
wherein phosphate salts are contained in the dengue vaccine formulation at an amount of less than 1.0 % w/w based on the dry weight of the vaccine formulation,
wherein the dengue vaccine formulation comprises:
   a. the trehalose at an amount of 20 to 34 mg/dose,
   b. the poloxamer at an amount of 0.1 to 3.0 mg/dose,
   c. the urea at an amount of 0.1 to 3.0 mg/dose,
   d. the arginine hydrochloride at an amount of 0.1 to 2.0 mg/dose,
   e. the tromethamine at an amount of 0.05 to 1.0 mg/dose, and
   f. the human serum albumin at an amount of 0.05 to 0.5 mg/dose.

The present invention is in particular directed to such a dengue vaccine formulation comprising:
A. a tetravalent dengue virus composition comprising:
   (i) live attenuated dengue virus serotype 1,
   (ii) live attenuated dengue virus serotype 2,
   (iii) live attenuated dengue virus serotype 3, and
   (iv) live attenuated dengue virus serotype 4; and
B. an excipient composition comprising:
   a. trehalose,
   b. poloxamer,
   c. urea,
   d. arginine hydrochloride,
   e. tromethamine, and
   f. human serum albumin;

wherein the dengue serotype 1 is a chimeric dengue serotype 2/1 strain, the dengue serotype 2 is a non-chimeric dengue serotype 2 strain, the dengue serotype 3 is a chimeric dengue serotype 2/3 strain and the dengue serotype 4 is a chimeric dengue serotype 2/4 strain; wherein the dengue serotype 2 strain is derived from the live attenuated DEN-2 PDK-53 virus strain and the three chimeric dengue strains are derived from the dengue serotype 2 strain by replacing the structural proteins prM and E from the dengue serotype 2 strain with the corresponding structural proteins from the other dengue serotypes, resulting in the four live attenuated dengue virus strains TDV-1, TDV-2, TDV-3 and TDV-4 (e.g. "TAK-003"),
wherein the dengue vaccine formulation comprises:
   a. the trehalose at an amount of 20 to 34 mg/dose,
   b. the poloxamer at an amount of 0.1 to 3.0 mg/dose,
   c. the urea at an amount of 0.1 to 3.0 mg/dose,
   d. the arginine hydrochloride at an amount of 0.1 to 2.0 mg/dose,
   e. the tromethamine at an amount of 0.05 to 1.0 mg/dose, and
   f. the human serum albumin at an amount of 0.05 to 0.5 mg/dose.

The present invention is in particular directed to such a dengue vaccine formulation comprising:
A. a tetravalent dengue virus composition comprising:
   (i) TDV-1,
   (ii) TDV-2,
   (iii) TDV-3, and
   (iv) TDV-4; and
B. an excipient composition comprising:
   a. trehalose,
   b. poloxamer,
   c. urea,
   d. arginine hydrochloride,
   e. tromethamine, and
   f. human serum albumin,
   wherein the dengue vaccine formulation comprises:
   a. the trehalose at an amount of 20 to 34 mg/dose,
   b. the poloxamer at an amount of 0.1 to 3.0 mg/dose,
   c. the urea at an amount of 0.1 to 3.0 mg/dose,
   d. the arginine hydrochloride at an amount of 0.1 to 2.0 mg/dose,
   e. the tromethamine at an amount of 0.05 to 1.0 mg/dose, and
   f. the human serum albumin at an amount of 0.05 to 0.5 mg/dose.

The present invention is in particular directed to such a dengue vaccine formulation comprising:
A. a tetravalent dengue virus composition comprising:
   (i) live attenuated dengue virus serotype 1,
   (ii) live attenuated dengue virus serotype 2,
   (iii) live attenuated dengue virus serotype 3, and
   (iv) live attenuated dengue virus serotype 4; and
B. an excipient composition comprising:
   a. trehalose,
   b. poloxamer,
   c. urea,
   d. arginine hydrochloride,
   e. tromethamine, and
   f. human serum albumin;
   wherein the dengue serotype 1 is a chimeric dengue serotype 2/1 strain, the dengue serotype 2 is a non-chimeric dengue serotype 2 strain, the dengue serotype 3 is a chimeric dengue serotype 2/3 strain and the dengue serotype 4 is a chimeric dengue serotype 2/4 strain; wherein the dengue serotype 2 strain is derived from the wild type virus strain DEN-2 16681 and differs in at least three nucleotides from the wild type as follows:
   a) 5'-noncoding region (NCR)-57 (nt-57)
   b) NS1-53 Gly-to-Asp (nt-2579)
   c) NS3-250 Glu-to-Val (nt-5270);
   wherein the three chimeric dengue strains are derived from the serotype 2 strain by replacing the structural proteins prM and E from serotype 2 strain with the corresponding structural proteins from the other dengue serotypes, resulting in the following chimeric dengue strains:
   - a DENV-2/1 chimera,
   - a DENV-2/3 chimera, and
   - a DENV-2/4 chimera,
   wherein the dengue vaccine formulation comprises:
   a. the trehalose at an amount of 20 to 34 mg/dose,
   b. the poloxamer at an amount of 0.1 to 3.0 mg/dose,
   c. the urea at an amount of 0.1 to 3.0 mg/dose,
   d. the arginine hydrochloride at an amount of 0.1 to 2.0 mg/dose,
   e. the tromethamine at an amount of 0.05 to 1.0 mg/dose, and
   f. the human serum albumin at an amount of 0.05 to 0.5 mg/dose.

The present invention is in particular directed to such a dengue vaccine formulation comprising:
A. a tetravalent dengue virus composition comprising four live attenuated dengue virus serotypes:
   (i) a chimeric dengue serotype 2/1 strain,
   (ii) a dengue serotype 2 strain,
   (iii) a chimeric dengue serotype 2/3 strain, and
   (iv) a chimeric dengue serotype 2/4 strain;
   wherein the dengue serotype 2 strain is derived from the wild type virus strain DEN-2 16681 and differs in at least three nucleotides from the wild type as follows:
   a) 5'-noncoding region (NCR)-57 (nt-57)
   b) NS1-53 Gly-to-Asp (nt-2579)
   c) NS3-250 Glu-to-Val (nt-5270);
   wherein the three chimeric dengue strains are derived from the serotype 2 strain by replacing the structural proteins prM and E from serotype 2 strain with the corresponding structural proteins from the other dengue serotypes, resulting in the following chimeric dengue strains:
   - a DENV-2/1 chimera,
   - a DENV-2/3 chimera, and
   - a DENV-2/4 chimera;
   and
B. an excipient composition comprising:
   a. trehalose,
   b. poloxamer,
   c. urea,
   d. arginine hydrochloride,
   e. tromethamine, and
   f. human serum albumin;

   wherein the human serum albumin is contained in the dengue vaccine formulation at an amount of about 0.1 to 1.0 % w/w based on the dry weight of the vaccine formulation, wherein the ratio of the amount of arginine hydrochloride to the amount of human serum albumin contained in the dengue vaccine formulation is about 1:1 to 4:1,
   wherein the dengue vaccine formulation comprises:
      a. the trehalose at an amount of 20 to 34 mg/dose,
      b. the poloxamer at an amount of 0.1 to 3.0 mg/dose,
      c. the urea at an amount of 0.1 to 3.0 mg/dose,
      d. the arginine hydrochloride at an amount of 0.1 to 2.0 mg/dose,
      e. the tromethamine at an amount of 0.05 to 1.0 mg/dose, and
      f. the human serum albumin at an amount of 0.05 to 0.5 mg/dose.

The present invention is in particular directed to such a dengue vaccine formulation (e.g. a lyophilized dengue vaccine formulation) comprising:
A. a tetravalent dengue virus composition comprising:
   (i) live attenuated dengue virus serotype 1,
   (ii) live attenuated dengue virus serotype 2,
   (iii) live attenuated dengue virus serotype 3, and
   (iv) live attenuated dengue virus serotype 4; and
B. an excipient composition comprising based on the dry weight of the vaccine formulation:
   a. about 65 to 95 % w/w trehalose,
   b. about 0.05 to 5.0 % w/w poloxamer,
   c. about 0.1 to 6.0 % w/w urea,
   d. about 0.1 to 5.0 % w/w arginine hydrochloride,
   e. about 0.05 to 5.0 % w/w tromethamine,
   f. about 0.05 to 3.0 % w/w human serum albumin
   g. about 0.05 to 3.0 % w/w chloride salts, and
   h. about 0.05 to 0.5 % w/w phosphate salts;

   wherein the dengue serotype 1 is a chimeric dengue serotype 2/1 strain, the dengue serotype 2 is a non-chimeric dengue serotype 2 strain, the dengue serotype 3 is a chimeric dengue serotype 2/3 strain and the dengue serotype 4 is a chimeric dengue serotype 2/4 strain;
   wherein the dengue serotype 2 strain is derived from the wild type virus strain DEN-2 16681 and differs in at least three nucleotides from the wild type as follows:
      a) 5'-noncoding region (NCR)-57 (nt-57)
      b) NS1-53 Gly-to-Asp (nt-2579)
      c) NS3-250 Glu-to-Val (nt-5270);
   wherein the three chimeric dengue strains are derived from the serotype 2 strain by replacing the structural proteins prM and E from serotype 2 strain with the corresponding structural proteins from the other dengue serotypes, resulting in the following chimeric dengue strains:
      - a DENV-2/1 chimera,
      - a DENV-2/3 chimera, and
      - a DENV-2/4 chimera,
   wherein the dengue vaccine formulation comprises:
      a. the trehalose at an amount of 20 to 34 mg/dose,
      b. the poloxamer at an amount of 0.1 to 3.0 mg/dose,
      c. the urea at an amount of 0.1 to 3.0 mg/dose,
      d. the arginine hydrochloride at an amount of 0.1 to 2.0 mg/dose,
      e. the tromethamine at an amount of 0.05 to 1.0 mg/dose, and
      f. the human serum albumin at an amount of 0.05 to 0.5 mg/dose.

The present invention is in particular directed to such a dengue vaccine formulation in dry form (e.g. a lyophilized dengue vaccine formulation) comprising:
A. a tetravalent dengue virus composition comprising four live attenuated dengue virus serotypes:
   (i) a chimeric dengue serotype 2/1 strain,
   (ii) a dengue serotype 2 strain,
   (iii) a chimeric dengue serotype 2/3 strain, and
   (iv) a chimeric dengue serotype 2/4 strain;
   wherein the dengue serotype 2 strain is derived from the wild type virus strain DEN-2 16681 and differs in at least three nucleotides from the wild type as follows:
   a) 5'-noncoding region (NCR)-57 (nt-57)
   b) NS1-53 Gly-to-Asp (nt-2579)
   c) NS3-250 Glu-to-Val (nt-5270);
   wherein the three chimeric dengue strains are derived from the serotype 2 strain by replacing the structural proteins prM and E from serotype 2 strain with the corresponding structural proteins from the other dengue serotypes, resulting in the following chimeric dengue strains:
   - a DENV-2/1 chimera,
   - a DENV-2/3 chimera, and
   - a DENV-2/4 chimera; and
B. an excipient composition comprising:
   a. trehalose,
   b. poloxamer,
   c. urea,
   d. arginine hydrochloride,
   e. tromethamine, and
   f. human serum albumin;
   wherein a unit dose of the dengue vaccine formulation upon reconstitution with 0.5 mL of a pharmaceutically acceptable aqueous diluent comprises:
   (i) the chimeric dengue serotype 2/1 strain in a concentration of at least 3.3 log10 pfu/0.5 mL,
   (ii) the dengue serotype 2 strain in a concentration of at least 2.7 log10 pfu/0.5 mL,
   (iii) the chimeric dengue serotype 2/3 strain in a concentration of at least 4.0 log10 pfu/0.5 mL,
   (iv) the chimeric dengue serotype 2/4 strain in a concentration of at least 4.5 log10 pfu/0.5 mL,
      a. the trehalose in a concentration of from 40 to 80 g/L, such as about 55 g/L,
      b. the poloxamer in a concentration of about 0.1 to 5.0 g/L, such as about 1.65 g/L,
      c. the urea in a concentration of about 0.1 to 6.0 g/L, such as about 1.65 g/L,
      d. the arginine hydrochloride in a concentration of about 0.5 to 5.0 g/L, such as about 1.4 g/L,
      e. the tromethamine in a concentration of about 0.1 to 1.5 g/L, such as about 0.7 g/L, and
      f. the human serum albumin in a concentration of from 0.1 to less than 1 g/L, such as about 0.55 g/L,
      wherein the dengue vaccine formulation comprises:
      a. the trehalose at an amount of 20 to 34 mg/dose,
      b. the poloxamer at an amount of 0.1 to 3.0 mg/dose,
      c. the urea at an amount of 0.1 to 3.0 mg/dose,
      d. the arginine hydrochloride at an amount of 0.1 to 2.0 mg/dose,
      e. the tromethamine at an amount of 0.05 to 1.0 mg/dose, and
      f. the human serum albumin at an amount of 0.05 to 0.5 mg/dose.

The present invention is in particular directed to such a dengue vaccine formulation in dry form (e.g. a lyophilized dengue vaccine formulation) comprising:
A. a tetravalent dengue virus composition comprising four live attenuated dengue virus serotypes:
   (i) a chimeric dengue serotype 2/1 strain,
   (ii) a dengue serotype 2 strain,
   (iii) a chimeric dengue serotype 2/3 strain, and
   (iv) a chimeric dengue serotype 2/4 strain;
   wherein the dengue serotype 2 strain is derived from the wild type virus strain DEN-2 16681 and differs in at least three nucleotides from the wild type as follows:
   a) 5'-noncoding region (NCR)-57 (nt-57)
   b) NS1-53 Gly-to-Asp (nt-2579)
   c) NS3-250 Glu-to-Val (nt-5270);
   wherein the three chimeric dengue strains are derived from the serotype 2 strain by replacing the structural proteins prM and E from serotype 2 strain with the corresponding structural proteins from the other dengue serotypes, resulting in the following chimeric dengue strains:
   - a DENV-2/1 chimera,
   - a DENV-2/3 chimera, and
   - a DENV-2/4 chimera; and
B. an excipient composition comprising:
   a. trehalose,
   b. poloxamer,
   c. urea,
   d. arginine hydrochloride,
   e. tromethamine, and
   f. human serum albumin;
   wherein a unit dose of the dengue vaccine formulation upon reconstitution with a pharmaceutically acceptable aqueous diluent to a volume of 0.5 mL comprises:
   (i) the chimeric dengue serotype 2/1 strain in a concentration of at least 3.3 log10 pfu/0.5 mL,
   (ii) the dengue serotype 2 strain in a concentration of at least 2.7 log10 pfu/0.5 mL, or at least 3.1 log10 pfu/0.5 mL,
   (iii) the chimeric dengue serotype 2/3 strain in a concentration of at least 4.0 log10 pfu/0.5 mL,
   (iv) the chimeric dengue serotype 2/4 strain in a concentration of at least 4.5 log10 pfu/0.5 mL,
      a. the trehalose in a concentration of from 40 to 80 g/L, such as about 43 g/L (corresponding to about 47 g/L trehalose dihydrate),
      b. the poloxamer in a concentration of about 0.1 to 5.0 g/L, such as about 1.42 g/L,
      c. the urea in a concentration of about 0.1 to 6.0 g/L, such as about 1.42 g/L,
      d. the arginine hydrochloride in a concentration of about 0.5 to 5.0 g/L, such as about 1.18 g/L,
      e. the tromethamine in a concentration of about 0.1 to 1.5 g/L, such as about 0.6 g/L, and
      f. the human serum albumin in a concentration of from 0.1 to less than 1 g/L, such as about 0.48 g/L,
      wherein the dengue vaccine formulation comprises:
      a. the trehalose at an amount of 20 to 34 mg/dose,
      b. the poloxamer at an amount of 0.1 to 3.0 mg/dose,
      c. the urea at an amount of 0.1 to 3.0 mg/dose,
      d. the arginine hydrochloride at an amount of 0.1 to 2.0 mg/dose,
      e. the tromethamine at an amount of 0.05 to 1.0 mg/dose, and
      f. the human serum albumin at an amount of 0.05 to 0.5 mg/dose.

The present invention is further directed in part to a container, such as a vial, comprising the dengue vaccine formulation of the invention as described herein, preferably in dry form, such as in lyophilized form.

The present invention is in particular directed to such a container, such as a vial, comprising one to ten unit dose(s), such as one unit dose or multiple (e.g. five) unit doses, of the dengue vaccine formulation of the invention as described herein, preferably in dry form, such as in lyophilized form.

The present invention is in particular directed to such a container, such as a vial, comprising the dengue vaccine formulation of the invention as described herein, preferably in dry form, such as in lyophilized form, wherein
a. the trehalose is contained at an amount of about 100 to 170 mg, such as about 149 mg,
b. the poloxamer is contained at an amount of about 2.0 to 8.0 mg, such as about 4.95 mg,
c. the urea is contained at an amount of about 2.0 to 8.0 mg, such as about 4.95 mg,
d. the arginine hydrochloride is contained at an amount of about 1.5 to 7.5 mg, such as about 4.1 mg,
e. the tromethamine is contained at an amount of about 0.5 to 4.0 mg, such as about 2.0 mg,
f. the human serum albumin is contained at an amount of about 0.1 to 2.5 mg, such as about 1.65 mg.

The present invention is in particular directed to such a container, such as a vial, comprising five unit doses of the dengue vaccine formulation of the invention as described herein, wherein
a. the trehalose is contained at an amount of about 100 to 170 mg, such as about 149 mg,
b. the poloxamer is contained at an amount of about 2.0 to 8.0 mg, such as about 4.95 mg,
c. the urea is contained at an amount of about 2.0 to 8.0 mg, such as about 4.95 mg,
d. the arginine hydrochloride is contained at an amount of about 1.5 to 7.5 mg, such as about 4.1 mg
e. the tromethamine is contained at an amount of about 0.5 to 4.0 mg, such as about 2.0 mg,
f. the human serum albumin is contained at an amount of about 0.1 to 2.5 mg, such as about 1.65 mg.

The present invention is further directed in part to a kit comprising one or more than one, such as ten, container(s) as described in the preceding paragraphs, and optionally one or more than one, such as ten, container(s) comprising a pharmaceutically acceptable aqueous diluent for reconstitution.

The present invention is further directed in part to a kit for preparing a dengue vaccine formulation in reconstituted form comprising: a) a container, such as a vial, comprising the dengue vaccine formulation of the invention as described herein in dry form, such as a lyophilized dengue vaccine formulation as described herein, and b) a container, such as a vial, comprising a pharmaceutically acceptable aqueous diluent for reconstitution.

The present invention is in particular directed to such a kit for preparing one to ten unit dose(s), such as one unit dose or multiple (e.g. five) unit doses, of a dengue vaccine formulation in reconstituted form comprising: a) a container, such as a vial, comprising one to ten unit dose(s), such as one unit dose or multiple (e.g. five) unit doses, of the dengue vaccine formulation of the invention as described herein in dry form, such as a lyophilized dengue vaccine formulation as described herein, and b) a container, such as a vial, comprising one to ten aliquot(s), such as one aliquot (e.g. 0.5 mL/unit dose) or multiple (e.g. five) aliquots (e.g. 2.5 mL), of a pharmaceutically acceptable aqueous diluent (e.g. 37 mM aqueous sodium chloride solution) for reconstitution, wherein the number of aliquots correspond to the number of unit doses. Said aliquot(s) are preferably provided in one container, such as in one vial.

The present invention is further directed in part to five unit doses of a dengue vaccine formulation in aqueous form obtainable by reconstituting five unit doses of the dengue vaccine formulation of the invention as described herein in dry form with five aliquots of a pharmaceutically acceptable aqueous diluent.

The present invention is further directed in part to a unit dose of a dengue vaccine formulation in aqueous form obtainable by reconstituting a unit dose of the dengue vaccine formulation of the invention as described herein in dry form with an aliquot (e.g. 0.5 mL/unit dose) of a pharmaceutically acceptable aqueous diluent (e.g. 37 mM aqueous sodium chloride solution).

The present invention is further directed in part to a unit dose of a dengue vaccine formulation in aqueous form obtainable by reconstituting multiple unit doses, e.g. five unit doses, of the dengue vaccine formulation of the invention as described herein in dry form with multiple aliquots, e.g. five aliquots, of a pharmaceutically acceptable aqueous diluent (e.g. 37 mM aqueous sodium chloride solution), wherein the number of aliquots correspond to the number of unit doses, and removing the volume of a unit dose (e.g. 0.5 mL) of the reconstituted dengue vaccine formulation.

The present disclosure is further directed in part to a method of preparing a dengue vaccine formulation in dry form comprising the following steps:
(a) providing a dengue vaccine formulation in aqueous form, (b) freezing the dengue vaccine formulation in aqueous form to form a frozen dengue vaccine formulation, (c) subjecting the frozen dengue vaccine formulation to a primary drying step in an apparatus with a shelf temperature, which ranges from above the glass transition temperature Tg of the formulation to not more than 25 °C above, or not more than 20 °C above the glass transition temperature Tg and at a pressure below 0.1 mbar to form a primary dried product, and (d) subjecting the primary dried product to a secondary drying step in an apparatus with a shelf temperature, which is above the shelf temperature of the primary drying step and at a pressure below 1 mbar to form a secondary dried product.

The present disclosure is further directed in part to a method of preparing a dengue vaccine formulation in dry form comprising the following steps:
(a) providing a dengue vaccine formulation in aqueous form, (b) freezing the dengue vaccine formulation in aqueous form to form a frozen dengue vaccine formulation, (c) subjecting the frozen dengue vaccine formulation to a primary drying step in an apparatus with a shelf temperature, which ranges from above the glass transition temperature Tg of the formulation to not more than 15 °C above the glass transition temperature Tg and at a pressure below 0.1 mbar to form a primary dried product, and (d) subjecting the primary dried product to a secondary drying step in an apparatus with a shelf temperature, which is above the shelf temperature of the primary drying step and at a pressure below 1 mbar to form a secondary dried product.

The present invention is further directed in part to a method of preparing a dengue vaccine formulation of the invention as described herein in dry form comprising the following steps:
(a) providing a dengue vaccine formulation in aqueous form comprising:
   A. a tetravalent dengue virus composition comprising :
      (i) live attenuated dengue virus serotype 1, (ii) live attenuated dengue virus serotype 2, (iii) live attenuated dengue virus serotype 3, and (iv) live attenuated dengue virus serotype 4; and
   B. an excipient composition comprising: a. trehalose, b. poloxamer, c. urea, d. arginine hydrochloride, e. tromethamine, and f. human serum albumin,
(b) freezing the dengue vaccine formulation in aqueous form in an apparatus with a shelf temperature of below -35 °C to form a frozen dengue vaccine formulation,
(c) subjecting the frozen dengue vaccine formulation to a primary drying step in an apparatus with a shelf temperature of from about -31 to -19 °C and at a pressure below 0.1 mbar to form a primary dried product, and
(d) subjecting the primary dried product to a secondary drying step in an apparatus with a shelf temperature of at least 15 °C and at a pressure below 1 mbar to form a secondary dried product,
wherein the dengue vaccine formulation comprises:
a. the trehalose at an amount of 20 to 34 mg/dose,
b. the poloxamer at an amount of 0.1 to 3.0 mg/dose,
c. the urea at an amount of 0.1 to 3.0 mg/dose,
d. the arginine hydrochloride at an amount of 0.1 to 2.0 mg/dose,
e. the tromethamine at an amount of 0.05 to 1.0 mg/dose, and
f. the human serum albumin at an amount of 0.05 to 0.5 mg/dose.

The present invention is further directed in part to a method of preparing a dengue vaccine formulation of the invention as described herein in dry form comprising the following steps:
(a) providing a dengue vaccine formulation in aqueous form comprising:
   A. a tetravalent dengue virus composition comprising:
      (i) live attenuated dengue virus serotype 1, (ii) live attenuated dengue virus serotype 2, (iii) live attenuated dengue virus serotype 3, and (iv) live attenuated dengue virus serotype 4; and
   B. an excipient composition comprising: a. trehalose, b. poloxamer, c. urea, d. arginine hydrochloride, e. tromethamine, and f. human serum albumin,
(b) freezing the dengue vaccine formulation in aqueous form in an apparatus with a shelf temperature of below -35 °C to form a frozen dengue vaccine formulation,
(c) subjecting the frozen dengue vaccine formulation to a primary drying step in an apparatus with a shelf temperature of from about -31 to -21 °C and at a pressure below 0.1 mbar to form a primary dried product, and
(d) subjecting the primary dried product to a secondary drying step in an apparatus with a shelf temperature of at least 20 °C and at a pressure below 1 mbar to form a secondary dried product,
wherein the dengue vaccine formulation comprises:
a. the trehalose at an amount of 20 to 34 mg/dose,
b. the poloxamer at an amount of 0.1 to 3.0 mg/dose,
c. the urea at an amount of 0.1 to 3.0 mg/dose,
d. the arginine hydrochloride at an amount of 0.1 to 2.0 mg/dose,
e. the tromethamine at an amount of 0.05 to 1.0 mg/dose, and
f. the human serum albumin at an amount of 0.05 to 0.5 mg/dose.

The advantageous settings of the method of preparing a dengue vaccine formulation of the invention in dry form as described herein help in reducing the time for drying the dengue vaccine formulation as described herein and in improving the appearance of the dengue vaccine formulation in dry form.

The present invention is further directed in part to a method of preparing five unit doses of a dengue vaccine formulation of the invention as described herein in dry form comprising the following steps:
(a) providing a dengue vaccine formulation in aqueous form comprising:
   A. a tetravalent dengue virus composition comprising:
      (i) a live attenuated dengue virus serotype 1, such as a chimeric dengue serotype 2/1 strain,
      (ii) a live attenuated dengue virus serotype 2, such as a dengue serotype 2 strain,
      (iii) a live attenuated dengue virus serotype 3, such as a chimeric dengue serotype 2/3 strain, and
      (iv) a live attenuated dengue virus serotype 4, such as a chimeric dengue serotype 2/4 strain; and
   B. an excipient composition comprising:
      a. about 9 to 11 % w/v, such as about 10 % w/v trehalose,
      b. about 0.2 to 0.4 % w/v, such as about 0.3 % w/v poloxamer,
      c. about 0.2 to 0.4 % w/v, such as about 0.3 % w/v urea,
      d. about 0.2 to 0.3 % w/v, such as about 0.25 % w/v arginine hydrochloride,
      e. about 0.05 to 0.15 % w/v, such as about 0.1 % w/v tromethamine, and
      f. about 0.05 to 0.15 % w/v, such as about 0.1 % w/v human serum albumin,
(b) freezing the dengue vaccine formulation in aqueous form an apparatus with a shelf temperature of about -40 to -50 °C to form a frozen dengue vaccine formulation,
(c) subjecting the frozen dengue vaccine formulation to a primary drying step in an apparatus with a shelf temperature of from about -31 to -19 °C, or from about -31 to -21 °C and at a pressure below 0.1 mbar for a period of about 30 to 100 hours to form a primary dried product, and
(d) subjecting the primary dried product to a secondary drying step in an apparatus with a shelf temperature of from about 15 to 40 °C and at a pressure below 1 mbar for a period of about 3 to 10 hours to form a secondary dried product,

wherein the dengue vaccine formulation in aqueous form is subjected to the freezing step b) in a volume, which is about half of the volume used for reconstituting the five unit doses of the dengue vaccine formulation in dry form,
wherein the dengue vaccine formulation comprises:
   a. the trehalose at an amount of 20 to 34 mg/dose,
   b. the poloxamer at an amount of 0.1 to 3.0 mg/dose,
   c. the urea at an amount of 0.1 to 3.0 mg/dose,
   d. the arginine hydrochloride at an amount of 0.1 to 2.0 mg/dose,
   e. the tromethamine at an amount of 0.05 to 1.0 mg/dose, and
   f. the human serum albumin at an amount of 0.05 to 0.5 mg/dose.

The present invention is further directed in part to a method of preparing five unit doses of a dengue vaccine formulation of the invention as described herein in dry form comprising the following steps:
(a) providing a dengue vaccine formulation in aqueous form comprising:
   A. a tetravalent dengue virus composition comprising:
      (i) a live attenuated dengue virus serotype 1, such as a chimeric dengue serotype 2/1 strain,
      (ii) a live attenuated dengue virus serotype 2, such as a dengue serotype 2 strain,
      (iii) a live attenuated dengue virus serotype 3, such as a chimeric dengue serotype 2/3 strain, and
      (iv) a live attenuated dengue virus serotype 4, such as a chimeric dengue serotype 2/4 strain; and
   B. an excipient composition comprising:
      a. about 9 to 11 % w/v, such as about 10 % w/v trehalose,
      b. about 0.2 to 0.4 % w/v, such as about 0.3 % w/v poloxamer,
      c. about 0.2 to 0.4 % w/v, such as about 0.3 % w/v urea,
      d. about 0.2 to 0.3 % w/v, such as about 0.25 % w/v arginine hydrochloride,
      e. about 0.05 to 0.15 % w/v, such as about 0.1 % w/v tromethamine, and
      f. about 0.05 to 0.15 % w/v, such as about 0.1 % w/v human serum albumin,
(b) freezing the dengue vaccine formulation in aqueous form an apparatus with a shelf temperature of about -40 to -50 °C to form a frozen dengue vaccine formulation,
(c) subjecting the frozen dengue vaccine formulation to a primary drying step in an apparatus with a shelf temperature of from about -31 to -19 °C, or from about -31 to -21 °C and at a pressure below 0.1 mbar for a period of about 30 to 100 hours to form a primary dried product, and
(d) subjecting the primary dried product to a secondary drying step in an apparatus with a shelf temperature of from about 20 to 40 °C and at a pressure below 1 mbar for a period of about 3 to 10 hours to form a secondary dried product,

wherein the dengue vaccine formulation in aqueous form is subjected to the freezing step b) in a volume, which is about half of the volume used for reconstituting the five unit doses of the dengue vaccine formulation in dry form,
wherein the dengue vaccine formulation comprises:
   a. the trehalose at an amount of 20 to 34 mg/dose,
   b. the poloxamer at an amount of 0.1 to 3.0 mg/dose,
   c. the urea at an amount of 0.1 to 3.0 mg/dose,
   d. the arginine hydrochloride at an amount of 0.1 to 2.0 mg/dose,
   e. the tromethamine at an amount of 0.05 to 1.0 mg/dose, and
   f. the human serum albumin at an amount of 0.05 to 0.5 mg/dose.

The advantageous dengue vaccine formulation of the invention as described herein in combination with the advantageous method of preparing it in dry form as described herein using a reduced fill volume for drying multiple unit doses (e.g. five unit doses) of the dengue vaccine formulation helps in reducing consumption of dengue virus (e.g. TDV-3), in reducing the filing time and the time in solution of the dengue vaccine formulation during manufacturing as well as in reducing the lyophilization cycle length, which results in a gain in product capacity.

The present invention is further directed in part to a dengue vaccine formulation of the invention as described herein for use in a method of preventing dengue disease in a subject, wherein the method comprises administering a primary vaccination with only two administrations of a unit dose comprising the steps of: administering a first unit dose of the dengue vaccine formulation to the subject, and administering a second unit dose of the dengue vaccine formulation to the subject, such as within 3 months of administration of the first unit dose, and optionally administering a booster dose at least 12 months after administration of said second unit dose of the dengue vaccine.

The present disclosure is further directed in part to a method of preventing dengue disease in a subject comprising administering a primary vaccination with only two administrations of a unit dose of the dengue vaccine formulation as described herein comprising the steps of: administering a first unit dose of the dengue vaccine formulation to the subject, and administering a second unit dose of the dengue vaccine formulation to the subject within 3 months of administration of the first unit dose, and optionally administering a booster dose at least 12 months after administration of said second unit dose of the dengue vaccine.

### BRIEF DESCRIPTION OF THE DRAWINGS

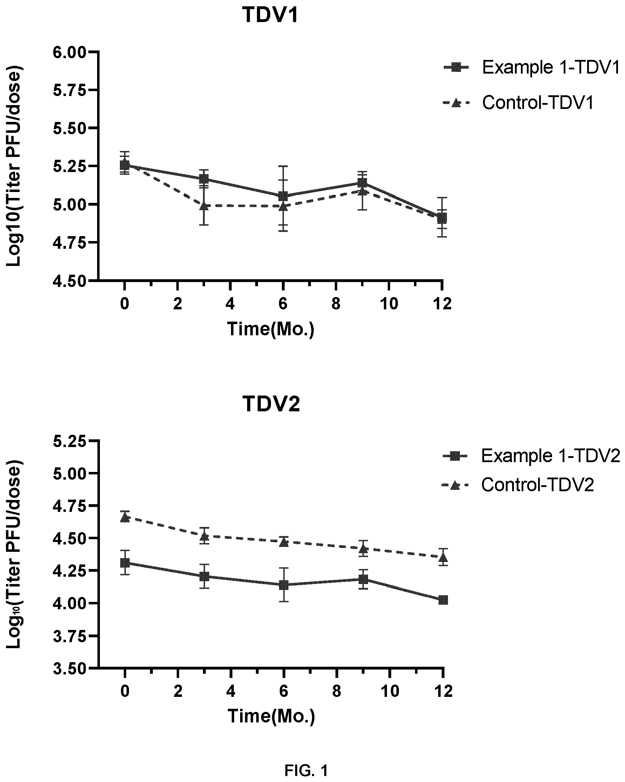

Fig. 1 illustrates data obtained from experiments using a dengue vaccine formulation prepared according to Example 1 disclosed herein and a dengue vaccine control formulation prepared according to the comparative example disclosed herein ("Control") for determining the titer loss (log loss) of TDV-1 and TDV-2 after exposure of the lyophilized formulations to about 5 °C for 12 months.

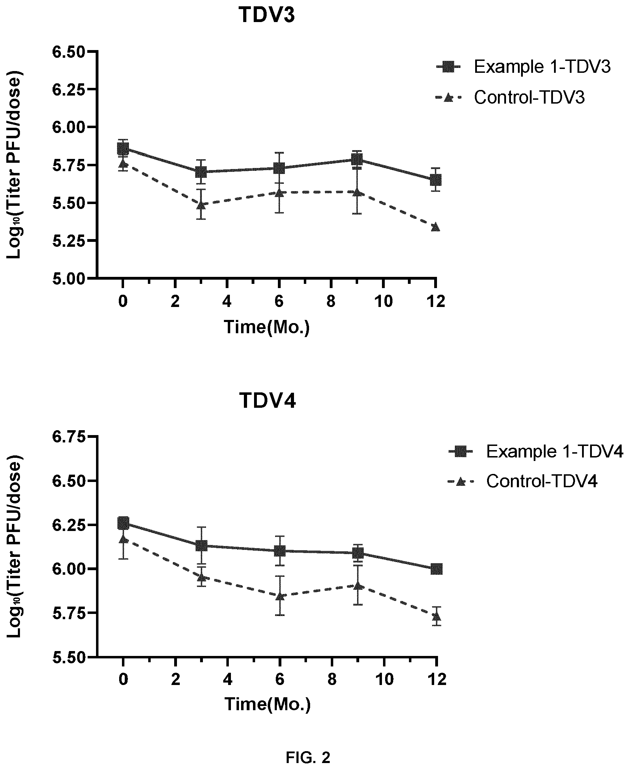

Fig. 2 illustrates data obtained from experiments using a dengue vaccine formulation prepared according to Example 1 disclosed herein and a dengue vaccine control formulation prepared according to the comparative example disclosed herein ("Control") for determining the titer loss (log loss) of TDV-3 and TDV-4 after exposure of the lyophilized formulations to about 5 °C for 12 months.

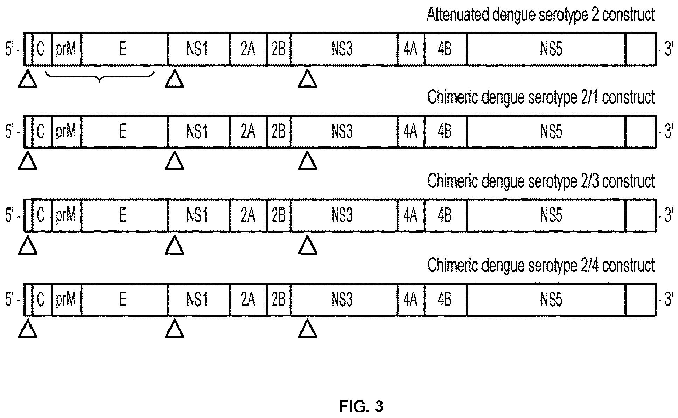

Fig. 3 illustrates the genetic structure of the four dengue strains contained in TDV as published in WO 2020/051334 A1. The triangles indicate the three attenuating mutations present in the 5'NCR, NS1 and NS3 proteins. The TDV-1, TDV-3 and TDV-4 strains are chimeric viruses where the prM and E genes from dengue serotype 1, 3 and 4, respectively, are inserted into the TDV-2 backbone.

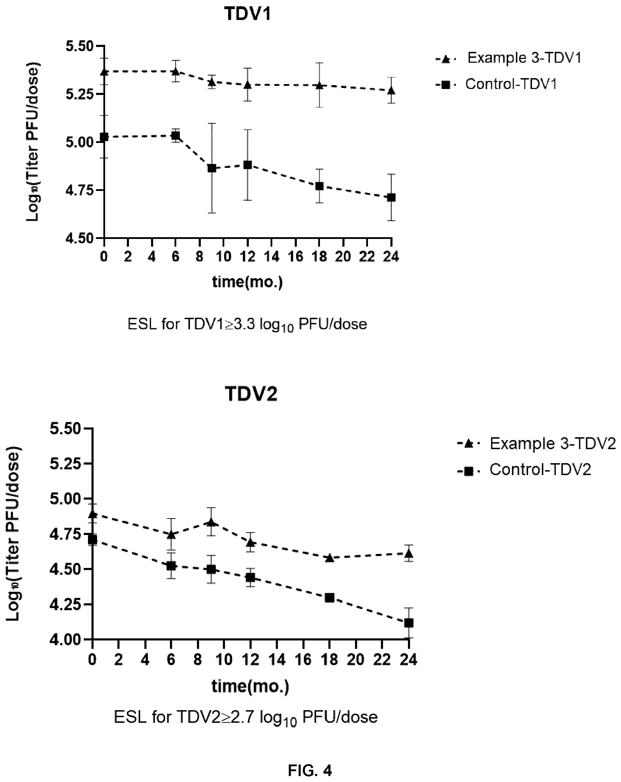

Fig. 4 illustrates data obtained from experiments using a dengue vaccine formulation prepared according to Example 3 disclosed herein and a dengue vaccine control formulation prepared according to the comparative example disclosed herein ("Control") for determining the titer loss (log loss) of TDV-1 and TDV-2 after exposure of the lyophilized formulations to about 5 °C for 6, 9, 12, 18, and 24 months.

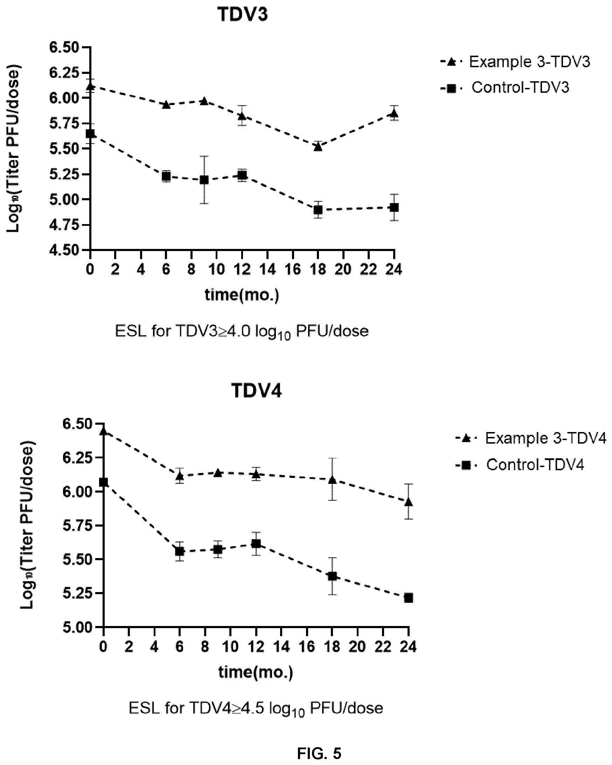

Fig. 5 illustrates data obtained from experiments using a dengue vaccine formulation prepared according to Example 3 disclosed herein and a dengue vaccine control formulation prepared according to the comparative example disclosed herein ("Control") for determining the titer loss (log loss) of TDV-3 and TDV-4 after exposure of the lyophilized formulations to about 5 °C for 6, 9, 12, 18, and 24 months.

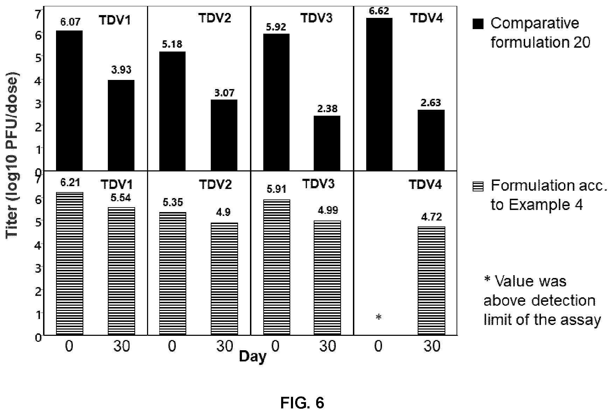

Fig. 6 illustrates data obtained from experiments using a dengue vaccine formulation prepared according to Example 4 disclosed herein and a dengue comparative formulation 20 disclosed herein for determining the titer at day 0, and at day 30 after exposure of the liquid formulations to 2-8 °C.

### DETAILED DESCRIPTION

### Definitions

In describing the present invention, the following terms are to be used as indicated below. As used herein, the singular forms "a", "an", and "the" include plural references unless the context clearly indicates otherwise.

As used herein, the terms "dose", "unit dose", "unit dose of a dengue vaccine formulation", and "unit dose of the invention as described herein" refer to the amount of a dengue vaccine which is administered to a subject in a single dose. In one embodiment, one unit dose is present in a container, such as a vial and this unit dose is administered to a subject, optionally after reconstitution. In one embodiment, more than one unit dose of the dengue vaccine formulation may be present in a vial so that with the content of one vial more than one subject can be vaccinated. In one specific embodiment, multiple (such as five) unit doses of the dengue vaccine formulation are present in a vial. If multiple (such as five) unit doses are present in a vial in dry form, a unit dose of the dengue vaccine formulation can be obtained by reconstituting the multiple (such as five) unit doses with the same number (such as five) of aliquots of a pharmaceutically acceptable aqueous diluent and removing the volume of a unit dose (e.g. 0.5 mL) of the reconstituted dengue vaccine formulation.

As used herein, the term "aliquot" refers to the volume of a pharmaceutically acceptable aqueous diluent per unit dose, expressed in mL/unit dose, to which a unit dose of a dengue vaccine formulation in dry form is subjected for reconstitution. Accordingly, if one unit dose is present in a container, such as a vial, one aliquot (e.g. 0.5 mL/unit dose) of a pharmaceutically acceptable aqueous diluent is added for reconstitution. If multiple (e.g. five) unit doses are present in a container, such as a vial, an equal number of aliquots (e.g. five aliquots) of a pharmaceutically acceptable aqueous diluent are added for reconstitution. In certain such embodiments, the multiple (e.g. five) aliquots for reconstitution are provided in one container, such as a vial, or a syringe, and the volume of the multiple (e.g. five) aliquots (e.g. 2.5 mL) are preferably added at once to the dengue vaccine formulation in dry form. The volume of the reconstituted vaccine formulation is greater than the aliquot(s) used for reconstitution, since the dissolved components of the vaccine formulation add to the final volume.

As used herein, the term "a container, such as a vial, comprising one or more than one unit dose (e.g. a unit dose, one to ten unit doses, multiple unit doses, or five unit doses) of the dengue vaccine formulation" permits an overfill of dengue vaccine formulation by a factor of about 1.3 per unit dose. This allows for an accurate removal of a unit dose from the container, such as the vial. For example, a container, such as a vial, comprising a unit dose of the dengue vaccine formulation in dry form contains 1.3 times the amount of the dengue vaccine to be administered to a subject in a single dose. The volume of the pharmaceutically acceptable aqueous diluent that is added for reconstitution is increased accordingly by the same factor (e.g. 1.3 x 0.5 mL/unit dose = 0.65 mL). The volume of a unit dose (e.g. 0.5 mL) is then removed, for example with a syringe, from the total volume of reconstituted dengue vaccine formulation, and administered to a subject. Alternatively, the total volume of the reconstituted dengue vaccine formulation (e.g. 0.7 mL) is removed, for example with a syringe, and the volume of a unit dose (e.g. 0.5 mL) is administered to a subject. In a preferred embodiment, the dengue vaccine formulation in the vial is reconstituted with the entire contents of a vial or pre-filled syringe of diluent (e.g. 37 mM sodium chloride solution) so that a 0.5 mL dose of vaccine can be withdrawn and administered.

As used herein, the term "upon reconstitution with 0.5 mL" is not limiting the reconstitution to be performed using 0.5 mL of the diluent but refers to the amount of virus and excipient that will be present in a unit dose of the dengue vaccine formulation when 0.5 mL diluent are used for reconstitution. While using a different volume for reconstituting a unit dose (e.g. 0.8 mL) will result in a different concentration of virus in the reconstituted unit dose, the administration of the total volume of the unit dose (e.g. 0.8 mL) will result in the same total amount of virus being administered. The same applies to the amount of excipient contained in a reconstituted unit dose unless the pharmaceutically acceptable aqueous diluent used for reconstitution contains additional excipients. If the diluent contains additional excipients (e.g. sodium chloride), the total amount of excipients contained in a reconstituted unit dose will be increased by the amount of the excipients added by the diluent. In a preferred embodiment of the invention, the diluent does not add excipients other than sodium chloride to the unit dose. In certain such embodiments, the diluent used for reconstitution adds less than 1.5 mg/unit dose (e.g. about 1.1 mg/unit dose) sodium chloride to the dengue vaccine formulation.

A "reconstituted unit dose" or "unit dose in reconstituted form" is obtained from the unit dose of the dengue vaccine formulation in dry form by reconstitution with a pharmaceutically acceptable aqueous diluent. The diluent does not contain dengue virus. The reconstituted unit dose is a liquid which can be administered to a subject, for example by injection, such as subcutaneous injection.

As used herein, the term "dengue vaccine formulation in dry form" refers to a formulation obtained and/or obtainable by subjecting a given volume of the liquid dengue vaccine formulation to a drying procedure, such as lyophilization, to remove the solvent. A lyophilized dengue vaccine formulation is obtained and/or obtainable by subjecting a given volume of the liquid dengue vaccine formulation to lyophilization, a drying procedure in which the formulation is first frozen followed by the removal of the ice or frozen solvent by sublimation under vacuum. As used herein, a lyophilized formulation is a formulation in dry form. The dengue vaccine formulation in dry form as described herein has a residual moisture content of preferably less than 5 % w/w.

As used herein, "% w/w" or "% (w/w)" refers to % mg/mg. The % w/w of a component "based on the dry weight of the vaccine formulation" as used herein can be calculated by dividing the amount of said component in mg by the amount of the dengue vaccine formulation in dry form in mg and multiplying the result by 100. Alternatively, the % w/w of a component "based on the dry weight of the vaccine formulation" as used herein can be calculated by dividing the amount of said component in mg by the total amount of excipients contained in the dengue vaccine formulation in dry form in mg and multiplying the result by 100, since the weight of the tetravalent dengue virus composition contained in the dengue vaccine formulation in dry form is negligible, and the difference in the result is insignificant.

As used herein, the term "about" used for numerical ranges refers to a variance of ± 10%.

As used herein, the term "dengue vaccine formulation in aqueous form" refers to a water-based liquid formulation. A dengue vaccine formulation in aqueous form may be subject to a drying procedure, such as lyophilization, to obtain a dengue vaccine formulation in dry form. A dengue vaccine formulation in aqueous form may also be formed from a dengue vaccine formulation in dry form by reconstitution with a pharmaceutically acceptable diluent. Thus, the terms "dengue vaccine formulation in reconstituted form" and "reconstituted dengue vaccine formulation" refer to a formulation obtained from a dengue vaccine formulation in dry form, such as a lyophilized dengue vaccine formulation, by reconstitution with a pharmaceutically acceptable aqueous diluent, such as 37 mM aqueous sodium chloride solution or water for injection. The diluent does not contain dengue virus. The reconstituted dengue vaccine formulation can be administered to a subject, for example by injection, such as subcutaneous injection.

As used herein "% w/v" or "% (w/v)" refers to % mg/mL wherein e.g. 100 mg/mL are 10% w/v.

As used herein, a "concentration of at least X log10 pfu/0.5 mL" refers to the concentration of a dengue serotype in 0.5 mL, but is not limiting the unit dose to be 0.5 mL. If the unit dose has a volume different than 0.5 mL, or is lyophilized from a volume different than 0.5 mL, or is reconstituted with a volume different than 0.5 mL, said concentration will differ from the "concentration of at least X log10 pfu/0.5 mL". However, if the unit dose has a volume of 0.5 mL, or is lyophilized from a volume of 0.5 mL, or is reconstituted with a volume of 0.5 mL, said concentration will be the "concentration of at least X log10 pfu/0.5 mL". Thus, while the concentration may differ, the total amount of virus in the unit dose remains the same.

The "total concentration in pfu/0.5 ml" which serves as a base value for the calculation of the percentage concentration for each individual component of a tetravalent dengue vaccine is shown for one exemplary tetravalent vaccine composition comprising dengue serotype 1 in a concentration of 3.60 log10pfu/0.5 mL, a dengue serotype 2 concentration of 4.00 log10pfu/0.5 mL, a dengue serotype 3 concentration of 4.60 log10pfu/0.5 mL and a dengue serotype 4 concentration of 5.11 log10pfu/0.5 mL. Primarily, the logarithmic values of the concentrations are converted into numerical values. The results of this conversion are 4x10³ pfu/0.5 mL for serotype 1, 1x10⁴ pfu/0.5mL for serotype 2, 4x10⁴ pfu/0.5 mL for serotype 3 and 1.3x10⁵ pfu/0.5 mL for serotype 4. The total concentration in pfu/0.5 mL is the sum of the preceding numerical values resulting in 1.84 x10⁵ pfu/0.5 mL.

The "percentage concentration" for each of the serotypes 1, 2, 3 and 4 is obtained by dividing the numerical concentration value (expressed as pfu/0.5 mL) of an individual serotype by the total concentration (expressed in pfu/0.5 mL) and multiplying the result by 100 i.e.: Percentage concentration of serotype 1 = (4x103 pfu/0.5 mL ÷ 1.84 x105 pfu/0.5 mL) x 100 = 2%. Percentage concentration of serotype 2 = (1x104 pfu/0.5mL ÷ 1.84 x105 pfu/0.5 mL) x 100 = 5%. Percentage concentration of serotype 3 = (4x104 pfu/0.5 mL ÷ 1.84 x105 pfu/0.5 mL) x 100 = 22%. Percentage concentration of serotype 4 = (1.3x105 pfu/0.5 mL ÷ 1.84 x105 pfu/0.5 mL) x 100 = 71%.

The percentage concentrations are rounded to whole numbers.

As used herein, the terms "dengue serotype" and "dengue virus serotype" refer to a species of dengue virus which is defined by its cell surface antigens and therefore can be distinguished by serological methods known in the art. At present, four serotypes of dengue virus are known, i.e. dengue serotype 1 (DENV-1), dengue serotype 2 (DENV-2), dengue serotype 3 (DENV-3) and dengue serotype 4 (DENV-4).

As used herein, the term "tetravalent dengue virus composition" refers to a dengue virus composition comprising four different immunogenic components from the four different dengue serotypes DENV-1, DENV-2, DENV-3 and DENV-4, preferably comprising four different live attenuated dengue viruses, each representing one dengue serotype, and which aims to stimulate immune responses to all four dengue serotypes.

As used herein, the term "live attenuated dengue virus" refers to a viable dengue virus which is mutated to provide reduced virulence. The live attenuated dengue virus can be a dengue virus in which all components are derived from the same dengue serotype or it can be a chimeric dengue virus having parts from two or more dengue serotypes.

A "virus strain" and in particular a "dengue virus strain" is a genetic subtype of a virus, in particular of a dengue virus, which is characterized by a specific nucleic acid sequence. A dengue serotype may comprise different strains with different nucleic acid sequences which have the same cell surface antigens. A dengue virus strain can be a dengue virus in which all components are derived from the same dengue serotype or it can be a chimeric dengue virus having parts from two or more dengue serotypes.

As used herein, "TDV-2" refers to a molecularly characterized and cloned dengue serotype 2 strain derived from the live attenuated DEN-2 PDK-53 virus strain. The PDK-53 strain is described for example in Bhamarapravati et al. (1987) Bulletin of the World Health Organization 65(2): 189-195. In one embodiment, the TDV-2 strain served as a backbone for the chimeric TDV-1, TDV-3 and TDV-4 strains into which parts from the TDV-1, TDV-3 and TDV-4 strains were introduced. TDV-1, TDV-2, TDV-3 and TDV-4 together form a tetravalent dengue virus composition, "TAK-003", a dengue vaccine candidate for which regulatory filings have recently been submitted by Takeda.

A "non-chimeric dengue virus" or "non-chimeric dengue serotype strain" or "non-chimeric dengue strain" comprises only parts from one dengue serotype. In particular, a non-chimeric dengue virus does not include parts from a different flavivirus such as yellow fever virus, Zika virus, West Nile virus, Japanese encephalitis virus, St. Louis encephalitis virus, tick-borne encephalitis virus. TDV-2 is an example of a non-chimeric dengue virus.

A "chimeric dengue virus" or "chimeric dengue serotype strain" or "chimeric dengue strain" comprises parts from at least two different dengue serotypes. Preferably, the chimeric dengue virus does not include parts from a different flavivirus such as yellow fever virus, Zika virus, West Nile virus, Japanese encephalitis virus, St. Louis encephalitis virus, tick-borne encephalitis virus. More preferably, the chimeric dengue virus described herein does not include parts from the yellow fever virus. As used herein, a "chimeric dengue serotype 2/1 strain" or "DENV-2/1 chimera" or "TDV-1" refers to a dengue virus chimeric construct which comprises parts from both DENV-2 and DENV-1. Preferably, in the chimeric dengue serotype 2/1 strain the prM and E proteins from DENV-1 replace the prM and E proteins from DENV-2 as detailed below. As used herein, a "chimeric dengue serotype 2/3 strain" or "DENV-2/3 chimera" or "TDV-3" refers to a dengue virus chimeric construct which comprises parts from both DENV-2 and DENV-3. Preferably, in the chimeric dengue serotype 2/3 strain the prM and E proteins from DENV-3 replace the prM and E proteins from DENV-2 as detailed below. As used herein, a "chimeric dengue serotype 2/4 strain" or "DENV-2/4 chimera" or "TDV-4" refers to a dengue virus chimeric construct which comprises parts from both DENV-2 and DENV-4. Preferably, in the chimeric dengue serotype 2/4 strain the prM and E proteins from DENV-4 replace the prM and E proteins from DENV-2 as detailed below.

As used herein, "TDV" refers to a tetravalent live attenuated dengue vaccine that comprises a mixture of the four live attenuated dengue virus strains TDV-1, TDV-2, TDV-3 and TDV-4 expressing surface antigens from the four dengue serotypes DENV-1, DENV-2, DENV-3 and DENV-4, respectively. In one embodiment (e.g. also in the examples), TDV-1 is characterized by the nucleotide sequence according to SEQ ID No. 1 and/or the amino acid sequence according to SEQ ID No. 2. In one embodiment, TDV-2 is characterized by the nucleotide sequence according to SEQ ID No. 3 and/or the amino acid sequence according to SEQ ID No. 4. In one embodiment, TDV-3 is characterized by the nucleotide sequence according to SEQ ID No. 5 and/or the amino acid sequence according to SEQ ID No. 6. In one embodiment, TDV-4 is characterized by the nucleotide sequence according to SEQ ID No. 7 and/or the amino acid sequence according to SEQ ID No. 8.

As used herein, the term "lyophilization loss" refers to the log10 loss in titer of live attenuated dengue virus (e.g. TDV-3) due to lyophilization and is calculated by subtracting the amount of live attenuated dengue virus in log10 pfu/unit dose of a lyophilized dengue vaccine formulation from the amount of live attenuated dengue virus in log10 pfu/unit dose of the dengue vaccine formulation in liquid form prior to lyophilization. For the analysis, the dengue vaccine formulation in liquid form is frozen and stored at -80 °C and thawed prior to testing. The lyophilized dengue vaccine formulation is reconstituted prior to testing.

As used herein, the term "stability loss" refers to the log10 loss in titer of live attenuated dengue virus (e.g. TDV-3) due to storage at a specified temperature (e.g. 2-8 °C) and is calculated by subtracting the amount of live attenuated dengue virus in log10 pfu/unit dose of a lyophilized dengue vaccine formulation, which was stored at a specified temperature, such as 2-8 °C, for a specified period, such as 3 months, 6 months, 8 months, or 12 months, from the amount of live attenuated dengue virus in log10 pfu/unit dose of the lyophilized dengue vaccine formulation prior to storage. The lyophilized dengue vaccine formulations are reconstituted prior to testing.

As used herein, the term "accelerated stability loss" refers to the log10 loss in titer of live attenuated dengue virus (e.g. TDV-3) due to storage at accelerated conditions and is calculated by subtracting the amount of live attenuated dengue virus in log10 pfu/unit dose of a lyophilized dengue vaccine formulation, which was stored at 25 °C for a specified period, such as 2 weeks, 5 weeks, or 12 weeks, from the amount of live attenuated dengue virus in log10 pfu/unit dose of the lyophilized dengue vaccine formulation prior to storage. Lyophilized dengue vaccine formulations are reconstituted prior to testing.

As used herein, the term "accelerated conditions" refers to a temperature of 25 °C and a relative humidity RH of 65%, or a temperature of 25 °C and a relative humidity RH of about 60% in the stability chamber. Accelerated conditions can be used to screen and select superior dengue vaccine formulations.

As used herein, the term "Tg" refers to the glass transition temperature determined by differential scanning calorimetry, such as low-temperature differential scanning calorimetry (LT-DSC). LT-DSC analysis can be performed by using the method described in USP <891> of the United States Pharmacopeial Convention.

As used herein, the term "dengue disease" refers to the disease which is caused by infection with dengue virus. Symptoms of dengue disease can e.g. include sudden high fever, headaches, joint and muscle pain, nausea, vomiting and skin rashes. The term dengue disease also includes the more severe forms of dengue hemorrhagic fever (DHF) and dengue shock syndrome (DSS). Symptoms of DHF include increased vascular permeability, hypovolemia and abnormal blood clotting mechanisms. Subjects with DHF may present with severe manifestations of plasma leakage and hemorrhage. When a subject with DHF experiences shock he or she will be categorized as having DSS. Symptoms of DSS include bleeding that may appear as tiny spots of blood on the skin and larger patches of blood under the skin. Prolonged shock is the main factor associated with complications including massive gastrointestinal hemorrhage that can lead to death. As used herein, DHF cases are defined as VCD cases meeting WHO 1997 DHF criteria. In the context of preventing dengue disease in elderly subjects, the term "preventing dengue disease" preferably includes preventing DHF and/or DSS. In the context of preventing dengue disease in elderly subjects, the term "preventing dengue disease" preferably includes preventing severe end-organ manifestations of dengue such as hepatomegaly and acute renal failure.

As used herein, "preventing dengue disease" refers to preventing a subject from developing one or more symptoms of dengue disease because of an infection with a dengue virus. In particular, preventing dengue disease is achieved by vaccinating or inoculating a subject with a dengue vaccine formulation, such as the reconstituted unit dose described herein. As used herein, the term "prophylactically treating dengue disease" is equivalent to "preventing dengue disease". In a particular embodiment, preventing dengue disease includes preventing DHS and/or DSS.

As used herein, "vaccinating" or "inoculating" refers to the administration of a vaccine to a subject, with the aim to prevent the subject, from developing one or more symptoms of a disease. As used herein, "vaccinating against dengue disease" or "inoculating against dengue disease" refers to the administration of a dengue vaccine composition to a subject, with the aim to prevent the subject, from developing one or more symptoms of dengue disease. In principle the method comprises a primary vaccination and optionally one or more booster vaccinations. The primary vaccination is defined as the primary administration schedule for administering the composition or unit dose as disclosed herein to establish a protective immune response and e.g. consists of two administrations e.g. within three months. Whenever an administration is mentioned within this disclosure, such administration refers to the primary vaccination unless it is specified as booster vaccination. The booster vaccination refers to an administration or administration schedule which takes place after the primary vaccination e.g. at least 1 year or even 5 or 10 years after the last administration, e.g. the second administration, of the primary vaccination schedule. The booster administration e.g. aims at enhancing the immune response of the primary vaccination.

As used herein, the terms "subject" or "subjects" are limited to human subjects (e.g. infants, children or adults). The terms "elderly subject" or "elderly subjects" refer to subjects with an age of more than 60 years, such as 61 years to 100 years, 61 years to 90 years, 61 years to 80 years, 61 years to 75 years, or 61 years to 70 years.

As used herein, "subject population" refers to a group of subjects. The subject population may refer to least 40 subjects, at least 50 subjects, at least 60 subjects, at least 100 subjects or at least 1000 subjects and is defined by certain parameters. The parameters that may be used to define a subject population include, but are not limited to, the age of the subjects, whether the subjects are from a dengue endemic region or from a dengue non-endemic region and the serostatus of the subjects.

As used herein, "endemic region" refers to a region where a disease or infectious agent is constantly present and/or usually prevalent in a population within this region. As used herein, "non-endemic region" refers to a region from which the disease is absent or in which it is usually not prevalent. Accordingly, a "dengue endemic region" refers to geographic areas in which an infection with dengue virus is constantly maintained at a baseline level. A "dengue non-endemic region" is a geographic area in which an infection with dengue virus is not constantly maintained at a baseline level. Accordingly, subject populations or subjects "from a dengue endemic region" or "from a dengue non-endemic region" refer to subject populations or subjects living in geographic areas as defined above. Whether a geographic area or a subject population is dengue-endemic or not can be determined by different calculatory methods such as the ones described in Bhatt et al. (2013) Nature 496 (7446): 504-507 and supplementary material and in Stanaway et al. (2016) Lancet Infect Dis. 16(6): 712-723 and supplementary material. Overviews of dengue endemic regions and dengue epidemiology are regularly published, for example, by the WHO or CDC. Typical dengue-endemic regions are in Latin America, Southeast Asia and the Pacific islands and dengue endemic countries include, but are not limited to, Australia, Brazil, Bangladesh, Colombia, China, Dominican Republic, Indonesia, India, Mexico, Malaysia, Nicaragua, Nigeria, Pakistan, Panama, Philippines, Puerto Rico, Singapore, Sri Lanka, Thailand and Vietnam. The area's force of infection is measured by seroprevalence surveys provided as seroprevalence rate. Areas with very high force of infection are considered to have a seroprevalence rate of more than 80%. As used herein the term "region" when it concerns seroprevalence rates refers to a geographic area where the seroprevalence rate could be determined or is known, e.g. a village, a town, a city, a region, a county, a state, a province or parts of the foregoing or a whole country.

As used herein, "serostatus" refers to the amount of antibodies a subject has with respect to a certain infectious agent, in particular dengue virus. As used herein, "seronegative" or "seronaïve" means that the subject does not have neutralizing antibodies against any one of dengue serotypes DENV-1, DENV-2, DENV-3 and DENV-4 in the serum. A seronegative or seronaïve subject or subject population is defined by a neutralizing antibody titer of less than 10 for each one of the four dengue serotypes. A subject or subject population having a neutralizing antibody titer of equal to or more than 10 for at least one dengue serotype is defined as being "seropositive" with respect to said dengue serotype. Serostatus at baseline refers to the serostatus before the administration of a dengue vaccine composition as described herein.

As used herein, a "neutralizing antibody titer" refers to the amount of antibodies in the serum of a subject that neutralize the respective dengue serotype. The neutralizing antibody titer against DENV-1, DENV-2, DENV-3 and DENV-4 is determined in a serum sample of the subject using known methods such as the plaque reduction neutralization test (PRNT) as described in the WHO Guidelines (World Health Organization Department of Immunization Vaccines Biologicals (2007) Guidelines for plaque reduction neutralization testing of human antibodies to dengue viruses, WHO/IVB/07.07) or a microneutralization (MNT50) assay as described herein. As used herein, the "ratio of not more than 20 for the neutralizing antibody titer of dengue serotype 2 to the neutralizing antibody titer of dengue serotype 4" means that the neutralizing antibody titer of dengue serotype 2 is divided by the neutralizing antibody titer of dengue serotype 4 and that the ratio obtained hereby is no more than 20. In other words, the neutralizing antibody titer of dengue serotype 2 is not more than 20-times higher than the neutralizing antibody titer of dengue serotype 4 in the subject.

As used herein, the terms "geometric mean neutralizing antibody titer" and "GMT" refer to the geometric mean value of the titer of neutralizing antibodies against the corresponding dengue serotype in the serum of subjects in a subject population. The geometric mean value is calculated by a well-known formula. As used herein, the "ratio of not more than 20 for the GMT of dengue serotype 2 to the GMT of dengue serotype 4" means that the geometric mean neutralizing antibody titer of dengue serotype 2 (GMT DENV-2) is divided by the geometric mean neutralizing antibody titer of dengue serotype 4 (GMT DENV-4) and that the ratio obtained hereby is no more than 20. In other words, the geometric mean neutralizing antibody titer of dengue serotype 2 is not more than 20-times higher than the geometric mean neutralizing antibody titer of dengue serotype 4 in the subject population.

As used herein, an "immune response" refers to a subject's response to the administration of the dengue vaccine. In particular, the immune response includes the formation of neutralizing antibodies to one or more dengue serotypes. It may also include the stimulation of a cell-mediated response or the formation of antibodies to non-structural proteins such as NS1. An immune response is stimulated by the administration of a unit dose of the invention as described herein, if the titer of neutralizing antibodies against at least one dengue virus serotype and preferably against all four dengue virus serotypes is increased after said administration of said unit dose. An immune response is stimulated by the administration of a unit dose of the invention as described herein, if the secretion of interferon gamma by peripheral blood mononuclear cells stimulated with peptides from dengue virus proteins is increased after said administration of said unit dose. An immune response is stimulated by the administration of a unit dose of the invention as described herein, if the titer of antibodies to non-structural proteins such as NS1 is increased after said administration of said unit dose. In a particular embodiment, the administration of a reconstituted unit dose of the present invention as described herein stimulates the formation of neutralizing antibodies to one or more dengue serotypes, a cell-mediated response and the formation of antibodies to non-structural proteins such as NS1.

As used herein, a "balanced immune response" means that the immune response to the four dengue serotypes is sufficient to provide protection against infection by all four dengue serotypes and preferably the immune response to the four dengue serotypes has a similar strength. In particular, the neutralizing antibody titer against the four dengue serotypes at day 180 or day 365 after administration of a first reconstituted unit dose of the invention as described herein is similar, i.e. it differs by less than factor 30, by less than factor 25 or by less than factor 20.

An RNA virus can be characterized by its RNA sequence. It is, alternatively or in addition, also common practice to characterize the genome of RNA viruses by the corresponding DNA sequences and corresponding DNA sequences are readily understood to reflect the RNA genome in the virus. Therefore, reference to "t" or "thymine" in the entire disclosure is to be understood as reference to "u" or "uracil", respectively, if the genomic RNA virus sequence is meant.

### Dengue vaccine formulation

The present disclosure relates to a dengue vaccine formulation comprising a tetravalent dengue virus composition comprising a live attenuated dengue virus serotype 1, a live attenuated dengue virus serotype 2, a live attenuated dengue virus serotype 3, and a live attenuated dengue virus serotype 4 (e.g. TDV-1, TDV-2, TDV-3 and TDV-4, i.e. "TAK-003"), at least one non-reducing disaccharide (e.g. trehalose and/or sucrose), at least one poloxamer (e.g. poloxamer 407), urea, at least one amino acid having a positively charged side chain at neutral pH (e.g. arginine, lysine and/or histidine), tromethamine (e.g. tromethamine in its free base form and tromethamine hydrochloride), and human serum albumin.

The dengue vaccine formulation of the invention comprises a tetravalent dengue virus composition comprising: a live attenuated dengue virus serotype 1, a live attenuated dengue virus serotype 2, a live attenuated dengue virus serotype 3, and a live attenuated dengue virus serotype 4 (e.g. TDV-1, TDV-2, TDV-3 and TDV-4, i.e. "TAK-003") and an excipient composition comprising trehalose, poloxamer, urea, arginine hydrochloride, tromethamine and human serum albumin, wherein the dengue vaccine formulation comprises the trehalose at an amount of 20 to 34 mg/dose, the poloxamer at an amount of 0.1 to 3.0 mg/dose, the urea at an amount of 0.1 to 3.0 mg/dose, the arginine hydrochloride at an amount of 0.1 to 2.0 mg/dose, the tromethamine at an amount of 0.05 to 1.0 mg/dose, and the human serum albumin at an amount of 0.05 to 0.5 mg/dose. In certain embodiments, the dengue vaccine formulation of the invention comprises sucrose. In certain other embodiments, the dengue vaccine formulation of the invention is free of sucrose.

In certain embodiments, the dengue vaccine formulation of the invention is free of mannitol and/or sorbitol.

In a preferred embodiment, the dengue vaccine formulation of the invention comprises TDV-1, TDV-2, TDV-3 and TDV-4 (i.e. "TAK-003") and an excipient composition comprising trehalose, poloxamer, urea, arginine hydrochloride, tromethamine and human serum albumin, wherein the dengue vaccine formulation comprises the trehalose at an amount of 20 to 34 mg/dose, the poloxamer at an amount of 0.1 to 3.0 mg/dose, the urea at an amount of 0.1 to 3.0 mg/dose, the arginine hydrochloride at an amount of 0.1 to 2.0 mg/dose, the tromethamine at an amount of 0.05 to 1.0 mg/dose, and the human serum albumin at an amount of 0.05 to 0.5 mg/dose.

In one embodiment, the dengue vaccine formulation of the invention as described herein comprises, in addition to the excipient composition disclosed in the preceding paragraph, phosphate salts, such as potassium dihydrogen phosphate or disodium hydrogen phosphate.

In one embodiment, the dengue vaccine formulation of the invention as described herein comprises, in addition to the excipient composition disclosed in the two preceding paragraphs, chloride salts, such as sodium chloride or potassium chloride. "Acid addition salts" such as arginine hydrochloride or tromethamine hydrochloride are no chloride salts within the meaning of the present invention. In one embodiment, the dengue vaccine formulation as described herein additionally comprises hydrochloric acid.

### Dry formulations

In one aspect of the invention the dengue vaccine formulation of the invention as described herein is in the form of a dry formulation, namely a dengue vaccine formulation in dry form. A dengue vaccine formulation in dry form may be obtained by lyophilization of a dengue vaccine formulation in aqueous form. The dry formulation can be stored and reconstituted with a pharmaceutically acceptable aqueous diluent, such as water for injection or 37 mM aqueous sodium chloride solution, prior to administration. It is preferred that the lyophilized dengue vaccine formulation is the final product after manufacture and the storage form of the dengue vaccine formulation.

In a preferred embodiment, the dengue vaccine formulation in dry form has a residual moisture content as determined by Karl Fischer Determination of equal to or less than 5.0% w/w, preferably equal to or less than 3.0% w/w. In certain embodiments the residual moisture content ranges from 0.1% w/w to 5% w/w or 0.1% w/w to 3% w/w or 0.1% w/w to 2% w/w or less than 2% w/w or 0.1% w/w to 1% w/w. In a particularly preferred embodiment, the dengue vaccine formulation in dry form has a residual moisture content of less than 1 % w/w, more preferably less than 0.9 % w/w, or less than 0.8 % w/w.

In one embodiment, a lyophilized dengue vaccine formulation is obtained by subjecting the aqueous dengue vaccine formulation as described herein to lyophilization. In a preferred embodiment, the lyophilized dengue vaccine formulation has a residual moisture content as determined by Karl Fischer Determination of equal to or less than 5.0% w/w, preferably equal to or less than 3.0% w/w. In certain embodiments the residual moisture content ranges from 0.1% w/w to 5% w/w or 0.1% w/w to 3% w/w or 0.1% w/w to 2% w/w or 0.1% w/w to 1% w/w. In a particularly preferred embodiment, the lyophilized dengue vaccine formulation has a residual moisture content of less than 1 % w/w, more preferably less than 0.9 % w/w, or less than 0.8 % w/w.

In one embodiment, a lyophilized unit dose is obtained by subjecting a volume of 0.5 mL of the aqueous dengue vaccine formulation as described herein to lyophilization. In a preferred embodiment, a lyophilized unit dose is obtained by subjecting a volume of about 0.25 mL of the aqueous dengue vaccine formulation as described herein to lyophilization.

In one embodiment, a lyophilized unit dose is present in a container, preferably a vial, and said unit dose is administered to a subject after reconstitution. In one embodiment, more than one unit dose (e.g. five unit doses) of the dengue vaccine composition are present in a container, preferably a vial, so that with the content of one container, preferably a vial, more than one subject (e.g. five subjects) can be vaccinated. In one embodiment, a container, preferably a vial, comprising more than one unit dose (e.g. five unit doses) of the dengue vaccine formulation as described herein is used for providing the reconstituted unit dose to be used in the methods of the invention as described herein.

In certain embodiments, the container comprising one or more unit dose(s) (e.g. one to ten unit doses) of the dengue vaccine formulation of the invention is part of a kit. Thus, the invention is directed in part to a kit for preparing a reconstituted dengue vaccine formulation comprising a dengue vaccine formulation as described herein in dry form (e.g. a lyophilized dengue vaccine formulation), and a pharmaceutically acceptable diluent for reconstitution.

In certain embodiments, the diluent for reconstitution is provided in a container, preferably a vial, or a pre-filled syringe. In some embodiments, the diluent for reconstitution is selected from water for injection, phosphate buffered saline or an aqueous sodium chloride solution. In a preferred embodiment, the diluent for reconstitution is 30 to 40 mM sodium chloride, such as 37 mM aqueous sodium chloride solution.

In one embodiment of the invention when a unit dose of a dengue vaccine formulation as described herein in dry form is part of a kit for preparing a unit dose of a dengue vaccine formulation in reconstituted form, an aliquot (e.g. 0.5 mL/unit dose) of a pharmaceutically acceptable aqueous diluent (e.g. aqueous sodium chloride solution) for reconstitution is provided in a container, preferably a vial. In one embodiment of the invention when five unit doses of a the dengue vaccine formulation in dry form are part of a kit, five aliquots (e.g. 2.5 mL) of a pharmaceutically acceptable aqueous diluent (e.g. aqueous sodium chloride solution) are provided in a container, preferably a vial. Accordingly, in certain embodiments, a kit for preparing multiple unit doses of a dengue vaccine formulation in reconstituted form comprises a) a container, such as a vial, comprising multiple unit doses of the dengue vaccine formulation as described herein in dry form, and b) a container, such as a vial, comprising multiple aliquots of a pharmaceutically acceptable aqueous diluent for reconstitution, wherein the number of aliquots correspond to the number of unit doses.

In one such embodiment, an aliquot has a volume of about 0.4 to 0.8 mL/unit dose, preferably of about 0.4 to 0.7 mL/unit dose, more preferably of about 0.45 to 0.65 mL/unit dose, such as 0.5 mL/unit dose.

Also contemplated within the present invention are kits of parts comprising one or more than one dengue vaccine formulation containers, such as vials, as described in the preceding paragraphs. In certain such embodiments, a kit comprises multiple, such as ten, dengue vaccine formulation vials, wherein each vial comprises five unit doses of the dengue vaccine formulation as described herein. In one embodiment, a kit comprises one or more diluent container(s) and one or more dengue vaccine formulation container(s), such as vials. In certain such embodiments, a kit comprises ten dengue vaccine formulation vials, wherein each vial comprises five unit doses of the dengue vaccine formulation as described herein and more than one diluent container for preparing fifty unit doses of a dengue vaccine formulation in reconstituted form.

### Aqueous formulations

In one aspect, the dengue vaccine formulation of the invention as described herein is a liquid formulation, namely a dengue vaccine formulation in aqueous form. A dengue vaccine formulation in aqueous form may be subject to a drying procedure, such as lyophilization, to obtain a dengue vaccine formulation in dry form.

In certain such embodiments, the dengue vaccine formulation in aqueous form that is subjected to a drying procedure, such as lyophilization, comprises about 5 to 15 % w/v, preferably about 6 to 12 % w/v, more preferably about 8 to 11 % w/v trehalose (e.g. about 9 % w/v α,α-trehalose based on 10 % w/v α,α-trehalose dihydrate), about 0.05 to 1.0 % w/v, preferably about 0.1 to 0.5 % w/v, more preferably about 0.2 to 0.4 % w/v poloxamer (e.g. about 0.3 % w/v Kolliphor P407), about 0.05 to 1.0 % w/v, preferably about 0.1 to 0.5 % w/v, more preferably about 0.2 to 0.4 % w/v urea (e.g. about 0.3 % w/v urea), about 0.05 to 1.0 % w/v, preferably 0.1 to 0.5 % w/v, more preferably about 0.2 to 0.3 % w/v arginine hydrochloride (e.g. about 0.25 % w/v L-Arginine hydrochloride), about 0.01 to 0.5 % w/v, preferably about 0.02 to 0.5 % w/v, more preferably about 0.05 to 0.15 % w/v tromethamine (e.g. about 0.12 % w/v tromethamine in the form of the free base, or about 0.02 % w/v tromethamine in combination with about 0.13 % w/v tromethamine HCl, i.e. about 10 mM tromethamine), and about 0.01 to 0.5 % w/v, preferably about 0.02 to 0.5 % w/v, more preferably about 0.05 to 0.15 % w/v human serum albumin (e.g. about 0.1 % w/v human serum albumin).

In certain such embodiments, small amounts of phosphate salts and chloride salts are contained in the dengue vaccine formulation as described herein. For example, the dengue vaccine formulation in aqueous form that is subjected to a drying procedure, such as lyophilization, comprises less than 0.1 % w/v, preferably about 0.01 to 0.06 % w/v, more preferably about 0.01 to 0.04 % w/v phosphate salts (e.g. about 0.028 % w/v disodium hydrogen phosphate dihydrate and 0.004 % w/v potassium dihydrogen phosphate), and less than 0.5 % w/v, preferably about 0.01 to 0.3 % w/v, more preferably about 0.01 to 0.25 % w/v chloride salts (e.g. about 0.22 % w/v sodium chloride and 0.003 % w/v potassium chloride).

A reduced amount of chloride salts helps in reducing the time for lyophilizing the dengue vaccine formulation as described herein.

In certain such embodiments, the dengue vaccine formulation in aqueous form that is subjected to a drying procedure, such as lyophilization, comprises about 9 to 11 % w/v, such as about 10 % w/v trehalose, about 0.2 to 0.4 % w/v, such as about 0.3 % w/v poloxamer, about 0.2 to 0.4 % w/v, such as about 0.3 % w/v urea, about 0.2 to 0.3 % w/v, such as about 0.25 % w/v arginine hydrochloride, about 0.05 to 0.15 % w/v, such as about 0.1 % w/v tromethamine, and about 0.05 to 0.15 % w/v, such as about 0.1 % w/v human serum albumin.

In certain such embodiments, the pH of the dengue vaccine formulation in aqueous form is from about 7.0 to about 8.5, preferably of about 7.3 to 8.0, more preferably of about 7.4 to 7.6, such as about 7.4, or about 7.5, or about 7.6, according to the USP <791> test method for pH measurements of the United States Pharmacopeial Convention.

Hydrochloric acid may be used for pH adjustment. Thus, in one embodiment, hydrochloric acid is contained in the dengue vaccine formulation as described herein. In other embodiments, preferably in embodiments wherein the tromethamine is provided by a combination of tromethamine and tromethamine HCl, no hydrochloric acid is added to the dengue vaccine formulation as described herein. Tromethamine HCl may be used for pH adjustment without the need for hydrochloric acid. Thus, in a preferred embodiment, tromethamine HCl is used to adjust the pH.

A dengue vaccine formulation in aqueous form may also be formed from a dengue vaccine formulation in dry form as a dengue vaccine formulation in reconstituted form. In one embodiment, a reconstituted unit dose is obtained by subjecting the lyophilized unit dose to reconstitution with a pharmaceutically acceptable diluent, preferably before administration of the dengue vaccine.

In certain such embodiments, reconstitution will be accomplished by adding a pharmaceutically acceptable diluent, such as water for injection, or an aqueous sodium chloride solution, to the lyophilized unit dose. In one embodiment, an aqueous sodium chloride solution, such as a 37 mM aqueous sodium chloride solution, is added to the lyophilized unit dose for reconstitution.

Thus, in one embodiment, a dengue vaccine formulation in aqueous form is obtainable by reconstituting the dengue vaccine formulation as described herein in dry form with a pharmaceutically acceptable aqueous diluent.

In one embodiment, a unit dose of a dengue vaccine formulation in aqueous form is obtainable by reconstituting a unit dose of the dengue vaccine formulation as described herein in dry form with an aliquot of a pharmaceutically acceptable aqueous diluent.

In one embodiment, a unit dose of a dengue vaccine formulation in aqueous form is obtainable by reconstituting multiple unit doses (e.g. five unit doses) of the dengue vaccine formulation as described herein in dry form with multiple aliquots (e.g. five aliquots) of a pharmaceutically acceptable aqueous diluent, wherein the number of aliquots correspond to the number of unit doses, and removing 0.5 mL of the reconstituted dengue vaccine formulation.

In one embodiment, five unit doses of a dengue vaccine formulation in aqueous form are obtainable by reconstituting five unit doses of the dengue vaccine formulation as described herein in dry form with five aliquots of a pharmaceutically acceptable aqueous diluent.

In one embodiment, a container, preferably a vial, comprising the dengue vaccine formulation as described herein, is used for providing the reconstituted dengue vaccine formulation. In certain such embodiments, the container comprises one unit dose or more than one unit dose of the dengue vaccine formulation as described herein.

In a preferred embodiment, a container, preferably a vial, comprising multiple unit doses (e.g. at least five unit doses) of the dengue vaccine formulation as described herein, is used for providing the reconstituted dengue vaccine formulation to be used in the methods of the invention as described herein.

In one such embodiment, the lyophilized dengue vaccine formulation will be reconstituted with 0.4 to 0.8 mL/unit dose, or 0.4 to 0.7 mL/unit dose, or 0.45 to 0.65 mL/unit dose, such as 0.5 mL/unit dose of diluent. In a preferred embodiment, the lyophilized dengue vaccine formulation is reconstituted with 0.5 mL/unit dose of 37 mM aqueous sodium chloride solution. In another preferred embodiment, the lyophilized dengue vaccine formulation is reconstituted with 37 mM aqueous sodium chloride solution to a volume of 0.5 mL/unit dose. In one embodiment, the reconstituted unit dose to be administered subcutaneously or intramuscularly has a volume of 0.5 mL. The reconstituted dengue vaccine formulation confirms to physiological pH, osmolarity and tonicity considerations.

In certain such embodiments, the pH of the reconstituted formulation is from about 7.0 to about 8.5, such as about 7.4, or about 7.5, or about 7.6, according to the USP <791> test method for pH measurements of the United States Pharmacopeial Convention.

In one embodiment, a unit dose of the dengue vaccine formulation in dry form upon reconstitution with 0.5 mL of a pharmaceutically acceptable aqueous diluent has a pH at 25 °C of about 7.0 to 8.5, preferably of about 7.3 to 8.0, more preferably of about 7.4 to 7.8, or about 7.6.

In one embodiment, a unit dose of the dengue vaccine formulation in dry form upon reconstitution with a pharmaceutically acceptable aqueous diluent to a volume of 0.5 mL has a pH at 25 °C of about 7.0 to 8.5, preferably of about 7.0 to 8.0, more preferably of about 7.3 to 7.8, or about 7.4.

In certain such embodiments, the osmolality of the reconstituted formulation is from about 100 to about 700 mOsmol/kg, such as about 300 mOsmol/kg according to the USP <785> test method for osmolality measurements of the United States Pharmacopeial Convention.

In one embodiment, a unit dose of the dengue vaccine formulation in dry form upon reconstitution with 0.5 mL of a pharmaceutically acceptable aqueous diluent has an osmolality of about 100 to 700 mOsmol/kg, preferably of about 100 to less than 700 mOsmol/kg, more preferably of about 100 to 400 mOsmol/kg, or about 300 mOsmol/kg.

In one embodiment, a unit dose of the dengue vaccine formulation in dry form upon reconstitution with a pharmaceutically acceptable aqueous diluent to a volume of 0.5 mL has an osmolality of about 100 to 700 mOsmol/kg, preferably of about 100 to less than 700 mOsmol/kg, more preferably of about 100 to 500 mOsmol/kg.

### Excipient composition

The dengue vaccine formulation of the invention comprises a combination of trehalose, at least one poloxamer, urea, arginine hydrochloride, tromethamine and human serum albumin, wherein the dengue vaccine formulation comprises the trehalose at an amount of 20 to 34 mg/dose, the poloxamer at an amount of 0.1 to 3.0 mg/dose, the urea at an amount of 0.1 to 3.0 mg/dose, the arginine hydrochloride at an amount of 0.1 to 2.0 mg/dose, the tromethamine at an amount of 0.05 to 1.0 mg/dose, and the human serum albumin at an amount of 0.05 to 0.5 mg/dose.

The tromethamine may be used in its free base form or, e.g., in the form of an acid addition salt (e.g., tromethamine hydrochloride, also known as Tris-HCl), or as a combination of two forms. In a preferred embodiment, a mixture of tromethamine (base) and tromethamine hydrochloride is used in the preparation. Unless otherwise indicated, the thromethamine contained in the dengue vaccine formulation is calculated based on tromethamine in the form of the free base. E.g., when a) 0.01 mmol (equal to 1.21 mg) tromethamine or b) 0.01 mmol (equal to 1.58 mg) tromethamine hydrochloride is used in the preparation, the amount of tromethamine in the dengue vaccine formulation is, within the meaning of the invention, in both cases 1.21 mg, i.e. 0.01 mmol.

In particular the presence of tromethamine, urea, and arginine hydrochloride helps in improving the stability of the human serum albumin-containing dengue vaccine formulation as described herein. The presence of tromethamine helps particularly in avoiding pH changes during freezing. PH changes contribute to the loss of potency for the virus due to conformational changes in the virus structure.

In one embodiment, tromethamine is contained in the dengue vaccine formulation at an amount of about 0.05 to 1.0 mg/unit dose, preferably about 0.1 to 0.6 mg/unit dose, more preferably about 0.2 to 0.5 mg/unit dose. That is, in case the unit dose is administered in a volume of 0.5 mL, the tromethamine contained in the dengue vaccine formulation is about 0.05 to 1.0 mg/0.5 mL, preferably about 0.1 to 0.6 mg/0.5 mL, more preferably about 0.2 to 0.5 mg/0.5 mL.

In one embodiment, tromethamine is contained in the dengue vaccine formulation at an amount of about 0.1 to 10.0 µmol/unit dose, preferably at an amount of 0.1 to 6.0 µmol/unit dose, more preferably at an amount of 1.0 to 3.0 µmol/unit dose. That is, in case the unit dose is administered in a volume of 0.5 mL, the tromethamine contained in the dengue vaccine formulation is about 0.1 to 10.0 µmol/0.5 mL, preferably at an amount of 0.1 to 6.0 µmol/0.5 mL, more preferably at an amount of 1.0 to 3.0 µmol/0.5 mL.

In one embodiment, tromethamine is contained in the dengue vaccine formulation at an amount of about 0.05 to 5.0 % w/w, preferably about 0.1 to 3.0 % w/w, more preferably about 0.5 to 2.0 % w/w.

The trehalose may be α,α-trehalose, the poloxamer may be poloxamer 407, the arginine hydrochloride may be L-arginine hydrochloride, the human serum albumin may be a native human serum albumin, or may comprise recombinant human serum albumin (rHSA).

In a preferred embodiment, the poloxamer is poloxamer 407. A poloxamer is a non-ionic triblock copolymer composed of a central hydrophobic chain of poly(propyleneoxide) flanked by two hydrophilic chains of poly(ethylene oxide). The length of the polymer blocks can be customized, leading to different poloxamers with slightly different properties. "Poloxamer 407" as used herein is a hydrophilic non-ionic surfactant which consists of a triblock copolymer consisting of a central propylene glycol block with about 56 repeat units and two flanking hydrophilic polyethylene glycol blocks each comprising about 101 repeat units. "Poloxamer 407" is also known by its trade names Pluronic F127 and Synperonic PE/F127.

In one embodiment, the ratio of the amount of arginine hydrochloride to the amount of human serum albumin contained in the dengue vaccine formulation is about 1:1 to 4:1, preferably about 2:1 to 3:1.

In one embodiment, the ratio of the amount of arginine hydrochloride to the amount of tromethamine contained in the dengue vaccine formulation is about 1:1 to 3:1, preferably about 1.5:1 to 2.5:1.

In one embodiment, the ratio of the amount of urea to the amount of human serum albumin contained in the dengue vaccine formulation is about 1:1 to 5:1, preferably about 2:1 to 4:1.

In one embodiment, the ratio of the amount of urea to the amount of arginine hydrochloride contained in the dengue vaccine formulation is about 1:1 to 2:1.

In one embodiment, the ratio of the amount of urea to the amount of poloxamer contained in the dengue vaccine formulation is about 1:1 to 2:1.

In one embodiment, the ratio of the amount of trehalose to the amount of urea contained in the dengue vaccine formulation is about 20:1 to 40:1, preferably about 25:1 to 35:1.

In one embodiment, the ratio of the amount of arginine hydrochloride to the amount of human serum albumin contained in the dengue vaccine formulation is about 1:1 to 4:1, and human serum albumin is contained in the dengue vaccine formulation as described herein at an amount of about 0.1 to 1.0 % w/w based on the dry weight of the vaccine formulation.

In one embodiment, the dengue vaccine formulation comprises trehalose, poloxamer, urea, arginine hydrochloride, tromethamine, human serum albumin, chloride salts and phosphate salts, wherein the dengue vaccine formulation comprises the trehalose at an amount of 20 to 34 mg/dose, the poloxamer at an amount of 0.1 to 3.0 mg/dose, the urea at an amount of 0.1 to 3.0 mg/dose, the arginine hydrochloride at an amount of 0.1 to 2.0 mg/dose, the tromethamine at an amount of 0.05 to 1.0 mg/dose, and the human serum albumin at an amount of 0.05 to 0.5 mg/dose. Preferably, the chloride salts comprise or consist of sodium chloride and potassium chloride. Preferably, the phosphate salts comprise or consist of potassium dihydrogen phosphate and disodium hydrogen phosphate. Most preferably, the excipients are a combination consisting of α,α-trehalose dihydrate, poloxamer 407 (Kolliphor^{®} P407 (F-127)), urea, L-arginine hydrochloride, tromethamine, Tris-HCl, human serum albumin, potassium dihydrogen phosphate, disodium hydrogen phosphate dihydrate, potassium chloride and sodium chloride.

In certain such embodiments, chloride salts are contained in the dengue vaccine formulation at an amount of less than 4.0 % w/w based on the dry weight of the vaccine formulation, phosphate salts are contained in the dengue vaccine formulation at an amount of less than 1.0 % w/w based on the dry weight of the vaccine formulation, and the ratio of the amount of arginine hydrochloride to the amount of human serum albumin contained in the dengue vaccine formulation is about 1:1 to 4:1.

In certain embodiments, phosphate salts are contained in the dengue vaccine formulation at an amount of less than 1.0 % w/w, or about 0.05 to 0.5 % w/w, preferably about 0.2 to 0.4 % w/w, more preferably 0.3 % w/w or less, based on the dry weight of the vaccine formulation.

In certain embodiments, chloride salts are contained in the dengue vaccine formulation at an amount of less than 4.0 % w/w, or about 0.05 to 3.0 % w/w, based on the dry weight of the vaccine formulation.

In one embodiment, hydrochloric acid is contained in the dengue vaccine formulation at an amount of less than 1.0 % w/w, or about 0.05 to 0.5 % w/w, preferably about 0.2 to 0.4 % w/w, more preferably about 0.3 % w/w or less based on the dry weight of the vaccine formulation.

In one embodiment, the arginine hydrochloride is contained in the dengue vaccine formulation at an amount of about 0.1 to 5.0 % w/w arginine hydrochloride, the urea is contained in the dengue vaccine formulation at an amount of about 0.1 to 6.0 % w/w urea, the human serum albumin is contained in the dengue vaccine formulation at an amount of about 0.05 to 3.0 % w/w, the trehalose is contained in the dengue vaccine formulation at an amount of about 65 to 95 % w/w, the poloxamer is contained in the dengue vaccine formulation at an amount of about 0.05 to 5.0 % w/w, and the tromethamine is contained in the dengue vaccine formulation at an amount of about 0.05 to 5.0 % w/w, based on the dry weight of the vaccine formulation. In certain such embodiments, phosphate salts may be contained in the dengue vaccine formulation at an amount of less than 1.0 % w/w, or about 0.05 to 0.5 % w/w, and/or chloride salts may be contained in the dengue vaccine formulation at an amount of less than 4.0 % w/w, or about 0.05 to 3.0 % w/w, based on the dry weight of the vaccine formulation.

In certain preferred embodiments, the arginine hydrochloride is contained in the dengue vaccine formulation at an amount of about 0.5 to 5.0 % w/w, the urea is contained in the dengue vaccine formulation at an amount of about 0.5 to 5.0 % w/w, the human serum albumin is contained in the dengue vaccine formulation at an amount of about 0.1 to 2.0 % w/w, the trehalose is contained in the dengue vaccine formulation at an amount of about 70 to 95 % w/w, the poloxamer is contained in the dengue vaccine formulation at an amount of about 0.1 to 5.0 % w/w, and the tromethamine is contained in the dengue vaccine formulation at an amount of about 0.1 to 3.0 % w/w, based on the dry weight of the vaccine formulation. In certain such embodiments, phosphate salts may be contained in the dengue vaccine formulation at an amount of about 0.2 to 0.4 % w/w, and/or chloride salts may be contained in the dengue vaccine formulation at an amount of less than 4.0 % w/w, or about 0.05 to 3.0 % w/w, based on the dry weight of the vaccine formulation.

In further more preferred embodiments, the arginine hydrochloride is contained in the dengue vaccine formulation at an amount of about 1.0 to 4.0 % w/w, the urea is contained in the dengue vaccine formulation at an amount of about 1.0 to 4.0 % w/w, the human serum albumin is contained in the dengue vaccine formulation at an amount of about 0.1 to 1.5 % w/w, the trehalose is contained in the dengue vaccine formulation at an amount of about 75 to 95 % w/w, the poloxamer is contained in the dengue vaccine formulation at an amount of about 0.5 to 4.0 % w/w, and the tromethamine is contained in the dengue vaccine formulation at an amount of about 0.5 to 2.0 % w/w, based on the dry weight of the vaccine formulation. In certain such embodiments, phosphate salts may be contained in the dengue vaccine formulation at an amount of 0.3 % w/w or less, and/or chloride salts may be contained in the dengue vaccine formulation at an amount of less than 4.0 % w/w, or about 0.05 to 3.0 % w/w, based on the dry weight of the vaccine formulation.

In particularly preferred embodiments, the arginine hydrochloride is contained in the dengue vaccine formulation at an amount of about 2.0 to 3.0 % w/w, such as about 2.4 % w/w, the urea is contained in the dengue vaccine formulation at an amount of about 2.0 to 4.0% w/w, such as about 2.9% w/w, the human serum albumin is contained in the dengue vaccine formulation at an amount of about 0.1 to 1.0 % w/w, or 1.0 % w/w or less, the trehalose is contained in the dengue vaccine formulation at an amount of at least 80 % w/w, such as 80 to 95 % w/w, or 80 to 90 % w/w, or 85 to 95 % w/w, or 85 to 90 % w/w, or about 87 % w/w, the poloxamer is contained in the dengue vaccine formulation at an amount of about 2.9% w/w or less, and the tromethamine is contained in the dengue vaccine formulation at an amount of less than 2.0 % w/w, such as about 1.2 % w/w, based on the dry weight of the vaccine formulation. In certain such embodiments, phosphate salts may be contained in the dengue vaccine formulation at an amount of 0.3 % w/w or less, and/or chloride salts may be contained in the dengue vaccine formulation at an amount of less than 4.0 % w/w, or about 0.05 to 3.0 % w/w, based on the dry weight of the vaccine formulation.

In particularly preferred embodiments, the arginine hydrochloride is contained in the dengue vaccine formulation at an amount of about 2.0 to 3.0 % w/w, such as about 2.4 % w/w, the urea is contained in the dengue vaccine formulation at an amount of about 2.0 to 4.0 % w/w, such as about 2.9% w/w, the human serum albumin is contained in the dengue vaccine formulation at an amount of about 0.5 to 1.5 % w/w, such as about 1.0% w/w, the trehalose is contained in the dengue vaccine formulation at an amount of about 85 to 90 % w/w, such as about 87 % w/w, the poloxamer is contained in the dengue vaccine formulation at an amount of about 2.0 to 4.0% w/w, such as about 2.9% w/w, and the tromethamine is contained in the dengue vaccine formulation at an amount of about 0.5 to 2.0 % w/w, such as about 1.2 % w/w, based on the dry weight of the vaccine formulation. In certain such embodiments, phosphate salts may be contained in the dengue vaccine formulation at an amount of about 0.2 to 0.4 % w/w, such as about 0.3 % w/w and/or chloride salts may be contained in the dengue vaccine formulation at an amount of about 1.0 to 3.0 % w/w, such as about 1.9 % w/w, based on the dry weight of the vaccine formulation.

In one embodiment, sodium chloride is contained in the dengue vaccine formulation at an amount of about 1.0 to 3.0 % w/w, preferably 2.1 % w/w or less, such as 1.9 % w/w, based on the dry weight of the vaccine formulation. In one embodiment, potassium chloride is contained in the dengue vaccine formulation at an amount of about 0.01 to 0.05 % w/w, preferably 0.03 % w/w or less based on the dry weight of the vaccine formulation.

In one embodiment, the arginine hydrochloride is contained in the dengue vaccine formulation at an amount of about 0.1 to 2.0 mg/unit dose, the urea is contained in the dengue vaccine formulation at an amount of about 0.1 to 3.0 mg/unit dose, the human serum albumin is contained in the dengue vaccine formulation at an amount of about 0.05 to 0.5 mg/unit dose, the trehalose is contained in the dengue vaccine formulation at an amount of about 20 to 34 mg/unit dose, the poloxamer is contained in the dengue vaccine formulation at an amount of about 0.1 to 3.0 mg/unit dose, and the tromethamine is contained in the dengue vaccine formulation at an amount of about 0.05 to 1.0 mg/unit dose. In certain such embodiments, phosphate salts may be contained in the dengue vaccine formulation at an amount of less than 0.3 mg/unit dose, or about 0.01 to 0.2 mg/unit dose, and/or chloride salts may be contained in the dengue vaccine formulation at an amount of less than 2.0 mg/unit dose.

In certain preferred embodiments, the arginine hydrochloride is contained in the dengue vaccine formulation at an amount of about 0.2 to 1.5 mg/unit dose, or about 0.5 to 1.2 mg/unit dose, the urea is contained in the dengue vaccine formulation at an amount of about 0.1 to 2.0 mg/unit dose, or about 0.3 to 1.5 mg/unit dose, the human serum albumin is contained in the dengue vaccine formulation at an amount of about 0.1 to 0.4 mg/unit dose, the trehalose is contained in the dengue vaccine formulation at an amount of about 22 to 32 mg/unit dose, the poloxamer is contained in the dengue vaccine formulation at an amount of about 0.3 to 1.5 mg/unit dose, and the tromethamine is contained in the dengue vaccine formulation at an amount of about 0.1 to 0.6 mg/unit dose. In certain such embodiments, phosphate salts may be contained in the dengue vaccine formulation at an amount of about 0.08 to 0.1 mg/unit dose, and/or chloride salts may be contained in the dengue vaccine formulation at an amount of about 0.1 to 1.0 mg/unit dose.

In more preferred embodiments, the arginine hydrochloride is contained in the dengue vaccine formulation at an amount of about 0.5 to 1.0 mg/unit dose, the urea is contained in the dengue vaccine formulation at an amount of about 0.6 to 1.2 mg/unit dose, the human serum albumin is contained in the dengue vaccine formulation at an amount of less than 0.4 mg/unit dose, the trehalose is contained in the dengue vaccine formulation at an amount of about 23 to 30 mg/unit dose, the poloxamer is contained in the dengue vaccine formulation at an amount of about 0.6 to 1.2 mg/unit dose, and the tromethamine is contained in the dengue vaccine formulation at an amount of about 0.2 to 0.5 mg/unit dose. In certain such embodiments, phosphate salts may be contained in the dengue vaccine formulation at an amount of 0.1 mg/unit dose or less, and/or chloride salts may be contained in the dengue vaccine formulation at an amount of about 0.55 to 0.75 mg/unit dose.

In particularly preferred embodiments, the arginine hydrochloride is contained in the dengue vaccine formulation at an amount of about 0.6 to 0.8 mg/unit dose, the urea is contained in the dengue vaccine formulation at an amount of about 0.75 to 1.0 mg/unit dose, the human serum albumin is contained in the dengue vaccine formulation at an amount of about 0.25 to 0.33 mg/unit dose, or 0.3 mg/unit dose or less, the trehalose is contained in the dengue vaccine formulation at an amount of about 23 to 27 mg/unit dose, or 27 mg/unit dose or less, the poloxamer is contained in the dengue vaccine formulation at an amount of about 0.75 to 1.0 mg/unit dose, or 1.0 mg/unit dose or less, and the tromethamine is contained in the dengue vaccine formulation at an amount of about 0.3 to 0.4 mg/unit dose, or 0.4 mg/unit dose or less. In certain such embodiments, phosphate salts may be contained in the dengue vaccine formulation at an amount of 0.1 mg/unit dose or less, and/or chloride salts may be contained in the dengue vaccine formulation at an amount of about 0.55 to 0.75 mg/unit dose.

In most preferred embodiments, the arginine hydrochloride is contained in the dengue vaccine formulation at an amount of about 0.5 to 0.7 mg/dose, the urea is contained in the dengue vaccine formulation at an amount of about 0.6 to 0.8 mg/dose, the human serum albumin is contained in the dengue vaccine formulation at an amount of about 0.2 to 0.3 mg/dose, the trehalose is contained in the dengue vaccine formulation at an amount of about 19 to 24 mg/dose (corresponding to about 21 to 27 mg/dose α,α-trehalose dihydrate), the poloxamer is contained in the dengue vaccine formulation at an amount of about 0.6 to 0.8 mg/dose, and the tromethamine is contained in the dengue vaccine formulation at an amount of about 0.2 to 0.4 mg/dose (for example, about 0.2 to 0.35 mg/dose tromethamine-HCl and about 0.02 to 0.08 mg/dose tromethamine in the form of the free base). In certain such embodiments, phosphate salts may be contained in the dengue vaccine formulation at an amount of 0.1 mg/dose or less, such as about 0.06 to 0.1 mg/dose, and/or chloride salts may be contained in the dengue vaccine formulation at an amount of about 0.4 to 0.6 mg/dose. In a preferred embodiment, the dose is administered in a volume of 0.5 mL.

In one embodiment, sodium chloride is contained in the dengue vaccine formulation as at an amount of 0.72 mg/unit dose or less. In one embodiment, potassium chloride is contained in the dengue vaccine formulation at an amount of 0.01 mg/unit dose or less.

In one embodiment, hydrochloric acid is contained in the dengue vaccine formulation at an amount of about 0.01 to 0.5 mg/unit dose, preferably less than 0.2 mg/unit dose, or about 0.07 to 0.1 mg/unit dose, more preferably 0.1 mg/unit dose or less.

In one embodiment, a unit dose of the dengue vaccine formulation in dry form upon reconstitution with 0.5 mL of a pharmaceutically acceptable aqueous diluent comprises the arginine hydrochloride in a concentration of about 0.5 to 5.0 g/L, the urea in a concentration of about 0.1 to 6.0 g/L, the human serum albumin in a concentration of from about 0.1 to less than 1 g/L, the trehalose in a concentration of from about 40 to 80 g/L, the poloxamer in a concentration of about 0.1 to 5.0 g/L, and the tromethamine in a concentration of about 0.1 to 1.5 g/L. In certain such embodiments, a unit dose of the dengue vaccine formulation in dry form upon reconstitution with 0.5 mL of a pharmaceutically acceptable aqueous diluent may further comprise phosphate salts in a concentration of less than 0.3 g/L, or about 0.16 to 0.22 g/L, and/or chloride salts in a concentration of less than 5 g/L.

In one embodiment, a unit dose of the dengue vaccine formulation in dry form upon reconstitution with a pharmaceutically acceptable aqueous diluent to a volume of 0.5 mL comprises the arginine hydrochloride in a concentration of about 0.5 to 5.0 g/L, the urea in a concentration of about 0.1 to 6.0 g/L, the human serum albumin in a concentration of from about 0.1 to less than 1 g/L, the trehalose in a concentration of from about 40 to 80 g/L, the poloxamer in a concentration of about 0.1 to 5.0 g/L, and the tromethamine in a concentration of about 0.1 to 1.5 g/L. In certain such embodiments, a unit dose of the dengue vaccine formulation in dry form upon reconstitution with a pharmaceutically acceptable aqueous diluent to a volume of 0.5 mL may further comprise phosphate salts in a concentration of less than 0.3 g/L, or about 0.13 to 0.22 g/L, and/or chloride salts in a concentration of less than 5 g/L.

In a preferred embodiment, a unit dose of the dengue vaccine formulation in dry form upon reconstitution with 0.5 mL of a pharmaceutically acceptable aqueous diluent comprises the arginine hydrochloride in a concentration of about 0.5 to less than 2.5 g/L, the urea in a concentration of about 0.5 to less than 3.0 g/L, the human serum albumin in a concentration of about 0.3 to 0.9 g/L, the trehalose in a concentration of about 40 to 70 g/L, the poloxamer in a concentration of about 0.5 to less than 3.0 g/L, and the tromethamine in a concentration of about 0.5 to less than 1.2 g/L. In certain such embodiments, a unit dose of the dengue vaccine formulation in dry form upon reconstitution with 0.5 mL of a pharmaceutically acceptable aqueous diluent may further comprise phosphate salts in a concentration of 0.22 g/L or less, such as about 0.18 g/L, and/or chloride salts in a concentration of about 3.3 to 3.6 g/L.

In a preferred embodiment, a unit dose of the dengue vaccine formulation in dry form upon reconstitution with a pharmaceutically acceptable aqueous diluent to a volume of 0.5 mL comprises the arginine hydrochloride in a concentration of about 0.5 to less than 2.5 g/L, the urea in a concentration of about 0.5 to less than 3.0 g/L, the human serum albumin in a concentration of about 0.3 to 0.9 g/L, the trehalose in a concentration of about 40 to 70 g/L, the poloxamer in a concentration of about 0.5 to less than 3.0 g/L, and the tromethamine in a concentration of about 0.5 to less than 1.2 g/L. In certain such embodiments, a unit dose of the dengue vaccine formulation in dry form upon reconstitution with a pharmaceutically acceptable aqueous diluent to a volume of 0.5 mL may further comprise phosphate salts in a concentration of 0.22 g/L or less, such as about 0.13 g/L, and/or chloride salts in a concentration of about 3.1 to 3.6 g/L.

In a more preferred embodiment, a unit dose of the dengue vaccine formulation in dry form upon reconstitution with 0.5 mL of a pharmaceutically acceptable aqueous diluent comprises the arginine hydrochloride in a concentration of about 1.3 to 1.65 g/L, the urea in a concentration of about 1.5 to 2.0 g/L, the human serum albumin in a concentration of about 0.5 to 0.7 g/L, the trehalose in a concentration of about 45 to 60 g/L, the poloxamer in a concentration of about 1.5 to 2.0 g/L, and the tromethamine in a concentration of about 0.5 to 1.0 g/L. In certain such embodiments, a unit dose of the dengue vaccine formulation in dry form upon reconstitution with 0.5 mL of a pharmaceutically acceptable aqueous diluent may further comprise phosphate salts in a concentration of 0.22 g/L or less, such as about 0.18 g/L, and/or chloride salts in a concentration of 3.6 g/L or less such as about 3.4 g/L.

In an even more preferred embodiment, a unit dose of the dengue vaccine formulation in dry form upon reconstitution with a pharmaceutically acceptable aqueous diluent to a volume of 0.5 mL comprises the arginine hydrochloride in a concentration of about 1.0 to 1.65 g/L, the urea in a concentration of about 1.0 to 2.0 g/L, the human serum albumin in a concentration of about 0.4 to 0.7 g/L, the trehalose in a concentration of about 40 to 48 g/L (corresponding to about 44 to 53 g/L α,α-trehalose dihydrate), the poloxamer in a concentration of about 1.0 to 2.0 g/L, and the tromethamine in a concentration of about 0.5 to 1.0 g/L (for example, about 0.5 to 0.7 g/L tromethamine-HCl and about 0.05 to 0.2 g/L tromethamine in the form of the free base). In certain such embodiments, a unit dose of the dengue vaccine formulation in dry form upon reconstitution with a pharmaceutically acceptable aqueous diluent such as a 37 mM aqueous sodium chloride solution to a volume of 0.5 mL may further comprise phosphate salts in a concentration of 0.22 g/L or less, such as about 0.16 g/L, and/or chloride salts in a concentration of 3.6 g/L or less such as about 3.1 g/L.

In a particular preferred embodiment, a unit dose of the dengue vaccine formulation in dry form upon reconstitution with 0.5 mL of a pharmaceutically acceptable aqueous diluent comprises the arginine hydrochloride in a concentration of 1.65 g/L or less, such as about 1.4 g/L, the urea in a concentration of about 2.0 g/L or less, such as about 1.65 g/L, the human serum albumin in a concentration of about 0.7 g/L or less, such as about 0.6 g/L, the trehalose in a concentration of 60 g/L or less, such as about 55 g/L, the poloxamer in a concentration of about 2.0 g/L or less, such as about 1.65 g/L, and the tromethamine in a concentration of 0.8 g/L or less, such as about 0.7 g/L. In certain such embodiments, a unit dose of the dengue vaccine formulation in dry form upon reconstitution with 0.5 mL of a pharmaceutically acceptable aqueous diluent may further comprise phosphate salts in a concentration of 0.22 g/L or less, such as about 0.18 g/L, and/or chloride salts in a concentration of 3.6 g/L or less such as about 3.4 g/L.

In a most preferred embodiment, a unit dose of the dengue vaccine formulation in dry form upon reconstitution with a pharmaceutically acceptable aqueous diluent to a volume of 0.5 mL comprises the arginine hydrochloride in a concentration of 1.65 g/L or less, such as about 1.2 g/L, the urea in a concentration of about 2.0 g/L or less, such as about 1.4 g/L, the human serum albumin in a concentration of about 0.7 g/L or less, such as about 0.5 g/L, the trehalose in a concentration of 60 g/L or less, such as about 43 g/L, the poloxamer in a concentration of about 2.0 g/L or less, such as about 1.4 g/L, and the tromethamine in a concentration of 0.8 g/L or less, such as about 0.6 g/L. In certain such embodiments, a unit dose of the dengue vaccine formulation in dry form upon reconstitution with a pharmaceutically acceptable aqueous diluent such as a 37 mM aqueous sodium chloride solution to a volume of 0.5 mL may further comprise phosphate salts in a concentration of 0.22 g/L or less, such as about 0.13 g/L, and/or chloride salts in a concentration of 3.6 g/L or less such as about 3.1 g/L.

In one embodiment, a unit dose of the dengue vaccine formulation as described herein in dry form upon reconstitution with 0.5 mL of a pharmaceutically acceptable aqueous diluent comprises sodium chloride in a concentration of less than 5 g/L, preferably about 3.3 to 3.6 g/L, more preferably 3.6 g/L or less such as about 3.4 g/L.

In one embodiment, a unit dose of the dengue vaccine formulation as described herein in dry form upon reconstitution with a pharmaceutically acceptable aqueous diluent to a volume of 0.5 mL comprises sodium chloride in a concentration of less than 5 g/L, preferably about 3.1 to 3.6 g/L, more preferably 3.6 g/L or less such as about 3.1 g/L.

In one embodiment, a unit dose of the dengue vaccine formulation as described herein in dry form upon reconstitution with 0.5 mL of a pharmaceutically acceptable aqueous diluent comprises hydrochloric acid in a concentration of about 0.05 to 0.3 g/L, preferably about 0.15 to 0.19 g/L, more preferably 0.19 g/L or less, such as about 0.16 g/L.

In one embodiment, a unit dose of the dengue vaccine formulation as described herein in dry form upon reconstitution with a pharmaceutically acceptable aqueous diluent to a volume of 0.5 mL comprises hydrochloric acid in a concentration of about 0.05 to 0.3 g/L, preferably about 0.15 to 0.19 g/L, more preferably 0.19 g/L or less, such as about 0.14 g/L.

The excipient concentrations post reconstitution can also be expressed in quantity per dose. In a preferred embodiment, a dose to be administered in 0.5 mL contains or consists of the following excipients: α,α-trehalose dihydrate in an amount of 20 to 28 mg/dose, more preferably 22 to 26 mg/dose (e.g. 23.64 mg/dose), poloxamer (e.g. Kolliphor^{®} P407 (F127)) in an amount of 0.1 to 1.5 mg/dose, preferably 0.5 to 1.0 mg/dose, more preferably 0.6 to 0.8 mg/dose (e.g. 0.71 mg/dose), humans serum albumin in an amount of 0.05 to 0.5 mg/dose, preferably 0.05 to 0.4 mg/dose, more preferably 0.1 to 0.3 mg/dose (e.g. 0.24 mg/dose), tromethamine in an amount of 0.1 to 0.7 µmoles/dose, preferably 0.2 to 0.6 µmoles/dose, more preferably 0.3 to 0.5 µmoles/dose (e.g. 0.39 µmoles/dose), Tris HCl in an amount of 0.5 to 6.0 µmoles/dose, preferably 1.0 to 5.0 µmoles/dose, more preferably 1.0 to 3.0 µmoles/dose (e.g. 1.97 µmoles/dose), L-Arginine HCl in an amount of 0.5 to 8.0 µmoles/dose, preferably 1.0 to 6.0 µmoles/dose, more preferably 2.0 to 4.0 µmoles/dose (e.g. 2.81 µmoles/dose), Urea in an amount of 5.0 to 20.0 µmoles/dose, preferably 8.0 to 16.0 µmoles/dose, more preferably 10.0 to 14.0 µmoles/dose (e.g. 11.81 µmoles/dose), potassium dihydrogen phosphate in an amount of 0.001 to 1.0 µmoles/dose, preferably 0.01 to 0.1 µmoles/dose, more preferably 0.05 to 0.09 µmoles/dose (e.g. 0.07 µmoles/dose), disodium hydrogen phosphate dihydrate in an amount of 0.01 to 1.0 µmoles/dose, preferably 0.1 to 0.6 µmoles/dose, more preferably 0.3 to 0.5 µmoles/dose (e.g. 0.38 µmoles/dose), potassium chloride in an amount of 0.001 to 1.0 µmoles/dose, preferably 0.01 to 0.5 µmoles/dose, more preferably 0.05 to 0.2 µmoles/dose (e.g. 0.10 µmoles/dose) and Sodium chloride in an amount of 15.0 to 40.0 µmoles/dose, preferably 15.0 to 35.0 µmoles/dose, more preferably 25.0 to 30.0 µmoles/dose (e.g. 26.66 µmoles/dose).

In one embodiment, the dengue vaccine formulation as described herein is present in a container, such as a vial. In certain such embodiments, the arginine hydrochloride is contained in the container at an amount of about 1.5 to 7.5 mg, the urea at an amount of about 2.0 to 8.0 mg, the human serum albumin at an amount of about 0.1 to 2.5 mg, the trehalose at an amount of about 100 to 170 mg, the poloxamer at an amount of about 2.0 to 8.0 mg, and the tromethamine at an amount of about 0.5 to 4.0 mg. In a preferred embodiment, the arginine hydrochloride is contained in the container at an amount of about 2.0 to 6.0 mg, the urea is contained at an amount of about 3.0 to 7.0 mg, the human serum albumin is contained at an amount of about 0.5 to 2.0 mg, the trehalose is contained at an amount of about 120 to 170 mg, the poloxamer is contained at an amount of about 4.0 to 6.0 mg, and the tromethamine is contained at an amount of about 1.0 to 3.0 mg. In a more preferred embodiment, the arginine hydrochloride is contained in the container at an amount of about 3.0 to 5.0 mg, the urea at an amount of about 4.0 to 6.0 mg, the human serum albumin at an amount of about 1.0 to 2.0 mg, the trehalose at an amount of about 140 to 160 mg, the poloxamer at an amount of about 4.0 to 6.0 mg, and the tromethamine at an amount of about 1.0 to 3.0 mg. In a particular preferred embodiment, the arginine hydrochloride is contained in the container at an amount of about 3.5 to 4.5 mg, such as about 4.1 mg, the urea at an amount of about 4.5 to 5.5 mg, such as about 4.95 mg, the human serum albumin at an amount of about 1.5 to 1.8 mg, such as about 1.65 mg, the trehalose at an amount of about 145 to 155 mg, such as about 149 mg, the poloxamer at an amount of about 4.5 to 5.5 mg, such as about 4.95 mg, and the tromethamine at an amount of about 1.5 to 2.5 mg, such as about 2.0 mg.

In a preferred embodiment, the amounts of excipients as described in the preceding paragraph are contained in a container, such as a vial, comprising five unit doses of the dengue vaccine formulation for providing five unit doses of a dengue vaccine formulation in reconstituted form.

### Tetravalent dengue virus composition

### Dengue virus strains

The dengue virus is a single stranded, positive sense RNA virus of the family flaviviridae. The taxonomy is outlined in Table 1. The family flaviviridae includes three genera, flavivirus, hepacivirus and pestivirus. The genus flavivirus contains highly pathogenic and potentially hemorrhagic fever viruses, such as yellow fever virus and dengue virus, encephalitic viruses, such as Japanese encephalitis virus, Murray Valley encephalitis virus and West Nile virus, and a number of less pathogenic viruses.

**Table 1. Dengue Virus Taxonomy of the TDV Parental Strain**

| | |
|---|---|
| **Family** | Flaviviridae |
| **Genus** | Flavivirus |
| **Species** | Dengue virus |
| **Strains** | Dengue Serotype 2 (Strain 16681), Strain DEN-2 PDK-53 |
| **TDV parent** | TDV-2 |

The flavivirus genome comprises in 5' to 3' direction (see Fig. 3):
- a 5'-noncoding region (5'-NCR),
- a capsid protein (C) encoding region,
- a pre-membrane protein (prM) encoding region,
- an envelope protein (E) encoding region,
- a region encoding nonstructural proteins (NS1, NS2A, NS2B, NS3, NS4A, NS4B, NS5) and
- a 3' noncoding region (3'-NCR).

The viral structural proteins are C, prM and E, and the nonstructural proteins are NS1 to NS5. The structural and nonstructural proteins are translated as a single polyprotein and processed by cellular and viral proteases.

The dengue vaccine formulation of the invention as described herein comprises a tetravalent dengue virus composition that comprises four live attenuated dengue virus strains representing dengue serotype 1, dengue serotype 2, dengue serotype 3 and dengue serotype 4. Preferably the composition comprises chimeric dengue viruses and optionally at least one non-chimeric dengue virus. For example, the tetravalent dengue virus composition can comprise four live attenuated dengue serotypes DENV-1, DENV-2, DENV-3 and DENV-4, of which at least one is a dengue-dengue chimera and the other serotypes are each either a dengue-dengue chimera or a non-chimeric dengue serotype. In particular the dengue virus composition comprises a molecularly characterized and cloned dengue serotype 2 strain derived from the live attenuated DEN-2 PDK-53 virus strain (TDV-2), and three chimeric dengue strains derived from the TDV-2 strain by replacing the structural proteins prM and E from TDV-2 with the corresponding structural proteins from the other dengue serotypes, resulting in the following chimeric dengue strains:
- a DENV-2/1 chimera (TDV-1),
- a DENV-2/3 chimera (TDV-3) and
- a DENV-2/4 chimera (TDV-4);
a non-limiting example of four such suitable dengue strains are the four Drug Substances of TAK-003, which are commercially available in the product "Qdenga".

The genetically modified tetravalent dengue vaccine TDV is based on a molecularly characterized and cloned dengue-2 virus strain (TDV-2). This attenuated TDV-2 strain was generated by cDNA cloning of the attenuated laboratory-derived DEN-2 PDK-53 virus strain that was originally isolated at Mahidol University, Bangkok, Thailand (Kinney et al. (1997) Virology 230(2): 300-308). DEN-2 PDK-53 was generated by 53 serial passages in primary dog kidney (PDK) cells at 32 °C (Bhamarapravati et al. (1987) Bull. World Health Organ. 65(2): 189-195).

The attenuated DEN-2 PDK-53 strain (the precursor of TDV-2) was derived from the wild type virus strain DEN-2 16681 and differs in nine nucleotides from the wild type, characterized as follows (Kinney et al. (1997) Virology 230(2): 300-308):
(i) 5'-noncoding region (NCR)-57 (nt-57 C-to-T): major attenuation locus
(ii) prM-29 Asp-to-Val (nt-524 A-to-T)
(iii) nt-2055 C-to-T (E gene) silent mutation
(iv) NS1-53 Gly-to-Asp (nt-2579 G-to-A): major attenuation locus
(v) NS2A-181 Leu-to-Phe (nt-4018 C-to-T)
(vi) NS3-250 Glu-to-Val (nt-5270 A-to-T): major attenuation locus
(vii) nt-5547 (NS3 gene) T-to-C silent mutation
(viii) NS4A-75 Gly-to-Ala (nt-6599 G-to-C)

* nt-8571 C-to-T (NS5 gene) silent mutation

The three nucleotide changes located in the 5' noncoding region (NCR) (nucleotide 57) (mutation (i)), the NS-1 (amino acid 828 of SEQ ID NO. 4) (mutation (iv)) and NS-3 genes (amino acid 1725 of SEQ ID NO. 4) (mutation (vi)) form the basis for the attenuation phenotype of the DEN-2 PDK-53 strain (Butrapet et al. (2000) J. Virol. 74(7): 3111-3119) (Table 2). These three mutations are referred to herein as the "attenuating mutations" and are comprised in TDV-1, TDV-2, TDV-3 and TDV-4.

**Table 2. Attenuating mutations in the common genetic backbone of all TDV strains**

| **Location of Mutation** | **Nucleotide Change in TDV-2** | **Amino Acid Change in TDV-2** |
|---|---|---|
| 5' Noncoding Region (5'NCR) | 57 C to T | Not applicable (silent) |
| Nonstructural Protein 1 (NS1) | 2579 G to A | 828 Gly to Asp |
| Nonstructural Protein 3 (NS3) | 5270 A to T | 1725 Glu to Val |

In one embodiment, TDV-2 comprises in addition to the three attenuating mutations one or more mutations selected from:
a) a mutation in the prM gene at nucleotide 524 from adenine to thymine resulting in an amino acid change at position 143 from aspartic acid to valine, and/or
b) a silent mutation in the E gene at nucleotide 2055 from cytosine to thymine, and/or
c) a mutation in the NS2A gene at nucleotide 4018 from cytosine to thymine resulting in an amino acid change at position 1308 from leucine to phenylalanine, and/or
d) a silent mutation in the NS3 gene at nucleotide 5547 from thymine to cytosine, and/or
e) a mutation in the NS4A gene at nucleotide 6599 from guanine to cytosine resulting in an amino acid change at position 2168 from glycine to alanine, and/or
f) a silent mutation in the prM gene at nucleotide 900 from thymine to cytosine.

The silent mutation in the NS5 gene at nucleotide 8571 from cytosine to thymine of DEN-2 PDK-53 is not present in the TDV-2 strain.

In another embodiment, TDV-2 comprises in addition to the three attenuating mutations one or more mutations selected from:
g) a mutation in the prM gene at nucleotide 592 from adenine to guanine resulting in an amino acid change at position 166 from lysine to glutamic acid, and/or
h) a mutation in the NS5 gene at nucleotide 8803 from adenine to guanine resulting in an amino acid change at position 2903 from isoleucine to valine.

In another embodiment, TDV-2 comprises in addition to the three attenuating mutations the mutations a) and g), preferably the mutations a), g), c), e) and h), more preferably the mutations a), g), c), e), h) and b), even more preferably the mutations a), g), c), e), h), b) and d), and most preferably the mutations a) to h). The nucleotide positions and amino acids positions of TDV-2 refer to the nucleotide sequence as shown in SEQ ID NO. 3 and amino acid sequence as shown in SEQ ID NO. 4.

The dengue virus structural envelope (E) protein and pre-membrane (prM) protein have been identified as the primary antigens that elicit a neutralizing protective antibody response (Plotkin 2001). For creation of the tetravalent dengue vaccine (TDV), TDV-2 was modified by replacing the nucleic acid sequence encoding the DENV-2 prM and E glycoproteins with the nucleic acid sequence encoding the corresponding wild type prM and E glycoproteins from the DENV-1, DENV-3, and DENV-4 wild type strains DENV-1 16007, DENV-3 16562 or DENV-4 1036 virus, respectively, (see Table 3) using standard molecular genetic engineering methods (Huang et al. (2003) J. Virol. 77(21): 11436-11447).

**Table 3. Viral origin of prM/E gene regions of the TDV virus strains**

| **Virus** | **Strain** | **Origin** | **Source** | **Reference** | **Nucleotide sequence** | **Amino acid sequence** |
|---|---|---|---|---|---|---|
| DENV-1 | 16007 | Thailand, 1964 | DHF/DSS patient | Halstead and Simasthien, 1970 | SEQ ID NO. 9 | SEQ ID NO. 10 |
| DENV-2 | 16681 | Thailand, 1964 | DHF/DSS patient | Halstead and Simasthien, 1970 | SEQ ID NO. 11 | SEQ ID NO. 12 |
| DENV-3 | 16562 | Philippines, 1964 | DHF patient | Halstead and Simasthien, 1970 | SEQ ID NO. 13 | SEQ ID NO. 14 |
| DENV-4 | 1036 | Indonesia, 1976 | DF patient | Gubler et al., 1979 | SEQ ID NO. 15 | SEQ ID NO. 16 |

A diagram of the four TDV strains comprised in the dengue vaccine formulation is shown in **Fig. 3****.**

The chimeric dengue strains TDV-1, TDV-3 and TDV-4 express the surface antigens prM and E of the DENV-1, DENV-3 or DENV-4 viruses, as depicted in Table 3 respectively, and retain the genetic alterations responsible for the attenuation of TDV-2. Thus, each of the TDV-1, TDV-3 and TDV-4 strains comprises the attenuating mutations described in Table 2.

In one embodiment, TDV-1 comprises in addition to the three attenuating mutations one or more mutations selected from:
c) a mutation in the NS2A gene at nucleotide 4018 from cytosine to thymine resulting in an amino acid change at position 1308 from leucine to phenylalanine, and/or
d) a silent mutation in the NS3 gene at nucleotide 5547 from thymine to cytosine, and/or
e) a mutation in the NS4A gene at nucleotide 6599 from guanine to cytosine resulting in an amino acid change at position 2168 from glycine to alanine, and/or
i) a silent mutation in the E gene at nucleotide 1575 from thymine to cytosine, and/or
j) a silent mutation in the junction site between the prM-E gene and the DEN-2 PDK-53 backbone at nucleotide 453 from adenine to guanine, and/or
k) a mutation in the junction site between the prM-E gene and the DEN-2 PDK-53 backbone at nucleotides 2381/2382 from thymine-guanine to cytosine-cytosine resulting in an amino acid change at position 762 from valine to alanine.

In another embodiment, TDV-1 comprises in addition to the three attenuating mutations one or more mutations selected from:
l) a mutation in the NS2A gene at nucleotide 3823 from adenine to cytosine resulting in an amino acid change at position 1243 from isoleucine to leucine, and/or
m) a mutation in the NS2B gene at nucleotide 4407 from adenine to thymine resulting in an amino acid change at position 1437 from glutamic acid to aspartic acid, and/or
n) a silent mutation in the NS4B gene at nucleotide 7311 from adenine to guanine.

In another embodiment, the TDV-1 strain comprises in addition to the three attenuating mutations the mutations l) and m), preferably the mutations I), m), c) and e), even more preferably the mutations I), m), c), e), d) and n), and most preferably the mutations I), m), c), e), d), n), i), j) and k). The nucleotide positions and amino acids positions of TDV-1 refer to the nucleotide sequence as shown in SEQ ID NO. 1 and amino acid sequence as shown in SEQ ID NO. 2.

In one embodiment, TDV-3 comprises in addition to the three attenuating mutations one or more mutations selected from:
c) a mutation in the NS2A gene at nucleotide 4012 from cytosine to thymine resulting in an amino acid change at position 1306 from leucine to phenylalanine, and/or
d) a silent mutation in the NS3 gene at nucleotide 5541 from thymine to cytosine, and/or
e) a mutation in the NS4A gene at nucleotide 6593 from guanine to cytosine resulting in an amino acid change at position 2166 from glycine to alanine, and/or
j) a silent mutation in the junction site between the prM-E gene and the DEN-2 PDK-53 backbone at nucleotide 453 from adenine to guanine, and/or
k) a mutation in the junction site between the prM-E gene and the DEN-2 PDK-53 backbone at nucleotides 2375/2376 from thymine-guanine to cytosine-cytosine resulting in an amino acid change at position 760 from valine to alanine, and/or
o) a silent mutation in the prM gene at nucleotide 552 from cytosine to thymine, and/or
p) a mutation in the E gene at nucleotide 1970 from adenine to thymine resulting in an amino acid change at position 625 from histidine to leucine.

In another embodiment, TDV-3 comprises in addition to the three attenuating mutations one or more mutations selected from:
q) a mutation in the E gene at nucleotide 1603 from adenine to thymine resulting in an amino acid change at position 503 from threonine to serine, and/or
r) a silent mutation in the NS5 gene at nucleotide 7620 from adenine to guanine.

In another embodiments, TDV-3 comprises in addition to the three attenuating mutations the mutations p) and q), preferably the mutations p), q), c) and e), even more preferably the mutations p), q), c), e), d) and r), and most preferably the mutations p), q), c), e), d), r), j), k) and o). The nucleotide positions and amino acids positions of TDV-3 refer to the nucleotide sequence as shown in SEQ ID NO. 5 and amino acid sequence as shown in SEQ ID NO. 6.

In one embodiment, TDV-4 comprises in addition to the three attenuating mutations one or more mutations selected from:
c) a mutation in the NS2A gene at nucleotide 4018 from cytosine to thymine resulting in an amino acid change at position 1308 from leucine to phenylalanine, and/or
d) a silent mutation in the NS3 gene at nucleotide 5547 from thymine to cytosine, and/or
e) a mutation in the NS4A gene at nucleotide 6599 from guanine to cytosine resulting in an amino acid change at position 2168 from glycine to alanine, and/or
j) a silent mutation in the junction site between the prM-E gene and the DEN-2 PDK-53 backbone at nucleotide 453 from adenine to guanine, and/or
k) a mutation in the junction site between the prM-E gene and the DEN-2 PDK-53 backbone at nucleotides 2381/2382 from thymine-guanine to cytosine-cytosine resulting in an amino acid change at position 762 from valine to alanine, and/or
s) a mutation in the C gene at nucleotide 396 from adenine to cytosine resulting in an amino acid change at position 100 from arginine to serine, and/or
t) a silent mutation in the E gene at nucleotide 1401 from adenine to guanine, and/or
u) a mutation in the E gene at nucleotide 2027 from cytosine to thymine resulting in an amino acid change at position 644 from alanine to valine, and/or
v) a mutation in the E gene at nucleotide 2275 from adenine to cytosine resulting in an amino acid change at position 727 from methionine to leucine.

In another embodiment, TDV-4 comprises in addition to the three attenuating mutations one or more mutations selected from:
w) a silent mutation in the C gene at nucleotide 225 from adenine to thymine, and/or
x) a mutation in the NS2A gene at nucleotide 3674 from adenine to guanine resulting in an amino acid change at position 1193 from aspartic acid to glycine, and/or
y) a mutation in the NS2A gene at nucleotide 3773 from adenine to an adenine/guanine mix resulting in an amino acid change at position 1226 from lysine to a lysine/arginine mix, and/or
z) a silent mutation in the NS3 gene at nucleotide 5391 from cytosine to thymine, and/or
aa) a mutation in the NS4A gene at nucleotide 6437 from cytosine to thymine resulting in an amino acid change at position 2114 from alanine to valine, and/or
bb) a silent mutation in the NS4B gene at nucleotide 7026 from thymine to a thymine/cytosine mix or to cytosine, and/or
cc) a silent mutation in the NS5 gene at nucleotide 9750 from adenine to cytosine.

In another embodiment, TDV-4 comprises in addition to the three attenuating mutations the mutation s), u) and v), preferably the mutations s), u), v), c), e), x), y) and aa), even more preferably the mutations s), u), v), c), e), x), y), aa) and w), even more preferably the mutations s), u), v), c), e), x), y), aa), w), d), z), bb) and cc), and most preferably the mutations s), u), v), c), e), x), y), aa), w), d), z), bb), cc), j), k) and t). The nucleotide positions and amino acids positions of TDV-4 refer to the nucleotide sequence as shown in SEQ ID NO. 7 and amino acid sequence as shown in SEQ ID NO. 8.

In a preferred embodiment, TDV-1 is characterized by the nucleotide sequence of SEQ ID NO. 1, TDV-2 is characterized by the nucleotide sequence of SEQ ID NO. 3, TDV-3 is characterized by the nucleotide sequence of SEQ ID NO. 5, and/or TDV-4 is characterized by the nucleotide sequence of SEQ ID NO. 7. In a further preferred embodiment, TDV-1 is characterized by the amino acid sequence of SEQ ID NO. 2, TDV-2 is characterized by the amino acid sequence of SEQ ID NO. 4, TDV-3 is characterized by the amino acid sequence of SEQ ID NO. 6, and TDV-4 is characterized by the amino acid sequence of SEQ ID NO. 8. In a further preferred embodiment, TDV-1 is characterized by a nucleotide sequence encoding the amino acid sequence of SEQ ID NO. 2, TDV-2 is characterized by a nucleotide sequence encoding the amino acid sequence of SEQ ID NO. 4, TDV-3 is characterized by a nucleotide sequence encoding the amino acid sequence of SEQ ID NO. 6, and TDV-4 is characterized by a nucleotide sequence encoding the amino acid sequence of SEQ ID NO. 8.

**Table 4. Sequences of the TDV virus strains**

| **SEQ ID NO.** | **dengue virus strain** | **sequence type** |
|---|---|---|
| **SEQ ID NO. 1** | TDV-1 | nucleotide sequence |
| **SEQ ID NO. 2** | TDV-1 | amino acid sequence |
| **SEQ ID NO. 3** | TDV-2 | nucleotide sequence |
| **SEQ ID NO. 4** | TDV-2 | amino acid sequence |
| **SEQ ID NO. 5** | TDV-3 | nucleotide sequence |
| **SEQ ID NO. 6** | TDV-3 | amino acid sequence |
| **SEQ ID NO. 7** | TDV-4 | nucleotide sequence |
| **SEQ ID NO. 8** | TDV-4 | amino acid sequence |

Thus, in a particularly preferred embodiment, the dengue vaccine formulation of the invention as described herein comprises the live attenuated dengue virus strains TDV-1, TDV-2, TDV-3 and TDV-4, wherein TDV-1, TDV-3 and TDV-4 are based on TDV-2 and comprise the prM and E regions of DENV-1, -3 and -4, respectively. In another particularly preferred embodiment, TDV-1 is characterized by the nucleotide sequence according to SEQ ID No. 1 and the amino acid sequence according to SEQ ID No. 2, TDV-2 is characterized by the nucleotide sequence according to SEQ ID No. 3 and the amino acid sequence according to SEQ ID No. 4, TDV-3 is characterized by the nucleotide sequence according to SEQ ID No. 5 and the amino acid sequence according to SEQ ID No. 6 and TDV-4 is characterized by the nucleotide sequence according to SEQ ID No. 7 and the amino acid sequence according to SEQ ID No. 8.

The E protein of DENV-3 has two fewer amino acids than the E protein of DENV-2. Therefore, the nucleotides and encoded amino acid backbone of TDV-2 starting after the E region of DENV-3 at nucleotide 2374 of SEQ ID NO. 5 and amino acid 760 of SEQ ID NO. 6 are 6 nucleotides less and 2 amino acids less than the original TDV-2 nucleotide and amino acid positions, respectively.

In a particularly preferred embodiment, the dengue vaccine formulation of the invention as described herein comprises the tetravalent dengue virus composition "TAK-003".

### Virus concentrations of the dengue vaccine formulation of the invention

The present invention is in part directed to a dengue vaccine composition comprising a tetravalent dengue virus composition including four live attenuated dengue virus strains:
(i) a dengue serotype 1 (e.g. chimeric dengue serotype 2/1 strain) in a concentration of at least 3.3 log10 pfu/0.5 mL,
(ii) a dengue serotype 2 (e.g dengue serotype 2 strain) in a concentration of at least 2.7 log10 pfu/0.5 mL, or at least 3.1 log10 pfu/0.5 mL,
(iii) a dengue serotype 3 (e.g. chimeric dengue serotype 2/3 strain) in a concentration of at least 4.0 log10 pfu/0.5 mL, and
(iv) a dengue serotype 4 (e.g. chimeric dengue serotype 2/4 strain) in a concentration of at least 4.5 log10 pfu/0.5 mL.

In one embodiment, the dengue vaccine composition comprises a tetravalent dengue virus composition including four live attenuated dengue virus strains:
(i) a dengue serotype 1 (e.g. chimeric dengue serotype 2/1 strain such as TDV-1) preferably in a concentration of at least 3.3 log10 pfu/0.5 mL to 3.8 log10 pfu/0.5 mL,
(ii) a dengue serotype 2 (e.g dengue serotype 2 strain such a TDV-2) preferably in a concentration of at least 2.7 log10 pfu/0.5 mL, or at least 3.1 log10 pfu/0.5 mL,
(iii) a dengue serotype 3 (e.g. chimeric dengue serotype 2/3 strain such as TDV-3) preferably in a concentration of at least 4.0 log10 pfu/0.5 mL, and
(iv) a dengue serotype 4 (e.g. chimeric dengue serotype 2/4 strain such as TDV-4) preferably in a concentration of at least 4.5 log10 pfu/0.5 ml or 4.6 log10 pfu/0.5 mL, optionally to 6.2 log10 pfu/0.5 ml.

The present invention is further in part directed to a unit dose of a dengue vaccine composition, wherein the dengue vaccine composition comprises a tetravalent dengue virus composition including four live attenuated dengue virus strains:
(i) a chimeric dengue serotype 2/1 strain in a concentration of at least 3.3 log10 pfu/0.5 mL,
(ii) a dengue serotype 2 strain in a concentration of at least 2.7 log10 pfu/0.5 mL, or at least 3.1 log10 pfu/0.5 mL,
(iii) a chimeric dengue serotype 2/3 strain in a concentration of at least 4.0 log10 pfu/0.5 mL, and
(iv) a chimeric dengue serotype 2/4 strain in a concentration of at least 4.5 log10 pfu/0.5 mL.

In one embodiment, the dengue vaccine composition comprises a tetravalent dengue virus composition including four live attenuated dengue virus strains:
(i) a chimeric dengue serotype 2/1 strain in a concentration of at least 3.3 log10 pfu/0.5 mL to 3.8 log10 pfu/0.5 ml,
(ii) a dengue serotype 2 strain in a concentration of at least 2.7 log10 pfu/0.5 mL, or at least 3.1 log10 pfu/0.5 mL,
(iii) a chimeric dengue serotype 2/3 strain in a concentration of at least 4.0 log10 pfu/0.5 mL, and
(iv) a chimeric dengue serotype 2/4 strain in a concentration of at least 4.5 log10 pfu/0.5 mL or at least 4.6 log10 pfu/0.5 mL to optionally 6.2 log10 pfu/0.5 ml.

Preferably, the chimeric dengue serotype 2/1 strain is TDV-1, the dengue serotype 2 strain is TDV-2, the chimeric dengue serotype 2/3 strain is TDV-3 and the chimeric dengue serotype 2/4 strain is TDV-4.

In one embodiment, the dengue vaccine composition comprises a tetravalent dengue virus composition including four live attenuated dengue virus strains wherein:
(i) the dengue serotype 1 (e.g. chimeric dengue serotype 2/1 strain) has a concentration of 3.3 log10 pfu/0.5 mL to 5.3 log10 pfu/0.5 mL,
(ii) the dengue serotype 2 (e.g. dengue serotype 2 strain) has a concentration of 2.7 log10 pfu/0.5 mL to 5.0 log10 pfu/0.5 mL, or of 3.1 log10 pfu/0.5 mL to 5.0 log10 pfu/0.5 mL,
(iii) the dengue serotype 3 (e.g. chimeric dengue serotype 2/3 strain) has a concentration of 4.0 log10 pfu/0.5 mL to 6.0 log10 pfu/0.5 mL, and
(iv) the dengue serotype 4 (e.g. chimeric dengue serotype 2/4 strain) has a concentration of 4.5 log10 pfu/0.5 mL to 6.5 log10 pfu/0.5 mL.

In one such embodiment, the dengue vaccine composition comprises a tetravalent dengue virus composition including four live attenuated dengue virus strains wherein:
(i) the dengue serotype 1 (e.g. chimeric dengue serotype 2/1 strain) has a concentration of 3.3 log10 pfu/0.5 mL to 5.0 log10 pfu/0.5 mL,
(ii) the dengue serotype 2 (e.g. dengue serotype 2 strain) has a concentration of 2.7 log10 pfu/0.5 mL to 4.9 log10 pfu/0.5 mL, or of 3.1 log10 pfu/0.5 mL to 4.9 log10 pfu/0.5 mL,
(iii) the dengue serotype 3 (e.g. chimeric dengue serotype 2/3 strain) has a concentration of 4.0 log10 pfu/0.5 mL to 5.7 log10 pfu/0.5 mL, and
(iv) the dengue serotype 4 (e.g. chimeric dengue serotype 2/4 strain) has a concentration of 4.5 log10 pfu/0.5 mL to 6.2 log10 pfu/0.5 mL.

In a further such embodiment, the dengue vaccine composition comprises a tetravalent dengue virus composition including four live attenuated dengue virus strains wherein:
(i) a dengue serotype 1 (e.g. chimeric dengue serotype 2/1 strain) has a concentration of 3.3 log10 pfu/dose to 5.0 log10 pfu/dose,
(ii) a dengue serotype 2 (e.g. dengue serotype 2 strain) has a concentration of 2.7 log10 pfu/dose to 4.9 log10 pfu/ dose, or of 3.1 log10 pfu/ dose to 4.9 log10 pfu/ dose,
(iii) a dengue serotype 3 (e.g. chimeric dengue serotype 2/3 strain) has a concentration of 4.0 log10 pfu/dose to 5.7 log10 pfu/ dose, and
(iv) a dengue serotype 4 (e.g. chimeric dengue serotype 2/4 strain) has a concentration of 4.5 log10 pfu/dose to 5.5 log10 pfu/dose.

In a further such embodiment, the dengue vaccine composition comprises a tetravalent dengue virus composition including four live attenuated dengue virus strains wherein:
(i) a dengue serotype 1 (e.g. chimeric dengue serotype 2/1 strain) has a concentration of 3.3 log10 pfu/dose to 4.1 log10 pfu/dose,
(ii) a dengue serotype 2 (e.g. dengue serotype 2 strain) has a concentration of 2.7 log10 pfu/dose to 3.6 log10 pfu/dose, or of 3.1 log10 pfu/ dose to 3.6 log10 pfu/ dose,
(iii) a dengue serotype 3 (e.g. chimeric dengue serotype 2/3 strain) has a concentration of 4.0 log10 pfu/dose to 4.7 log10 pfu/dose, and
(iv) a dengue serotype 4 (e.g. chimeric dengue serotype 2/4 strain) has a concentration of 4.5 log10 pfu/dose to 5.3 log10 pfu/dose.

In a further such embodiment, the dengue vaccine composition comprises a tetravalent dengue virus composition including four live attenuated dengue virus strains wherein:
(i) a dengue serotype 1 (e.g. chimeric dengue serotype 2/1 strain) has a concentration of 3.3 log10 pfu/0.5 mL to 3.6 log10 pfu/0.5 mL,
(ii) a dengue serotype 2 (e.g. dengue serotype 2 strain) has a concentration of 2.7 log10 pfu/0.5 mL to 4.0 log10 pfu/0.5 mL, or of 3.1 log10 pfu/0.5 mL to 4.0 log10 pfu/0.5 mL,
(iii) a dengue serotype 3 (e.g. chimeric dengue serotype 2/3 strain) has a concentration of 4.0 log10 pfu/0.5 mL to 4.6 log10 pfu/0.5 mL, and
(iv) a dengue serotype 4 (e.g. chimeric dengue serotype 2/4 strain) has a concentration of 4.5 log10 pfu/0.5 ml or 4.6 log10 pfu/0.5 mL to 5.1 log10 pfu/0.5 mL.

In another embodiment, the dengue vaccine composition comprises a tetravalent dengue virus composition including four live attenuated dengue virus strains wherein:
(i) the dengue serotype 1 (e.g. chimeric dengue serotype 2/1 strain) has a concentration of 4.3 log10 pfu/0.5 mL to 4.4 log10 pfu/0.5 mL,
(ii) the dengue serotype 2 (e.g. dengue serotype 2 strain) has a concentration of 3.7 log10 pfu/0.5 mL to 3.8 log10 pfu/0.5 mL,
(iii) the dengue serotype 3 (e.g. chimeric dengue serotype 2/3 strain) has a concentration of 4.5 log10 pfu/0.5 mL to 5.0 log10 pfu/0.5 mL, and
(iv) the dengue serotype 4 (e.g. chimeric dengue serotype 2/4 strain) has a concentration of 5.5 log10 pfu/0.5 mL to 5.6 log10 pfu/0.5 mL.

In another particularly preferred embodiment, the dengue vaccine composition comprises a tetravalent dengue virus composition including four live attenuated dengue virus strains wherein:
(i) the dengue serotype 1 (e.g. chimeric dengue serotype 2/1 strain) has a concentration of 3.6 log10 pfu/0.5 mL,
(ii) the dengue serotype 2 (e.g. dengue serotype 2 strain) has a concentration of 4.0 log10 pfu/0.5 mL,
(iii) the dengue serotype 3 (e.g. chimeric dengue serotype 2/3 strain) has a concentration of 4.6 log10 pfu/0.5 mL, and
(iv) the dengue serotype 4 (e.g. chimeric dengue serotype 2/4 strain) has a concentration of 5.1 log10 pfu/0.5 mL.

In another preferred embodiment, the dengue vaccine composition comprises a tetravalent dengue virus composition including four live attenuated dengue virus strains wherein the arithmetic sum of all four serotypes is less than 6.7 log10 pfu/0.5 mL, preferably less than 5.5 log10 pfu/0.5 mL. In certain such embodiments, the arithmetic sum of all four serotypes is at least 4.6 log10 pfu/0.5 mL. In a preferred embodiment, the dengue vaccine composition comprises a tetravalent dengue virus composition including four live attenuated dengue virus strains wherein the arithmetic sum of all four serotypes is in the range of 4.6 log10 pfu/0.5 mL to 6.7 log10 pfu/0.5 mL, preferably in the range of 4.6 log10 pfu/0.5 mL to 5.5 log10 pfu/0.5 mL.

Preferably, in said embodiments the chimeric dengue serotype 2/1 strain is TDV-1, the dengue serotype 2 strain is TDV-2, the chimeric dengue serotype 2/3 strain is TDV-3 and the chimeric dengue serotype 2/4 strain is TDV-4. More preferably, TDV-1 is characterized by the nucleotide sequence according to SEQ ID No. 1 and the amino acid sequence according to SEQ ID No. 2, TDV-2 is characterized by the nucleotide sequence according to SEQ ID No. 3 and the amino acid sequence according to SEQ ID No. 4, TDV-3 is characterized by the nucleotide sequence according to SEQ ID No. 5 and the amino acid sequence according to SEQ ID No. 6 and TDV-4 is characterized by the nucleotide sequence according to SEQ ID No. 7 and the amino acid sequence according to SEQ ID No. 8.

The present invention is in part directed to a unit dose of a dengue vaccine composition, wherein the dengue vaccine composition comprises a tetravalent dengue virus composition including four live attenuated dengue virus strains:
(i) a dengue serotype 1 (e.g. chimeric dengue serotype 2/1 strain) in a concentration of at least 3.3 log10 pfu/dose,
(ii) a dengue serotype 2 (e.g. dengue serotype 2 strain) in a concentration of at least 2.7 log10 pfu/dose, or at least 3.1 log10 pfu/dose,
(iii) a dengue serotype 3 (e.g. chimeric dengue serotype 2/3 strain) in a concentration of at least 4.0 log10 pfu/dose, and
(iv) a dengue serotype 4 (e.g. chimeric dengue serotype 2/4 strain) in a concentration of at least 4.5 log10 pfu/dose.

In one embodiment, the dengue vaccine composition comprises a tetravalent dengue virus composition including four live attenuated dengue virus strains wherein:
(i) the dengue serotype 1 (e.g. chimeric dengue serotype 2/1 strain) has a concentration of 3.3 log10 pfu/dose to 5.3 log10 pfu/dose,
(ii) the dengue serotype 2 (e.g. dengue serotype 2 strain) has a concentration of 2.7 log10 pfu/dose to 5.0 log10 pfu/dose, or of 3.1 log10 pfu/dose to 5.0 log10 pfu/dose,
(iii) the dengue serotype 3 (e.g. chimeric dengue serotype 2/3 strain) has a concentration of 4.0 log10 pfu/dose to 6.0 log10 pfu/dose, and
(iv) the dengue serotype 4 (e.g. chimeric dengue serotype 2/4 strain) has a concentration of 4.5 log10 pfu/dose to 6.5 log10 pfu/dose.

In one such embodiment, the dengue vaccine composition comprises a tetravalent dengue virus composition including four live attenuated dengue virus strains wherein:
(i) the dengue serotype 1 (e.g. chimeric dengue serotype 2/1 strain) has a concentration of 3.3 log10 pfu/dose to 5.0 log10 pfu/dose,
(ii) the dengue serotype 2 (e.g. dengue serotype 2 strain) has a concentration of 2.7 log10 pfu/dose to 4.9 log10 pfu/dose, or of 3.1 log10 pfu/dose to 4.9 log10 pfu/dose,
(iii) the dengue serotype 3 (e.g. chimeric dengue serotype 2/3 strain) has a concentration of 4.0 log10 pfu/dose to 5.7 log10 pfu/dose, and
(iv) the dengue serotype 4 (e.g. chimeric dengue serotype 2/4 strain) has a concentration of 4.5 log10 pfu/dose to 6.2 log10 pfu/dose.

In a further such embodiment, the dengue vaccine composition comprises a tetravalent dengue virus composition including four live attenuated dengue virus strains wherein:
(i) a dengue serotype 1 (e.g. chimeric dengue serotype 2/1 strain) has a concentration of 3.3 log10 pfu/dose to 5.0 log10 pfu/dose,
(ii) a dengue serotype 2 (e.g. dengue serotype 2 strain) has a concentration of 2.7 log10 pfu/dose to 4.9 log10 pfu/dose, or of 3.1 log10 pfu/dose to 4.9 log10 pfu/dose,
(iii) a dengue serotype 3 (e.g. chimeric dengue serotype 2/3 strain) has a concentration of 4.0 log10 pfu/dose to 5.7 log10 pfu/dose, and
(iv) a dengue serotype 4 (e.g. chimeric dengue serotype 2/4 strain) has a concentration of 4.5 log10 pfu/dose to 5.5 log10 pfu/dose.

In a further such embodiment, the dengue vaccine composition comprises a tetravalent dengue virus composition including four live attenuated dengue virus strains wherein:
(i) a dengue serotype 1 (e.g. chimeric dengue serotype 2/1 strain) has a concentration of 3.3 log10 pfu/dose to 4.1 log10 pfu/dose,
(ii) a dengue serotype 2 (e.g. dengue serotype 2 strain) has a concentration of 2.7 log10 pfu/dose to 3.6 log10 pfu/ dose, or of 3.1 log10 pfu/dose to 3.6 log10 pfu/dose,
(iii) a dengue serotype 3 (e.g. chimeric dengue serotype 2/3 strain) has a concentration of 4.0 log10 pfu/dose to 4.7 log10 pfu/dose, and
(iv) a dengue serotype 4 (e.g. chimeric dengue serotype 2/4 strain) has a concentration of 4.5 log10 pfu/dose to 5.3 log10 pfu/dose.

In a further such embodiment, the dengue vaccine composition comprises a tetravalent dengue virus composition including four live attenuated dengue virus strains wherein:
(i) a dengue serotype 1 (e.g. chimeric dengue serotype 2/1 strain) has a concentration of 3.3 log10 pfu/dose to 3.6 log10 pfu/dose,
(ii) a dengue serotype 2 (e.g. dengue serotype 2 strain) has a concentration of 2.7 log10 pfu/dose to 4.0 log10 pfu/dose, or of 3.1 log10 pfu/dose to 4.0 log10 pfu/dose,
(iii) a dengue serotype 3 (e.g. chimeric dengue serotype 2/3 strain) has a concentration of 4.0 log10 pfu/dose to 4.6 log10 pfu/dose, and
(iv) a dengue serotype 4 (e.g. chimeric dengue serotype 2/4 strain) has a concentration of 4.5 log10 pfu/dose 4.6 log10 pfu/dose to 5.1 log10 pfu/dose.

In another embodiment, the dengue vaccine composition comprises a tetravalent dengue virus composition including four live attenuated dengue virus strains wherein:
(i) the dengue serotype 1 (e.g. chimeric dengue serotype 2/1 strain) has a concentration of 4.3 log10 pfu/dose to 4.4 log10 pfu/dose,
(ii) the dengue serotype 2 (e.g. dengue serotype 2 strain) has a concentration of 3.7 log10 pfu/dose to 3.8 log10 pfu/dose,
(iii) the dengue serotype 3 (e.g. chimeric dengue serotype 2/3 strain) has a concentration of 4.5 log10 pfu/dose to 5.0 log10 pfu/dose, and
(iv) the dengue serotype 4 (e.g. chimeric dengue serotype 2/4 strain) has a concentration of 5.5 log10 pfu/dose to 5.6 log10 pfu/dose.

In a particularly preferred embodiment, the dengue vaccine composition comprises a tetravalent dengue virus composition including four live attenuated dengue virus strains wherein:
(i) the dengue serotype 1 (e.g. chimeric dengue serotype 2/1 strain) has a concentration of 4.4 log10 pfu/dose,
(ii) the dengue serotype 2 (e.g. dengue serotype 2 strain) has a concentration of 3.8 log10 pfu/dose,
(iii) the dengue serotype 3 (e.g. chimeric dengue serotype 2/3 strain) has a concentration of 4.5 log10 pfu/dose, and
(iv) the dengue serotype 4 (e.g. chimeric dengue serotype 2/4 strain) has a concentration of 5.6 log10 pfu/dose.

In another particularly preferred embodiment, the dengue vaccine composition comprises a tetravalent dengue virus composition including four live attenuated dengue virus strains wherein:
(i) the dengue serotype 1 (e.g. chimeric dengue serotype 2/1 strain) has a concentration of 3.6 log10 pfu/dose,
(ii) the dengue serotype 2 (e.g. dengue serotype 2 strain) has a concentration of 4.0 log10 pfu/dose,
(iii) the dengue serotype 3 (e.g. chimeric dengue serotype 2/3 strain) has a concentration of 4.6 log10 pfu/dose, and
(iv) the dengue serotype 4 (e.g. chimeric dengue serotype 2/4 strain) has a concentration of 5.1 log10 pfu/dose.

In another preferred embodiment, the dengue vaccine composition comprises a tetravalent dengue virus composition including four live attenuated dengue virus strains wherein the arithmetic sum of all four serotypes is less than 6.7 log10 pfu/dose, preferably less than 5.5 log10 pfu/dose. In certain such embodiments, the arithmetic sum of all four serotypes is at least 4.6 log10 pfu/dose. in a preferred embodiment, the dengue vaccine composition comprises a tetravalent dengue virus composition including four live attenuated dengue virus strains wherein the arithmetic sum of all four serotypes is in the range of 4.6 log10 pfu/dose to 6.7 log10 pfu/dose, preferably in the range of 4.6 log10 pfu/dose to 5.5 log10 pfu/dose.

In one embodiment in the composition (i), (ii), (iii), and (iv) provide a total concentration of pfu/0.5 mL and based on said concentration, the concentration of (iii) at least 10% of the total concentration in pfu/0.5 mL.

In one embodiment in the composition (i), (ii), (iii), and (iv) provide a total concentration of pfu/0.5 mL and based on said total concentration the concentration of (ii) in pfu/0.5 mL is less than 10%, and the concentration of (iv) in pfu/0.5 mL is at least 50%, and the concentration of (i) in pfu/0.5 mL is at least 1%, and the concentration of (iii) in pfu/0.5 mL is at least 8%, or at least 10%, or at least 12%, or at least 14%, or at least 16%, or at least 18%.

It is preferred that the concentration in the reconstituted unit dose of (iii) in pfu/0.5 mL is at least 10%.

In one embodiment in the composition (i), (ii), (iii), and (iv) provide a total concentration of pfu/0.5 mL and based on said total concentration the concentration of (ii) in pfu/0.5 mL is less than 2%, the concentration of (iv) in pfu/0.5 mL is at least 50%, the concentration of (i) in pfu/0.5 mL is at least 1%, and the concentration of (iii) in pfu/0.5 mL is at least 6%.

Preferably, in said embodiments the chimeric dengue serotype 2/1 strain is TDV-1, the dengue serotype 2 strain is TDV-2, the chimeric dengue serotype 2/3 strain is TDV-3 and the chimeric dengue serotype 2/4 strain is TDV-4. More preferably, TDV-1 is characterized by the nucleotide sequence according to SEQ ID No. 1 and the amino acid sequence according to SEQ ID No. 2, TDV-2 is characterized by the nucleotide sequence according to SEQ ID No. 3 and the amino acid sequence according to SEQ ID No. 4, TDV-3 is characterized by the nucleotide sequence according to SEQ ID No. 5 and the amino acid sequence according to SEQ ID No. 6 and TDV-4 is characterized by the nucleotide sequence according to SEQ ID No. 7 and the amino acid sequence according to SEQ ID No. 8.

The concentration of the different dengue viruses is preferably determined by an immuno-focus assay known in the art. For example, the concentration may be determined by an immuno-focus assay wherein serial dilutions of dengue virus are applied to monolayers of adherent cells, such as Vero cells. After a period of time which allows infectious viruses to bind to the cells and to be taken up by the cells, an overlay containing thickening agents, such as agarose or carboxymethylcellulose, is added to prevent diffusion of viruses so that progeny viruses can only infect cells adjacent to the original infected cells. After a period of incubation to allow viral replication, cells are fixed and stained using serotype-specific anti-dengue monoclonal antibodies and a secondary antibody such as an antibody labeled with alkaline phosphatase. The foci are stained by adding a suitable substrate for the enzyme attached to the secondary antibody, such as 5-bromo-4-chloro-3-indolyl-phosphate/nitro blue tetrazolium phosphatase substrate. The number of plaques on the plate corresponds to the plaque forming units of the virus in the solutions applied to the cells. For example, a concentration of 1,000 pfu/µl indicates that 1 µl of the solution applied to the cells contains enough viruses to produce 1,000 plaques in a cell monolayer.

The dengue vaccine composition comprises a tetravalent dengue virus composition including four live attenuated dengue virus strains, wherein a chimeric dengue serotype 2/1 strain, a dengue serotype 2 strain, a chimeric dengue serotype 2/3 strain, and a chimeric dengue serotype 2/4 strain provide a total concentration in pfu/0.5 mL. The term "total concentration in pfu/0.5 mL" or "total concentration in pfu/dose" is the sum of the concentrations of the dengue serotype 1 (e.g. chimeric dengue serotype 2/1 strain), dengue serotype 2 (e.g. the dengue serotype 2 strain), the dengue serotype 3 (e.g. chimeric dengue serotype 2/3 strain) and the dengue serotype 4 (e.g. chimeric dengue serotype 2/4 strain), preferably the sum of the concentrations of TDV-1, TDV-2, TDV-3 and TDV-4, and is defined as 100% of the dengue virus concentration as determined by pfu (plaque forming units) in 0.5 mL or in a dose.

In one embodiment, the dengue vaccine composition comprises a tetravalent dengue virus composition including four live attenuated dengue virus strains, wherein a dengue serotype 1 (e.g. chimeric dengue serotype 2/1 strain), a dengue serotype 2 (e.g. dengue serotype 2 strain), a dengue serotype 3 (e.g. chimeric dengue serotype 2/3 strain), and a dengue serotype 4 (e.g. chimeric dengue serotype 2/4 strain) provide a total concentration in pfu/0.5 mL, wherein based on said total concentration the concentration of a dengue serotype 2 (e.g. dengue serotype 2 strain) measured in pfu/0.5 mL is less than 10% of the total concentration, or less than 8%, or less than 6% of the total concentration, and wherein the concentration of a dengue serotype 4 (e.g. chimeric dengue serotype 2/4 strain) measured in pfu/0.5 mL is at least 50% or at least 60% or at least 65% of the total concentration. In one embodiment, based on said total concentration the concentration of a dengue serotype 2 (e.g. dengue serotype 2 strain) measured in pfu/0.5 mL is 0.3 to 10% or 0.5 to 8% of the total concentration and the concentration of a dengue serotype 4 (e.g. chimeric dengue serotype 2/4 strain) measured in pfu/0.5 mL is 50% to 90% or 60% to 88% of the total concentration. This means that the concentration of the dengue serotype 2 (e.g. dengue serotype 2 strain) is lower than the concentration of the dengue serotype 4 (e.g. chimeric dengue serotype 2/4 strain).

In one such embodiment, the concentration of a dengue serotype 1 (e.g. chimeric dengue serotype 2/1 strain) measured in pfu/0.5 mL is at least 1% of the total concentration, and/or the concentration of a dengue serotype 3 (e.g. chimeric dengue serotype 2/3 strain) measured in pfu/0.5 mL is at least 6% of the total concentration, or at least 7% or 8%, 10%, 12%, 14%, 16% or 18% of the total concentration. In one such embodiment, the concentration of a dengue serotype 2 (e.g. chimeric dengue serotype 2/1 strain) measured in pfu/0.5 mL is 1% to 7% or 2% to 6% or 2.0% to 5.0% of the total concentration, and/or the concentration of a dengue serotype 3 (e.g. chimeric dengue serotype 2/3 strain) measured in pfu/0.5 mL is 6% to 25% or 7% to 25% or 10% to 25% or 18% to 25% of the total concentration. This means that the concentration of the dengue serotype 1 (e.g. chimeric dengue serotype 2/1 strain) is lower than the concentration of the dengue serotype 3 (e.g. chimeric dengue serotype 2/3 strain).

In a preferred embodiment, the concentration of a dengue serotype 2 strain, such as TDV-2, measured in pfu/0.5 mL is less than 10% of the total concentration, preferably less than 6% or less than 2%, the concentration of a dengue serotype 4 (e.g. chimeric dengue serotype 2/4 strain), such as TDV-4, measured in pfu/0.5 mL is at least 50% of the total concentration, preferably at least 65%, the concentration of a dengue serotype 1 (e.g. chimeric dengue serotype 2/1 strain), such as TDV-1, measured in pfu/0.5 mL is at least 1% of the total concentration, preferably between 1% and 7% or 2.0% to 5.0%, and the concentration of a dengue serotype 3 (e.g. chimeric dengue serotype 2/3 strain), such as TDV-3, measured in pfu/0.5 mL is at least 6% of the total concentration, preferably between 6% and 25% or 10% to 25% or 18% to 25%.

In a further preferred embodiment, a dengue virus composition comprising a dengue serotype 1 (e.g. chimeric dengue serotype 2/1 strain), a dengue serotype 2 (e.g. dengue serotype 2 strain), a dengue serotype 1 (e.g. chimeric dengue serotype 2/3 strain), and a dengue serotype 4 (e.g. chimeric dengue serotype 2/4 strain), such as TDV-1, TDV-2, TDV-3 and TDV-4, is provided, wherein the concentration of the dengue serotype 1 (e.g. chimeric dengue serotype 2/1 strain) measured in pfu/0.5 mL is at least 1% of the total concentration, preferably between 1% and 7% or 2.0% and 5.0%, the concentration of the dengue serotype 2 (e.g. dengue serotype 2 strain) measured in pfu/0.5 mL is less than 10% of the total concentration, preferably less than 6% or less than 2% and the concentration of the dengue serotype 3 (e.g. chimeric dengue serotype 2/3 strain) measured in pfu/0.5 mL is at least 6% of the total concentration, preferably between 6% and 25% or 10% to 25% or 18% to 25%. It is particularly preferred that the dengue serotype 4 (e.g. chimeric dengue serotype 2/4 strain) has the highest concentration of all four dengue serotypes.

In a further preferred embodiment, the dengue vaccine composition comprises a tetravalent dengue virus composition including four live attenuated dengue virus strains, wherein the concentration of the dengue serotype 1 (e.g. chimeric dengue serotype 2/1 strain) measured in pfu/0.5 mL is 1% to 7% of the total concentration, the concentration of the dengue serotype 2 (e.g. dengue serotype 2 strain) measured in pfu/0.5 mL is less than 8% of the total concentration, such as in the range of 1% to 8% of the total concentration, the concentration of the dengue serotype 3 (e.g. chimeric dengue serotype 2/3 strain) measured in pfu/0.5 mL is at least 10% of the total concentration, and the concentration of the dengue serotype 4 (e.g. chimeric dengue serotype 2/4 strain) measured in pfu/0.5 mL is at least 65% of the total concentration, such as in the range of 65% to 80%. In certain such embodiments, the arithmetic sum of all four serotypes is in the range of 4.6 log10 pfu/0.5 mL to 6.7 log10 pfu/0.5 mL, preferably in the range of 4.6 log10 pfu/0.5 mL to 5.5 log10 pfu/0.5 mL.

In a further preferred embodiment the dengue serotype 1 (e.g. chimeric dengue serotype 2/1 strain) such as TDV-1 and the dengue serotype 2 (e.g. dengue serotype 2 strain) such as TDV-2 are present each in a concentration based on the total concentration in pfu/0.5 mL which is within 5%-points of each other and/or are together less than about 10% of the total concentration in pfu/0.5 mL. In certain such embodiments the dengue serotype 3 (e.g. chimeric dengue serotype 2/3 strain) such as TDV-3 is preferably at least about 10% of the total concentration in pfu/0.5 mL and more preferably the dengue serotype 4 (e.g. chimeric dengue serotype 2/4 strain) such as TDV-4 is at least about 70% of the total concentration in pfu/0.5 mL. In certain such embodiments the dengue serotype 4 (e.g. chimeric dengue serotype 2/4 strain) such as TDV-4, represents the highest concentration in the composition of all four serotypes, preferably with at least about 70% of the total concentration in pfu/0.5 mL, dengue serotype 3 (e.g. chimeric dengue serotype 2/3 strain) such as TDV-3 represents the second highest concentration in the composition of all four serotypes, preferably with at least about 10% of the total concentration in pfu/0.5 mL, and dengue serotype 1 (e.g. chimeric dengue serotype 2/1 strain) such as TDV-1 and dengue serotype 2 (e.g. dengue serotype 2 strain) such as TDV-2 each represent lower concentrations than the concentration of serotype 3 (e.g. chimeric dengue serotype 2/3 strain) such as TDV-3, and optionally together represent less than about 10% of the total concentration in pfu/0.5 mL.

Preferably, in said embodiments the chimeric dengue serotype 2/1 strain is TDV-1, the dengue serotype 2 strain is TDV-2, the chimeric dengue serotype 2/3 strain is TDV-3 and the chimeric dengue serotype 2/4 strain is TDV-4. More preferably, TDV-1 is characterized by the nucleotide sequence according to SEQ ID No. 1 and the amino acid sequence according to SEQ ID No. 2, TDV-2 is characterized by the nucleotide sequence according to SEQ ID No. 3 and the amino acid sequence according to SEQ ID No. 4, TDV-3 is characterized by the nucleotide sequence according to SEQ ID No. 5 and the amino acid sequence according to SEQ ID No. 6 and TDV-4 is characterized by the nucleotide sequence according to SEQ ID No. 7 and the amino acid sequence according to SEQ ID No. 8.

According to a further embodiment, the chimeric dengue serotype 2/4 strain, preferably TDV-4, has the highest concentration in the dengue vaccine composition, followed by the chimeric dengue serotype 2/3 strain, preferably TDV-3, followed by the chimeric dengue serotype 2/1 strain, preferably TDV-1, followed by the dengue serotype 2 strain, preferably TDV-2. It is particularly preferred that the dengue serotype 2 strain has the lowest concentration of the four strains present in the dengue vaccine composition.

Whenever reference is made to a concentration/0.5mL, this does not limit the volume of the unit dose described herein to 0.5mL. 0.5mL is the reference volume for the determination of the concentrations of the virus strains in the composition in pfu/ml. The volume and/or amount per unit dose is described in the respective chapter.

### Virus stability

The dengue vaccine formulation of the invention as described herein provides superior stability of the tetravalent dengue virus composition comprising a live attenuated dengue virus serotype 1, a live attenuated dengue virus serotype 2, a live attenuated dengue virus serotype 3, and a live attenuated dengue virus serotype 4 (e.g. "TAK-003"), for example during lyophilization and storage.

In one embodiment, the amount of dengue virus before lyophilization and the amount of dengue virus immediately after lyophilization of the dengue vaccine formulation as described herein differs by less than 0.46 log10 pfu/unit dose, preferably by less than 0.4 log10 pfu/unit dose, more preferably by less than 0.3 log10 pfu/unit dose, or less than 0.25 log10 pfu/unit dose for each of serotypes 1 to 4. Thus, in a preferred embodiment, the lyophilization loss for each of serotypes 1 to 4 in the dengue vaccine formulation as described herein is less than 0.4 log10 pfu/unit dose.

In one embodiment, the amount of dengue virus immediately after lyophilization and the amount of dengue virus after lyophilization and storage of the dengue vaccine formulation as described herein at about 25 °C and 65% relative humidity for 12 weeks differs by less than 1.6 log10 pfu/unit dose, preferably by less than 1.4 log10 pfu/unit dose, more preferably by less than 1.2 log10 pfu/unit dose, or less than 1.0 log10 pfu/unit dose for each of serotypes 1 to 4. Thus, in a preferred embodiment, the stability loss due to storage at accelerated conditions for 12 weeks is less than 1.4 log10 pfu/unit dose for each of serotypes 1 to 4.

In one embodiment, the amount of dengue virus immediately after lyophilization and the amount of dengue virus after lyophilization and storage of the dengue vaccine formulation as described herein at 2-8 °C for 12 months differs by less than 0.44 log10 pfu/unit dose, preferably by less than 0.4 log10 pfu/unit dose, more preferably by less than 0.35 log10 pfu/unit dose for each of serotypes 1 to 4. Thus, in a preferred embodiment, the stability loss due to storage at 2-8 °C for 12 months is less than 0.4 log10 pfu/unit dose for each of serotypes 1 to 4 in the dengue vaccine formulation as described herein.

In one embodiment, the amount of dengue virus immediately after lyophilization and the amount of dengue virus after lyophilization and storage of the dengue vaccine formulation as described herein at 2-8 °C for 18 months differs by less than 0.7 log10 pfu/unit dose, preferably by less than 0.6 log10 pfu/unit dose, more preferably by less than 0.5 log10 pfu/unit dose for each of serotypes 1 to 4. Thus, in a preferred embodiment, the stability loss due to storage at 2-8 °C for 18 months is less than 0.6 log10 pfu/unit dose for each of serotypes 1 to 4 in the dengue vaccine formulation as described herein.

In one embodiment, the amount of dengue virus immediately after lyophilization and the amount of dengue virus after lyophilization and storage of the dengue vaccine formulation as described herein at 2-8 °C for 24 months differ by less than 0.7 log10 pfu/unit dose, preferably by less than 0.6 log10 pfu/unit dose, more preferably by less than 0.5 log10 pfu/unit dose for each of serotypes 1 to 4. Thus, in a preferred embodiment, the stability loss due to storage at 2-8 °C for 24 months is less than 0.6 log10 pfu/unit dose for each of serotypes 1 to 4 in the dengue vaccine formulation as described herein.

In one embodiment, the amount of dengue virus immediately after lyophilization and the amount of dengue virus after lyophilization and storage of the dengue vaccine formulation as described herein at -20 °C for 24 months differ by less than 0.2 log10 pfu/unit dose, preferably by less than 0.15 log10 pfu/unit dose, more preferably by less than 0.1 log10 pfu/unit dose for each of serotypes 1 to 4. Thus, in a preferred embodiment, the stability loss due to storage at 2-8 °C for 24 months is less than 0.15 log10 pfu/unit dose for each of serotypes 1 to 4 in the dengue vaccine formulation as described herein.

In one embodiment, the lyophilized dengue vaccine formulation as described herein has a shelf life of at least 2 years at 2-8 °C, preferably of more than 2 years at 2-8 °C. In certain such embodiments, the end of shelf life (ESL) specification for the dengue serotypes 1 to 4 is at least 3.3 log10 PFU/dose for serotype 1, at least 2.7 log10 PFU/dose for serotype 2, at least 4.0 log10 PFU/dose for serotype 3, and at least 4.5 log10 PFU/dose for serotype 4. In certain other embodiments, the end of shelf life (ESL) specification for the dengue serotypes 1 to 4 is at least 3.3 log10 PFU/dose for serotype 1, at least 3.1 log10 PFU/dose for serotype 2, at least 4.0 log10 PFU/dose for serotype 3, and at least 4.5 log10 PFU/dose for serotype 4.

In one embodiment, the lyophilized dengue vaccine formulation as described herein provides a stability which qualifies for vaccine vial monitor (VVM) Type 7. VVM Type 7 conditions are: 7 days at 37°C, 45 days at 25°C, and greater than 2 years at 2°C to 8°C. VVMs are small indicators that adhere to vaccine vials and change colour as the vaccine is exposed to cumulative heat, letting health workers know whether the vaccine has exceeded a pre-set limit beyond which the vaccine should not be used for immunization. Depending on the stability of the vaccine on exposure to heat, vaccines are assigned a VVM category.

In one embodiment, the lyophilized dengue vaccine formulation meets the end of shelf life (ESL) specification for the dengue serotypes when exposed to 37°C for 7 days, to 25°C for 45 days, or to 2°C to 8°C for greater than 2 years. In such embodiments, the end of shelf life (ESL) specification for the dengue serotypes 1 to 4 is at least 3.3 log10 PFU/dose for serotype 1, at least 2.7 log10 PFU/dose or at least 3.1 log10 PFU/dose for serotype 2, at least 4.0 log10 PFU/dose for serotype 3, and at least 4.5 log10 PFU/dose for serotype 4.

In one embodiment, the amount of dengue virus stability loss due to storage of the liquid dengue vaccine formulation as described herein at 2-8 °C for 30 days is less than 1.0 log10 pfu/unit dose for each of serotypes 1 to 4.

### Method of manufacturing

The present disclosure is directed in part to a method of preparing a dengue vaccine formulation in dry form comprising the following steps: (a) providing a dengue vaccine formulation in aqueous form, (b) freezing the dengue vaccine formulation in aqueous form to form a frozen dengue vaccine formulation, (c) subjecting the frozen dengue vaccine formulation to a primary drying step in an apparatus with a shelf temperature, which ranges from above the glass transition temperature Tg of the formulation to not more than 25 °C above, or not more than 20 °C above, or not more than 15 °C above the glass transition temperature Tg and at a pressure below 0.1 mbar to form a primary dried product, and (d) subjecting the primary dried product to a secondary drying step in an apparatus with a shelf temperature, which is above the shelf temperature of the primary drying step and at a pressure below 1 mbar to form a secondary dried product.

The present invention is in particular directed in part to a method of preparing a dengue vaccine formulation of the invention as described herein in dry form comprising the following steps:
(a) providing a dengue vaccine formulation in aqueous form comprising:
   A. a tetravalent dengue virus composition comprising:
      (i) a live attenuated dengue virus serotype 1, (ii) a live attenuated dengue virus serotype 2, (iii) a live attenuated dengue virus serotype 3, and (iv) a live attenuated dengue virus serotype 4; and
   B. an excipient composition comprising: a. trehalose, b. poloxamer, c. urea, d. arginine hydrochloride, e. tromethamine, and f. human serum albumin,
(b) freezing the dengue vaccine formulation in aqueous form in an apparatus with a shelf temperature of below -35 °C to form a frozen dengue vaccine formulation,
(c) subjecting the frozen dengue vaccine formulation to a primary drying step in an apparatus with a shelf temperature of from about -31 to -19 °C, or from about -31 to -21 °C and at a pressure below 0.1 mbar to form a primary dried product, and
(d) subjecting the primary dried product to a secondary drying step in an apparatus with a shelf temperature of at least 15 °C and at a pressure below 1 mbar, preferably below 0.1 mbar to form a secondary dried product,
wherein the dengue vaccine formulation comprises:
a. the trehalose at an amount of 20 to 34 mg/dose,
b. the poloxamer at an amount of 0.1 to 3.0 mg/dose,
c. the urea at an amount of 0.1 to 3.0 mg/dose,
d. the arginine hydrochloride at an amount of 0.1 to 2.0 mg/dose,
e. the tromethamine at an amount of 0.05 to 1.0 mg/dose, and
f. the human serum albumin at an amount of 0.05 to 0.5 mg/dose.

The present invention is in particular directed in part to a method of preparing a dengue vaccine formulation of the invention as described herein in dry form comprising the following steps:
(a) providing a dengue vaccine formulation in aqueous form comprising:
   A. a tetravalent dengue virus composition comprising:
      (i) a live attenuated dengue virus serotype 1, (ii) a live attenuated dengue virus serotype 2, (iii) a live attenuated dengue virus serotype 3, and (iv) a live attenuated dengue virus serotype 4; and
   B. an excipient composition comprising: a. trehalose, b. poloxamer, c. urea, d. arginine hydrochloride, e. tromethamine, and f. human serum albumin,
(b) freezing the dengue vaccine formulation in aqueous form in an apparatus with a shelf temperature of below -35 °C to form a frozen dengue vaccine formulation,
(c) subjecting the frozen dengue vaccine formulation to a primary drying step in an apparatus with a shelf temperature of from about -31 to -19 °C, or from about -31 to -21 °C and at a pressure below 0.1 mbar to form a primary dried product, and
(d) subjecting the primary dried product to a secondary drying step in an apparatus with a shelf temperature of at least 20 °C and at a pressure below 1 mbar to form a secondary dried product,
wherein the dengue vaccine formulation comprises:
a. the trehalose at an amount of 20 to 34 mg/dose,
b. the poloxamer at an amount of 0.1 to 3.0 mg/dose,
c. the urea at an amount of 0.1 to 3.0 mg/dose,
d. the arginine hydrochloride at an amount of 0.1 to 2.0 mg/dose,
e. the tromethamine at an amount of 0.05 to 1.0 mg/dose, and
f. the human serum albumin at an amount of 0.05 to 0.5 mg/dose.

In one embodiment, the freezing step (b) in the method as described in the preceding paragraphs is carried out in an apparatus with a shelf temperature of below -35 °C or below - 40 °C, preferably about -40 to -50 °C, such as about -45 °C.

In certain embodiments, the dengue vaccine formulation in aqueous form is subjected to the freezing step b) of the method as described in the preceding paragraphs in containers, such as vials, each comprising multiple (such as five) unit doses of the dengue vaccine formulation. In certain such embodiments, the dengue vaccine formulation in aqueous form is subjected to the freezing step b) in a volume, which is less than the volume used for reconstituting the five unit doses of the lyophilized dengue vaccine formulation. In certain preferred embodiments, the volume of the dengue vaccine formulation in aqueous form that is subjected to the freezing step b) is about half of the volume used for reconstituting the lyophilized dengue vaccine formulation.

In certain other embodiments, the dengue vaccine formulation in aqueous form is subjected to the freezing step b) of the method as described in the preceding paragraphs in containers, such as vials, each comprising a unit dose of the dengue vaccine formulation. In certain such embodiments, the dengue vaccine formulation in aqueous form is subjected to the freezing step b) in a volume, which is less than the volume used for reconstituting the unit dose of the lyophilized dengue vaccine formulation. In certain preferred embodiments, the volume of the dengue vaccine formulation in aqueous form that is subjected to the freezing step b) is about half of the volume used for reconstituting the lyophilized dengue vaccine formulation.

In one embodiment, the dengue vaccine formulation in aqueous form used in the method of drying as described in the preceding paragraphs comprises:
about 9 to 11 % w/v, such as about 10 % w/v trehalose,
about 0.2 to 0.4 % w/v, such as about 0.3 % w/v poloxamer,
about 0.2 to 0.4 % w/v, such as about 0.3 % w/v urea,
about 0.2 to 0.3 % w/v, such as about 0.25 % w/v arginine hydrochloride,
about 0.05 to 0.15 % w/v, such as about 0.1 % w/v tromethamine, and
about 0.05 to 0.15 % w/v, such about 0.1 % w/v human serum albumin.

In one embodiment, the primary drying step (c) in the method as described in the preceding paragraphs is carried out in an apparatus with a shelf temperature of from about -31 °C to -19 °C, or from about -31 to -21 °C, preferably about -29 to -25 °C, such as about -27 °C and optionally at a pressure of less than 0.05 mbar, preferably of from about 0.01 to less than 0.05 mbar, or of about 0.04 mbar. In certain such embodiments, the primary drying step (c) is carried out for a period of from about 20 to 120 hours, preferably about 30 to 100 hours, more preferably about 40 to 70 hours, or about 50 to 70 hours.

In a preferred embodiment, the primary drying step (c) in the method as described in the preceding paragraphs is carried out in an apparatus with a shelf temperature of from about -31 °C to -21 °C, such as about -27 °C or about -24°C, and at a pressure of less than 0.05 mbar, preferably of from about 0.01 to less than 0.05 mbar, such as of about 0.03 mbar.

In one embodiment, the secondary drying step (d) is carried out in an apparatus with a shelf temperature of at least 15 °C, or at least 20 °C, or of from about 15 to 40 °C, or from about 20 to 40 °C, or about 25 °C and optionally at a pressure of less than 0.5 mbar, preferably of from about 0.1 to less than 0.4 mbar, or of about 0.3 mbar. In certain such embodiments, the secondary drying step (d) is carried out for a period of from about 1 to 15 hours, preferably about 3 to 10 hours, more preferably about 6 to 10 hours, or about 7 to 9 hours.

In a preferred embodiment, the secondary drying step (d) is carried out in an apparatus with a shelf temperature of at least 15 °C, such as 25°C, and at a pressure of less than 0.05 mbar, preferably of from about 0.01 to less than 0.04 mbar, such as about 0.03 mbar.

In a preferred embodiment, the same pressure is used in the primary drying step and in the secondary drying step. In certain such embodiments, the pressure in the primary drying step and the secondary drying step is from about 0.025 mbar to 0.035 mbar such as about 0.030 mbar.

In one embodiment, the primary and secondary drying step in the method as described in the preceding paragraphs is carried out for a period of less than 160 hours, preferably about 50 to 120 hours, more preferably about 50 to less than 100 hours, or about 60 to 90 hours.

In a preferred embodiment, the steps b), c) and d) of the method of preparing a lyophilized dengue vaccine formulation as described in the preceding paragraphs is carried out for a period of less than 120 hours, preferably about 50 to less than 100 hours, more preferably about 40 to less than 90 hours.

In one embodiment, the primary dried product in the method as described in the preceding paragraphs has a residual moisture content of about 5 to 10 % w/w, or less than 10 % w/w as determined by Karl Fischer Determination. In one embodiment, the secondary dried product has a residual moisture content of not more than 3.0% w/w, or of about 0.01 to 2 % w/w, preferably less than 2 % w/w, more preferably less than 1 % w/w as determined by Karl Fischer Determination.

### Uses

The present disclosure is directed in part to a method of preventing dengue disease (in particular virologically confirmable dengue, VCD) in a subject. Thus, in certain embodiments the disclosure is directed to a method of preventing dengue disease in a subject, comprising administering to the subject, a unit dose, in particular a reconstituted unit dose, of the dengue vaccine formulation as described herein, for example by subcutaneous injection or intramuscular administration.

The present disclosure is in particular directed in part to a method of preventing dengue disease in a subject comprising administering a primary vaccination with only two administrations of a unit dose of the dengue vaccine formulation as described herein comprising the steps of: administering a first unit dose of the dengue vaccine formulation to the subject, and administering a second unit dose of the dengue vaccine formulation to the subject within 3 months of administration of the first unit dose, and optionally administering a booster dose at least 12 months after administration of said second unit dose of the dengue vaccine.

The present invention is further directed in part to a dengue vaccine formulation of the invention as described herein for use in a method of preventing dengue disease (in particular virologically confirmable dengue, VCD) in a subject. Thus, in certain embodiments the invention is directed to a dengue vaccine formulation of the invention as described herein for use in a method of preventing dengue disease in a subject, wherein the method comprises administering to the subject, a unit dose, in particular a reconstituted unit dose, of the dengue vaccine formulation, for example by subcutaneous injection or intramuscular administration.

The present invention is in particular directed in part to a dengue vaccine formulation of the invention as described herein for use in a method of preventing dengue disease in a subject, wherein the method comprises administering a primary vaccination with only two administrations of a unit dose comprising the steps of: administering a first unit dose of the dengue vaccine formulation to the subject, and administering a second unit dose of the dengue vaccine formulation to the subject, such as within 3 months of administration of the first unit dose, and optionally administering a booster dose at least 12 months after administration of said second unit dose of the dengue vaccine.

The present disclosure is further directed in part to a use of the dengue vaccine formulation as described herein for the manufacture of a medicament for preventing dengue disease (in particular virologically confirmable dengue, VCD) in a subject. Thus, in certain embodiments, the disclosure is directed to a use of the dengue vaccine formulation as described herein for the manufacture of a medicament for preventing dengue disease in a subject by the administration of a unit dose, in particular a reconstituted unit dose, of the dengue vaccine formulation to the subject, for example by subcutaneous injection or intramuscular administration.

The present disclosure is in particular directed in part to a use of the dengue vaccine formulation as described herein for the manufacture of a medicament for preventing dengue disease in a subject by the administration of a primary vaccination with only two administrations of a unit dose comprising the administration of a first unit dose of the dengue vaccine formulation to the subject, and the administration of a second unit dose of the dengue vaccine formulation to the subject, such as within 3 months of administration of the first unit dose, and optionally the administration of a booster dose at least 12 months after administration of said second unit dose of the dengue vaccine.

### EXAMPLES

The following examples are included to demonstrate certain aspects and embodiments of the invention as described in the claims. It should be appreciated by those of skill in the art, however, that the following description is illustrative only and should not be taken in any way as a restriction of the invention. The concentrations and amounts given in the following tables are rounded off to, for example, three or less significant digits.

### Preparation of the dengue virus strains and the tetravalent dengue virus composition

The methods used to generate the chimeric dengue strains TDV-1, -3 and -4 were standard molecular cloning and DNA engineering methods and are described in Huang et al. (2003) J. Virology 77(21): 11436-11447. The following well-known methods were used to construct and introduce the prM-E genes of dengue serotypes 1, 3 and 4 into the TDV-2 backbone: Reverse-transcriptase PCR (RT-PCR), PCR, restriction enzyme digestion, DNA fragment ligation, bacterial transformations by electroporation, plasmid DNA preparations, *in vitro* transcription by T7 RNA polymerase, and transfection of Vero cells by electroporation. Growing and purifying of the different dengue serotypes were conducted as described in Huang et al. (2013) PLOS Neglected Dis, 7(5):e2243.

The chimeric dengue strains TDV-1, -3 and -4 were combined with TDV-2 to form a tetravalent dengue virus composition in the concentrations described herein.

### EXAMPLE 1

### Preparation of the dengue vaccine formulation in aqueous form

The tetravalent dengue virus composition was combined with pharmaceutically acceptable excipients resulting in a dengue vaccine formulation in aqueous form comprising the excipient concentrations shown in Table 5.

**Table 5. Excipient contributions to the dengue vaccine formulation in aqueous form prior to lyophilization.**

| **Component** | **g/L** | **%(w/v)** |
|---|---|---|
| α,α-Trehalose, dihydrate | 100 | 10 |
| Kolliphor^{®} P407 (Pluronic^{®} F-127, BioReagent, Poloxamer 407) | 3 | 0.3 |
| Human serum albumin | 1 | 0.1 |
| L-Arginine hydrochloride | 2.5 | 0.25 |
| Urea | 3.0 | 0.3 |
| Tromethamine | 1.2 | 0.12 |
| Potassium dihydrogen phosphate | 0.04 | 0.004 |
| Disodium hydrogen phosphate, dihydrate | 0.35 | 0.035 |
| Potassium chloride | 0.03 | 0.003 |
| Sodium chloride | 2.2 | 0.22 |
| Hydrochloric acid (1 M HCl, pH adjustment to pH 7.4 ± 0.2) | | |

### Preparation of the dengue vaccine formulation in dry form

For the provision of five unit doses of the dengue vaccine formulation, 1.65 mL of the dengue vaccine formulation in aqueous form were lyophilized in a vial according to the process shown in Table 6.

**Table 6. Lyophilization cycle steps.**

| **Step** | **Parameter** | **Target (Shelf Temperature/ Chamber Pressure)** | **Actual (Shelf Temperature / Chamber Pressure)** |
|---|---|---|---|
| | | | |
| Loading | Temp (°C) | 5 | 6.2 to 6.7 |
| | Time (hh:mm) | 00:30 | 00:30 |
| | Pressure (µm Hg) | <ATM | <ATM |
| | | | |
| Freezing Rate | Rate (°C/time) | 0.5/min | 0.5/min |
| | Total time (hh:mm) | 1:20 | n/a |
| | Pressure (µm Hg) | <ATM | <ATM |
| | | | |
| Freezing Hold | Temp (°C) | -45 | -43.7 to -43.1 |
| | Time (hh:mm) | 01:00 | 01:00 |
| | Pressure (µm Hg) | <ATM | <ATM |
| | | | |
| Primary Drying Ramp | Rate (°C/time) | 0.5/min | 0.5/min |
| | Total time (hh:mm) | 0:36 | 0:36 |
| | Pressure (µm Hg) | 30 ± 10 | 30 ± 10 |
| | | | |
| Primary Drying Hold | Temp (°C) | -27 | -26.0 to -25.3 |
| | Time (hh:mm) | 65:00 | 65:00 |
| | Pressure (µm Hg) | 30 ± 10 | 33 to 34 |
| | | | |
| Secondary drying Ramp | Rate (°C/time) | 0.1/min | 0.1/min |
| | Total time (hh:mm) | 08:40 | n/a |
| | Pressure (µm Hg) | 250 ± 10 | 250 ± 10 |
| | | | |
| Secondary drying Hold | Temp (°C) | 25 | 25.7 to 26.3 |
| | Time (hh:mm) | 8:00 | 8:00 |
| | Pressure (µm Hg) | 250 ± 10 | 247 to 247 |
| | Total time(hh:mm) on production scale | <100:00 | <100:00 |

The excipient composition of the lyophilized dengue vaccine formulation is shown in Table 7. The residual moisture content as determined by Karl Fischer Determination was 0.58 % w/w.

**Table 7. Excipient composition of the dengue vaccine formulation in dry form.**

| **Component** | **mg/vial** | **% (w/w)** |
|---|---|---|
| α,α-Trehalose | 149.3 | 87.0 |
| Kolliphor^{®} P407 (Pluronic^{®} F-127, BioReagent, Poloxamer 407) | 4.95 | 2.9 |
| Human serum albumin | 1.65 | 0.96 |
| L-Arginine hydrochloride | 4.1 | 2.4 |
| Urea | 4.95 | 2.9 |
| Tromethamine | 2.0 | 1.2 |
| Potassium dihydrogen phosphate | 0.06 | 0.03 |
| Disodium hydrogen phosphate | 0.47 | 0.3 |
| Potassium chloride | 0.05 | 0.03 |
| Sodium chloride | 3.59 | 2.1 |
| Hydrochloric acid | 0.48 | 0.3 |
| Total | 171.7 | 100 |

The characteristics of the cake of the lyophilized dengue vaccine formulation are summarized in Table 8.

**Table 8. Cake characteristics of the lyophilized dengue vaccine formulation.**

| | |
|---|---|
| Color | White |
| Structure | Dense |
| Dimension | Fill Height: 8 mm, Cake height 5-6 mm and shrinkage around 1mm uniform |
| Topography | Concave and Textured |
| Finish | Top: Sheen, Sides and Bottom: Matte |
| Friability Inversion | Cake remained intact and at the bottom of the vial |
| Friability Jarring | Cake fell to the top of vial with some powder and fragments breaking off |
| Residual Material | Variable thickness, white film at the original fill height |

When the cake, i.e. the lyophilized dengue vaccine formulation was reconstituted with 3.25 mL 37 mM aqueous sodium chloride solution, the appearance after 70 seconds was clear and colorless with a ring of bubbles on the surface. The observed pH was 7.6 according to the USP <791> test method for pH measurements of the United States Pharmacopeial Convention. The observed osmolality was about 300 mOsmol/kg according to the USP <785> test method for osmolality measurements of the United States Pharmacopeial Convention. The excipient concentrations in the reconstituted dengue vaccine formulation are shown in Table 9.

**Table 9. Excipient composition of the dengue vaccine formulation in aqueous form after reconstitution.**

| **Component** | **g/L** | **%(w/v)** | **mg/unit dose of 0.5 mL** |
|---|---|---|---|
| α,α-Trehalose | 45.95 | 4.6 | 22.97 |
| Kolliphor^{®} P407 (Pluronic^{®} F-127, BioReagent, Poloxamer 407) | 1.52 | 0.15 | 0.76 |
| Human serum albumin | 0.51 | 0.05 | 0.25 |
| L-Arginine hydrochloride | 1.3 | 0.13 | 0.63 |
| Urea | 1.52 | 0.15 | 0.76 |
| Tromethamine | 0.61 | 0.06 | 0.31 |
| Potassium dihydrogen phosphate | 0.02 | 0.002 | 0.01 |
| Disodium hydrogen phosphate | 0.14 | 0.014 | 0.07 |
| Potassium chloride | 0.02 | 0.002 | 0.01 |
| Sodium chloride | 3.27 | 0.33 | 1.64 |
| Hydrochloric acid | 0.15 | 0.015 | 0.07 |

### EXAMPLE 2

Using the process described in Example 1 a dengue vaccine formulation containing 10 mM tromethamine (0.12 % w/v) prior to lyophilization was prepared by using a combination of tromethamine and tromethamine hydrochloride (Tris-HCl). The excipient composition of the lyophilized dengue vaccine formulation is shown in Table 10.

**Table 10. Excipient composition of the dengue vaccine formulation in dry form.**

| **Component** | **mg/vial** | **% (w/w)** |
|---|---|---|
| Urea | 5.0 | 2.88 |
| L-Arginine Hydrochloride | 4.1 | 2.40 |
| Sodium Chloride | 3.59 | 2.09 |
| Tromethamine | 0.33 | 0.19 |
| Tris-HCl | 2.17 | 1.27 |
| α,α-Trehalose | 149.3 | 86.98 |
| Human Serum Albumin | 1.65 | 0.96 |
| Potassium dihydrogen phosphate | 0.06 | 0.04 |
| Disodium hydrogen phosphate | 0.47 | 0.27 |
| Potassium Chloride | 0.05 | 0.03 |
| Pluronic^{®} F-127 | 4.95 | 2.88 |
| Total | 171.7 | 100.00 |

As described in Example 1, one vial contains five unit doses of the dengue vaccine formulation. At the time of administration, the vial is reconstituted with the entire contents of a vial or pre-filled syringe of diluent (e.g. 37 mM sodium chloride solution) so that five 0.5 mL doses of vaccine can be withdrawn and administered.

After reconstituting the vial with 3.38 mL 37 mM aqueous sodium chloride solution to a volume of about 3.49 mL, a 0.5 mL dose contains, for example, about 0.47 g/L human serum albumin (1.65 mg/vial : 3.49 mL), or about 0.24 mg human serum albumin (0.47 g/L x 0.5 mL), respectively.

### EXAMPLE 3

### Preparation of the dengue vaccine formulation in aqueous form

The tetravalent dengue virus composition as described herein was combined with pharmaceutically acceptable excipients resulting in a dengue vaccine formulation in aqueous form comprising the excipient concentrations shown in Table 11.

**Table 11. Excipient contributions to the dengue vaccine formulation in aqueous form prior to lyophilization.**

| **Component** | **g/L** | **%(w/v)** |
|---|---|---|
| α,α-Trehalose, dihydrate | 100.00 | 10 |
| Kolliphor^{®} P407 (Pluronic^{®} F-127, BioReagent, Poloxamer 407) | 3.00 | 0.3 |
| Human serum albumin | 1.00 | 0.1 |
| L-Arginine hydrochloride | 2.50 | 0.25 |
| Urea | 3.00 | 0.3 |
| Tromethamine | 1.20 | 0.12 |
| Potassium dihydrogen phosphate | 0.04 | 0.004 |
| Disodium hydrogen phosphate, dihydrate | 0.29 | 0.029 |
| Potassium chloride | 0.03 | 0.003 |
| Sodium chloride | 2.17 | 0.22 |
| Hydrochloric acid (1 M HCl, pH adjustment to pH 7.4 ± 0.2) | | |

### Preparation of the dengue vaccine formulation in dry form

For the provision of five unit doses of the dengue vaccine formulation, 1.65 mL of the dengue vaccine formulation in aqueous form were lyophilized in a vial according to the process shown in Table 12. Once a pressure rise test is successfully completed, the chamber pressure is increased to 700 mbar for stoppering using dry Nitrogen (N2). The shelf temperature is decreased to 5°C in preparation for stoppering. The total lyophilization time from the 5°C equilibration to stoppering step is approximately 72 hours.

**Table 12. Lyophilization parameters.**

| | | |
|---|---|---|
| Shelf temperature loading | | +5°C |
| Freezing | Ramp | 0.5°C/min |
| | Time | 01:40 |
| | Final Temperature | -45°C |
| | Chamber Pressure | atmosphere |
| | Hold Time | 02:20 |
| Primary | Ramp | 0.5°C/min |
| | Time | 00:42 |
| | Final Temperature | -24°C |
| | Chamber Pressure | 0.030 mbar |
| | Hold Time | 55:00 |
| Secondary | Ramp | 0.1°C/min |
| | Time | 08:10 |
| | Final Temperature | +25°C |
| | Chamber Pressure | 0.030 mbar |
| | Hold Time | 08:00 |
| Storage temperature | | +5°C |
| Chamber pressure after drying | | 700 mbar |
| *Time is listed in (hh:mm)* | | |

The excipient composition of the lyophilized dengue vaccine formulation is shown in Table 13.

**Table 13. Excipient composition of the dengue vaccine formulation in dry form.**

| **Component** | **mg/vial** | **% (w/w)** |
|---|---|---|
| α,α-Trehalose | 149.28 | 87.03 |
| Kolliphor^{®} P407 (Pluronic^{®} F-127, BioReagent, Poloxamer 407) | 4.95 | 2.89 |
| Human serum albumin | 1.65 | 0.96 |
| L-Arginine hydrochloride | 4.12 | 2.40 |
| Urea | 4.95 | 2.9 |
| Tromethamine | 2.0 | 1.2 |
| Potassium dihydrogen phosphate | 0.06 | 0.03 |
| Disodium hydrogen phosphate | 0.38 | 0.22 |
| Potassium chloride | 0.05 | 0.03 |
| Sodium chloride | 3.59 | 2.1 |
| Hydrochloric acid | 0.48 | 0.3 |
| Total | 171.6 | 100 |

The excipient concentrations in the reconstituted dengue vaccine formulation after reconstitution with 3.38 mL 37 mM aqueous sodium chloride solution to a volume of about 3.49 mL are shown in Table 14.

**Table 14. Excipient composition of the dengue vaccine formulation in aqueous form after reconstitution.**

| **Component** | **g/L** | **%(w/v)** | **mg/unit dose of 0.5 mL** |
|---|---|---|---|
| α,α-Trehalose | 42.77 | 4.3 | 21.39 |
| Kolliphor^{®} P407 (Pluronic^{®} F-127, BioReagent, Poloxamer 407) | 1.42 | 0.14 | 0.71 |
| Human serum albumin | 0.47 | 0.05 | 0.24 |
| L-Arginine hydrochloride | 1.18 | 0.12 | 0.59 |
| Urea | 1.42 | 0.14 | 0.71 |
| Tromethamine | 0.57 | 0.06 | 0.29 |
| Potassium dihydrogen phosphate | 0.02 | 0.002 | 0.01 |
| Disodium hydrogen phosphate | 0.11 | 0.011 | 0.05 |
| Potassium chloride | 0.02 | 0.002 | 0.01 |
| Sodium chloride | 3.12 | 0.31 | 1.56 |
| Hydrochloric acid | 0.14 | 0.014 | 0.07 |

The physiochemical attributes of the dengue vaccine formulation are summarized in Table 15.

**Table 15. Physiochemical attributes of the dengue vaccine formulation.**

| | |
|---|---|
| pH (USP <791> Ph. Eur. 2.2.3) | 7.4 |
| Residual Moisture (Karl Fischer) (USP <921> Ph. Eur. 2.5.32 | ≤3.0% |
| Appearance Visual Method (lyophilized) | White to off-white colored compact cake |
| Re-suspension Time | ≤60 seconds |
| Appearance Visual Method (Reconstituted liquid) | Clear, colorless to pale yellow solution, essentially free of foreign particulate |

### EXAMPLE 4

Using the process described in Example 3 a dengue vaccine formulation containing 10 mM tromethamine (0.12 % w/v) prior to lyophilization was prepared by using a combination of tromethamine and tromethamine hydrochloride (Tris-HCl). The excipient concentrations in the dengue vaccine formulation before lyophilization are shown in Table 16.

**Table 16. Excipient contributions to the dengue vaccine formulation in aqueous form prior to lyophilization.**

| **Component** | **g/L** | **%(w/v)** |
|---|---|---|
| α,α-Trehalose, dihydrate | 100.00 | 10.00 |
| Kolliphor^{®} P407 (Pluronic^{®} F-127, BioReagent, Poloxamer 407) | 3.00 | 0.30 |
| Human serum albumin | 1.00 | 0.10 |
| L-Arginine hydrochloride | 2.50 | 0.25 |
| Urea | 3.00 | 0.30 |
| Tromethamine (Tris) | 0.20 | 0.02 |
| Tris-HCl | 1.32 | 0.13 |
| Potassium dihydrogen phosphate | 0.04 | 0.004 |
| Disodium hydrogen phosphate, dihydrate | 0.28 | 0.028 |
| Potassium chloride | 0.03 | 0.003 |
| Sodium chloride | 2.17 | 0.22 |

For the provision of five unit doses of the dengue vaccine formulation, 1.65 mL of the dengue vaccine formulation in aqueous form were lyophilized in a vial. The total lyophilization cycle time was approximately 77 hours.The excipient composition of the lyophilized dengue vaccine formulation is shown in Table 17.

**Table 17. Excipient composition of the dengue vaccine formulation in dry form.**

| **Component** | **mg/vial** | **% (w/w)** |
|---|---|---|
| Urea | 4.95 | 2.89 |
| L-Arginine Hydrochloride | 4.12 | 2.40 |
| Sodium Chloride | 3.59 | 2.09 |
| Tromethamine | 0.33 | 0.19 |
| Tris-HCl | 2.17 | 1.27 |
| α,α-Trehalose | 149.28 | 87.03 |
| Human Serum Albumin | 1.65 | 0.96 |
| Potassium dihydrogen phosphate | 0.06 | 0.04 |
| Disodium hydrogen phosphate | 0.37 | 0.22 |
| Potassium Chloride | 0.05 | 0.03 |
| Pluronic^{®} F-127 | 4.95 | 2.89 |
| Total | 171.5 | 100.00 |

The excipient concentrations in the reconstituted dengue vaccine formulation after reconstitution with 3.38 mL 37 mM aqueous sodium chloride solution to a volume of about 3.49 mL are shown in Table 18.

**Table 18. Excipient composition of the dengue vaccine formulation in aqueous form after reconstitution.**

| **Component** | **g/L** | **%(w/v)** |
|---|---|---|
| Urea | 1.42 | 0.14 |
| L-Arginine Hydrochloride | 1.18 | 0.12 |
| Sodium Chloride | 3.12^{a} | 0.31^{a} |
| Tromethamine | 0.09 | 0.01 |
| Tris-HCl | 0.62 | 0.06 |
| α,α-Trehalose | 42.77 | 4.28 |
| Human Serum Albumin | 0.47 | 0.05 |
| Potassium dihydrogen phosphate | 0.02 | 0.002 |
| Disodium hydrogen phosphate | 0.11 | 0.01 |
| Potassium Chloride | 0.02 | 0.002 |
| Pluronic^{®} F-127 | 1.42 | 0.14 |
| *(a) Includes contribution from lyophilized formulation and 37 mM sodium chloride diluent.* | | |

For the provision of one unit dose of the dengue vaccine formulation, 0.33 mL of the dengue vaccine formulation in aqueous form were lyophilized in a vial. The total lyophilization cycle time was approximately 49 hours. The excipient composition of the lyophilized dengue vaccine formulation is shown in Table 19.

**Table 19. Excipient composition of the dengue vaccine formulation in dry form.**

| **Component** | **mg/vial** | **% (w/w)** |
|---|---|---|
| Urea | 0.99 | 2.89 |
| L-Arginine Hydrochloride | 0.82 | 2.40 |
| Sodium Chloride | 0.72 | 2.09 |
| Tromethamine | 0.07 | 0.19 |
| Tris-HCl | 0.43 | 1.27 |
| α,α-Trehalose | 29.86 | 87.03 |
| Human Serum Albumin | 0.33 | 0.96 |
| Potassium dihydrogen phosphate | 0.01 | 0.04 |
| Disodium hydrogen phosphate | 0.07 | 0.22 |
| Potassium Chloride | 0.01 | 0.03 |
| Pluronic^{®} F-127 | 0.99 | 2.89 |
| Total | 34.3 | 100.00 |

The excipient concentrations in the reconstituted dengue vaccine formulation after reconstitution with 0.68 mL 37 mM aqueous sodium chloride solution to a volume of about 0.70 mL are shown in Table 20.

**Table 20. Excipient composition of the dengue vaccine formulation in aqueous form after reconstitution.**

| **Component** | **g/L** | **%(w/v)** | **mg/unit dose of 0.5 mL** |
|---|---|---|---|
| Urea | 1.41 | 0.14 | 0.71 |
| L-Arginine Hydrochloride | 1.18 | 0.12 | 0.59 |
| Sodium Chloride | 3.12^{a} | 0.318 | 1.56 |
| Tromethamine | 0.09 | 0.01 | 0.05 |
| Tris-HCl | 0.62 | 0.06 | 0.31 |
| α,α-Trehalose | 42.65 | 4.27 | 21.33 |
| Human Serum Albumin | 0.47 | 0.05 | 0.24 |
| Potassium dihydrogen phosphate | 0.02 | 0.002 | 0.01 |
| Disodium hydrogen phosphate | 0.11 | 0.01 | 0.05 |
| Potassium Chloride | 0.02 | 0.002 | 0.01 |
| Pluronic^{®} F-127 | 1.41 | 0.14 | 0.71 |
| *(a) Includes contribution from lyophilized formulation and 37 mM sodium chloride diluent.* | | | |

The composition of the dengue vaccine formulation prepared according to Example 4 is shown in Table 21. The physiochemical attributes of the dengue vaccine formulation correspond to those described in Table 15 above.

**Table 21. Composition of the dengue vaccine formulation.**

| **Ingredients** | | **Quantity per Dose^{b}** |
|---|---|---|
| Active^{a} | Serotype TDV-1 | ≥ 3.3 log₁₀ PFU/dose |
| | Serotype TDV-2 | ≥ 2.7 log₁₀ PFU/dose |
| | Serotype TDV-3 | ≥ 4.0 log₁₀ PFU/dose |
| | Serotype TDV-4 | ≥ 4.5 log₁₀ PFU/dose |
| Excipients | α,α-Trehalose, dihydrate | 23.64 mg/dose |
| | Kolliphor^{®} P407 (F-127) | 0.71 mg/dose |
| | Human Serum Albumin | 0.24 mg/dose |
| | Potassium dihydrogen phosphate | 0.07 µmoles/dose |
| | Disodium hydrogen phosphate dihydrate | 0.38 µmoles/dose |
| | Tromethamine | 0.39 µmoles/dose |
| | Tris HCl | 1.97 µmoles/dose |
| | L-Arginine HCl | 2.81 µmoles/dose |
| | Urea | 11.81 µmoles/dose |
| | Potassium chloride | 0.10 µmoles/dose |
| | Sodium chloride | 26.66 µmoles/dose^{c} |
| *(a) The four monovalent drug substances of the tetravalent dengue vaccine are referred to* as *TDV. Concentrations listed align with the End of Shelf Life (ESL) specification range for the approved product "Qdenga", current approved ESL for TDV2 is* ≥ *3.1 log10 PFU*/*dose for Indonesia.* | | |
| *(b) The administered dose volume is 0.5 mL.* | | |
| *(c)Includes contribution from lyophilized formulation and 37 mM sodium chloride diluent.* | | |

### COMPARATIVE EXAMPLE

### Preparation of the dengue virus control formulation in aqueous form

The tetravalent dengue virus composition was combined with a composition of pharmaceutically acceptable excipients resulting in a dengue vaccine control formulation ("Control") in aqueous form comprising the excipient concentrations shown in Table 22.

**Table 22. Excipient contributions to the dengue vaccine control formulation in aqueous form prior to lyophilization.**

| **Component** | **g/L** | **%(w/v)** |
|---|---|---|
| α,α-Trehalose, dihydrate | 165.8 | 16.58 |
| Kolliphor^{®} P407 (Pluronic^{®} F-127, BioReagent, Poloxamer 407) | 10 | 1 |
| Human serum albumin | 1 | 0.1 |
| Potassium dihydrogen phosphate | 0.24 | 0.02 |
| Disodium hydrogen phosphate, dihydrate | 1.78 | 0.18 |
| Potassium chloride | 0.2 | 0.02 |
| Sodium chloride | 5.8 | 0.58 |

### Preparation of the dengue virus control formulation in dry form

For the provision of five unit doses of the dengue vaccine control formulation, 3.25 mL of the dengue vaccine control formulation in aqueous form were lyophilized in a vial according to the process shown in Table 23.

**Table 23. Lyophilization cycle steps.**

| **Step** | **Parameter** | **Target (Shelf Temperature/ Chamber Pressure)** | **Actual (Shelf Temperature / Chamber Pressure)** |
|---|---|---|---|
| | | | |
| Loading | Temp (°C) | 5 | 6.2 to 6.7 |
| | Time (hh:mm) | 00:30 | 00:30 |
| | Pressure (µm Hg) | <ATM | <ATM |
| | | | |
| Freezing Rate | Rate (°C/time) | 0.5/min | 0.5/min |
| | Total time (hh:mm) | 1:20 | n/a |
| | Pressure (µm Hg) | <ATM | <ATM |
| | | | |
| Freezing Hold | Temp (°C) | -45 | -43.7 to -43.1 |
| | Time (hh:mm) | 01:00 | 01:00 |
| | Pressure (µm Hg) | <ATM | <ATM |
| | | | |
| Primary Drying Ramp | Rate (°C/time) | 0.5/min | 0.5/min |
| | Total time (hh:mm) | 0:36 | 0:36 |
| | Pressure (µm Hg) | 30 ± 10 | 30 ± 10 |
| | | | |
| Primary Drying Hold | Temp (°C) | -27 | -26.0 to -25.3 |
| | Time (hh:mm) | 136:00 | 136:00 |
| | Pressure (µm Hg) | 30 ± 10 | 33 to 34 |
| | | | |
| Secondary drying Ramp | Rate (°C/time) | 0.1/min | 0.1/min |
| | Total time (hh:mm) | 08:40 | n/a |
| | Pressure (µm Hg) | 250 ± 10 | 250 ± 10 |
| | | | |
| Secondary drying Hold | Temp (°C) | 25 | 25.7 to 26.3 |
| | Time (hh:mm) | 10:00 | 10:00 |
| | Pressure (µm Hg) | 250 ± 10 | 247 to 247 |
| | Total time(hh:mm) on production scale | about 160:00 | about 160:00 |

The excipient composition of the lyophilized dengue vaccine control formulation is shown in Table 24. The residual moisture content was 0.77 % w/w as determined by Karl Fischer Determination.

**Table 24. Excipient composition of the dengue vaccine control formulation in dry form.**

| **Component** | **% (w/w)** |
|---|---|
| α,α-Trehalose | 88.9 |
| Kolliphor^{®} P407 (Pluronic^{®} F-127, BioReagent, Poloxamer 407) | 5.9 |
| Human serum albumin | 0.6 |
| Potassium dihydrogen phosphate | 0.1 |
| Disodium hydrogen phosphate | 0.8 |
| Potassium chloride | 0.1 |
| Sodium chloride | 3.4 |
| Total | 100 |

The characteristics of the cake of the lyophilized dengue vaccine control formulation are summarized in Table 25.

**Table 25. Cake characteristics of the lyophilized dengue vaccine control formulation.**

| | |
|---|---|
| Color | White |
| Structure | Dense |
| Dimension | Fill Height: 12-13 mm, Cake height 11-12 mm and shrinkage around 1mm uniform |
| Topography | Concave with cracks, textured with chips at the edge |
| Finish | Top: Sheen, Sides and Bottom: Matte |
| Friability Inversion | Cake remained intact and moved slightly in the vial |
| Friability Jarring | Cake fell to the top of vial with some powder and fragments breaking off |
| Residual Material | Thick white film at the original fill height and where the cake was seated |

When the cake, i.e. the lyophilized dengue vaccine control formulation was reconstituted with 3.25 mL 37 mM aqueous sodium chloride solution, the appearance after 20 seconds was clear and colorless with a ring of bubbles on the surface. The observed pH was 7.6 according to the USP <791> test method for pH measurements of the United States Pharmacopeial Convention. The observed osmolality was about 800 mOsmol/kg according to the USP <785> test method for osmolality measurements of the United States Pharmacopeial Convention. The excipient concentrations in the reconstituted control formulation are shown in Table 26.

**Table 26. Excipient composition of the dengue vaccine control formulation in aqueous form after reconstitution.**

| **Component** | **g/L** | **%(w/v)** | **mg/unit dose of 0.5 mL** |
|---|---|---|---|
| α,α-Trehalose | 150 | 15.0 | 75 |
| Kolliphor^{®} P407 (Pluronic^{®} F-127, BioReagent, Poloxamer 407) | 10 | 1 | 5 |
| Human serum albumin | 1 | 0.1 | 0.5 |
| Potassium dihydrogen phosphate | 0.2 | 0.02 | 0.12 |
| Disodium hydrogen phosphate | 1.4 | 0.01 | 0.71 |
| Potassium chloride | 0.2 | 0.02 | 0.1 |
| Sodium chloride | 8 | 0.8 | 4 |

**Table 27. Comparison of excipient composition of the dengue vaccine formulations according to Examples 1 and 4 and the dengue vaccine control formulation in aqueous form after reconstitution.**

| **Component** | **mg/unit dose of 0.5 mL** | | | |
|---|---|---|---|---|
| | Formulation acc. to Example 1 | Control formulation | Formulation acc. to Example 3 | Formulation acc. to Example 4 |
| α,α-Trehalose | 23 | 75 | 21.39 | 21.39 |
| Kolliphor^{®} P407 (Pluronic^{®} F-127, BioReagent, Poloxamer 407) | 0.76 | 5 | 0.71 | 0.71 |
| Human serum albumin | 0.25 | 0.5 | 0.24 | 0.24 |
| L-Arginine hydrochloride | 0.63 | - | 0.59 | 0.59 |
| Urea | 0.76 | - | 0.71 | 0.71 |
| Tromethamine | 0.31 | - | 0.29 | 0.05 |
| Potassium dihydrogen phosphate | 0.01 | 0.12 | 0.01 | 0.01 |
| Disodium hydrogen phosphate | 0.07 | 0.71 | 0.05 | 0.05 |
| Potassium chloride | 0.01 | 0.1 | 0.01 | 0.01 |
| Sodium chloride | 1.64 | 4 | 1.56 | 1.56 |
| Hydrochloric acid | 0.07 | - | 0.07 | - |
| Tris HCl | - | - | - | 0.31 |

**Table 28. Comparison of the dengue vaccine formulation according to Example 4 and the dengue vaccine control formulation.**

| **Ingredients** | | **Quantity per Dose^{b}** | |
|---|---|---|---|
| | | **Example 4** | **Control** |
| Active^{a} | Serotype TDV-1 | ≥ 3.3 log₁₀ PFU/dose | ≥ 3.3 log₁₀ PFU/dose |
| | Serotype TDV-2 | ≥ 2.7 log₁₀ PFU/dose | ≥ 2.7 log₁₀ PFU/dose |
| | Serotype TDV-3 | ≥ 4.0 log₁₀ PFU/dose | ≥ 4.0 log₁₀ PFU/dose |
| | Serotype TDV-4 | ≥ 4.5 log₁₀ PFU/dose | ≥ 4.5 log₁₀ PFU/dose |
| Excipients | α,α-Trehalose, dihydrate | 23.64 mg/dose | 82.9 mg/dose |
| | Kolliphor^{®} P407 (F-127) | 0.71 mg/dose | 5.0 mg/dose |
| | Human Serum Albumin | 0.24 mg/dose | 0.5 mg/dose |
| | Potassium dihydrogen phosphate | 0.07 µmoles/dose | 0.88 µmoles/dose |
| | Disodium hydrogen phosphate dihydrate | 0.38 µmoles/dose | 5.01 µmoles/dose |
| | Tromethamine | 0.39 µmoles/dose | - |
| | Tris HCl | 1.97 µmoles/dose | - |
| | L-Arginine HCl | 2.81 µmoles/dose | - |
| | Urea | 11.81 µmoles/dose | - |
| | Potassium chloride | 0.10 µmoles/dose | 1.35 µmoles/dose |
| | Sodium chloride | 26.66 µmoles/dose^{a} | 68.5 µmoles/dose^{c} |
| *(a) The four monovalent drug substances of the tetravalent dengue vaccine are referred to* as *TDV. Concentrations listed align with the End of Shelf Life (ESL) specification range for the approved product "Qdenga", current approved ESL for TDV2 is* ≥ *3.1 log 10 PFU*/*dose for Indonesia.* | | | |
| *(b) The administered dose volume is 0.5 mL.* | | | |
| *(c) Includes contribution from lyophilized formulation and 37 mM sodium chloride diluent.* | | | |

### STABILITY MEASUREMENTS

Virus concentrations are determined by an immune focus assay that results in plaque forming units (pfu). In one example, the immune focus assay is carried out as described in detail in section 2.5 of Brewoo et al. (Vaccine. 2012 February 14; 30(8): 1513-1520. doi:10.1016/j.vaccine.2011.11.072.). Each sample was measured in triplicate and the results were reported as an average of the values.

### Lyophilization loss

The log10 loss in virus potency due to lyophilization was determined for the dengue vaccine formulation prepared according to Example 1 and the dengue vaccine control formulation prepared according to the comparative example. The results are shown in Table 29.

**Table 29. Log10 loss in virus titer due to lyophilization.**

| | **TDV-1** | **TDV-2** | **TDV-3** | **TDV-4** |
|---|---|---|---|---|
| Lyophilization loss Control formulation | -0.46 | -0.33 | -0.40 | -0.33 |
| Lyophilization loss Formulation acc. to Ex. 1 | -0.20 | -0.11 | -0.31 | -0.09 |

### Stability loss

The log10 loss in virus titer due to storage at a temperature of 25 °C and a relative humidity RH of 65% for 1, 2, 4, 6, 8, and 12 weeks was determined for the dengue vaccine formulation prepared according to Example 1 and the dengue vaccine control formulation prepared according to the comparative example. The results for the 12 weeks time point are shown in Table 30.

**Table 30. Log10 loss in virus titer due to storage at accelerated conditions for 12 weeks time point.**

| | **TDV-1** | **TDV-2** | **TDV-3** | **TDV-4** |
|---|---|---|---|---|
| Accelerated stability loss Control formulation | -1.19 | -1.44 | -1.35 | -1.60 |
| Accelerated stability loss Formulation acc. to Ex. 1 | -0.52 | -0.38 | -0.60 | -0.92 |

The log10 loss in virus titer due to storage at a temperature of 2-8 °C for 3, 6, 9, and 12 months was determined for the dengue vaccine formulation prepared according to Example 1 and the dengue vaccine control formulation prepared according to the comparative example. The results are shown in Table 31 and **Fig. 1** and **Fig. 2****.**

**Table 31. Log10 loss in virus titer at 2-8 °C for 12 months time point.**

| | **TDV-1** | **TDV-2** | **TDV-3** | **TDV-4** |
|---|---|---|---|---|
| Stability loss Control formulation | -0.38 | -0.31 | -0.41 | -0.44 |
| Stability loss Formulation acc. to Ex. 1 | -0.34 | -0.29 | -0.21 | -0.26 |

The log10 loss in virus titer due to storage at a temperature of 2-8 °C for 6, 9, 12, 18, and 24 months was determined for the dengue vaccine formulation prepared according to Example 3 and the dengue vaccine control formulation prepared according to the comparative example. The results are shown in Table 32 and **Fig 4** and **Fig. 5****.**

**Table 32. Log10 loss in virus titer at 2-8 °C for 24 months time point.**

| | **TDV-1** | **TDV-2** | **TDV-3** | **TDV-4** |
|---|---|---|---|---|
| Stability loss Control formulation | -0.32 | -0.59 | -0.73 | -0.86 |
| Stability loss Formulation acc. to Ex. 3 | -0.10 | -0.28 | -0.27 | -0.52 |

The log10 loss in virus titer due to storage at a temperature of -20 °C for 24 months was determined for the dengue vaccine formulation prepared according to Example 3 and the dengue vaccine control formulation prepared according to the comparative example. The results are shown in Table 33.

**Table 33. Log10 loss in virus titer at -20 °C for 24 months time point.**

| | **TDV-1** | **TDV-2** | **TDV-3** | **TDV-4** |
|---|---|---|---|---|
| Stability loss Control formulation | -0.24 | -0.05 | -0.07 | -0.11 |
| Stability loss Formulation acc. to Ex. 3 | -0.09 | -0.06 | -0.01 | -0.09 |

The time in solution (TIS) duration up to 36 hours was studied for the dengue vaccine formulation prepared according to Example 4 and the dengue vaccine control formulation prepared according to the comparative example. TIS post-formulation begins with completion of formulation additions and ends at the initiation of the lyophilization process. The results are shown in Table 34.

**Table 34. Post-formulation TIS data (Degradation rate).**

| | **TDV-1** | **TDV-2** | **TDV-3** | **TDV-4** |
|---|---|---|---|---|
| | log PFU/ml/hr | log PFU/ml/hr | log PFU/ml/hr | log PFU/ml/hr |
| Degradation rate Control formulation | -0.0159 | -0.0092 | -0.0291 | -0.0029 |
| Degradation rate Formulation acc. to Ex. 4 | -0.0131 | -0.0050 | -0.0094 | -0.0024 |
| Ratio (Control / Ex. 4) | 1.21 | 1.84 | 3.10 | 1.21 |

Post-formulation TIS degradation rates for all TDV serotypes have been improved for the formulation according to the present invention compared to the control formulation. With a loss rate that is 3.1 times lower than post-formulation TIS for the control formulation, TDV3 exhibits the greatest improvement. The second-best improvement, TDV2, had a loss rate that was 1.84 times lower than the post-formulation TIS for the control formulation. The loss rate showed a small improvement in TDV1 and TDV4.

### COMPARATIVE STUDY

The objective of this study was to evaluate the differences in performance and characteristics between the formulation according to the present invention and the formulation 20 proposed in US 2020/0390877, Example 11. The excipient concentrations of formulation 20 are shown in table 35.

**Table 35. Excipient contributions Formulation 20.**

| Excipient | Concentration (g/L) |
|---|---|
| Potassium phosphate monobasic anhydrous ACS 99% | 1.50 |
| Sucrose | 90.00 |
| Sodium chloride | 2.92 |
| Sodium Carboxyl Methyl Cellulose | 5.00 |
| Propylene Gylcol | 5.00 |
| L Leucine | 3.28 |
| Adjust pH to 7.5 | |

For comparing the stabilizing effect of formulation 20 and the inventive formulation on dengue virus, TDV1, TDV2, TDV3, and TDV4 drug substance was formulated with formulation 20 ("comparative formulation 20" in the following) and was tested against the inventive formulation according to Example 4 disclosed herein. The final concentration of drug substance was the same in both formulations as shown in table 36.

**Table 36. Composition of dengue formulations.**

| Total batch volume: 250 mL | | |
|---|---|---|
| | Formulation acc. to Example 4 | Comparative formulation 20 |
| TDV1 (g) | 12.12 | 12.12 |
| TDV2 (g) | 12.40 | 12.40 |
| TDV3 (g) | 1.08 | 1.08 |
| TDV4 (g) | 0.36 | 0.36 |
| Excipients (g) | 233.82 | 233.82 |
| | (trehalose dihydrate, poloxamer 407, HSA, disodium hydrogen phosphate dihydrate, tromethamine, Tris HCl, L-Arginine HCl, Urea, potassium chloride sodium chloride) | (potassium phosphate, sucrose, sodium chloride, sodium carboxylmethylcellulose, propylene glycol, L-leucine) |

### Preliminary observations

The excipient composition of formulation 20 was more than 2-times more viscous than the excipient composition of Example 4. While 1 L of the excipient composition of Example 4 could be filtered through 0.22 µm filter for sterilization, the excipient composition of formulation 20 clogged the 0.22 µm cup filter after filtering 300 mL, so 2 x 1 L 0.22 µm filter cups were used as well as 1 x 500 mL 0.22 µm filter cup to filter the whole volume. Filter clogging could be due to the high viscosity of the excipient composition of formulation 20 and/or the presence of excipients such as sodium carboxy methyl cellulose.

Each excipient composition was combined with the tetravalent dengue virus composition resulting in a dengue vaccine formulation in aqueous form. The biophysical properties of the dengue vaccine formulations in aqueous form are shown in table 37.

**Table 37. Biophysical properties of dengue formulations.**

| | Formulation acc. to Example 4 | Comparative formulation 20 |
|---|---|---|
| Density at 20°C (g/mL) | 1.039 | 1.042 |
| Dynamic Viscosity (cP) | 1.4 | 4.0 |
| Final pH | 7.56 | 7.57 |
| Osmolality (mOsm/kg) | 495.00±1.00 | 595.67±5.78 |

### Stability measurements

The liquid stability of the comparative formulation 20 and the formulation according to Example 4 was assessed at 2 - 8 °C for a total duration of 30 days using Immuno-Focus Assay (IFA) for potency. Samples were taken at time points: Day 0 and Day 30. Day 0 samples were frozen in -80 °C till the time of testing. The results are shown in Table 38 and **Fig. 6****.**

**Table 38. Mean virus titer at day 0, and at day 30 after exposure of the liquid formulations to 2-8 °C.**

| | **Titer (log10 PFU/dose)** | | | | | | | |
|---|---|---|---|---|---|---|---|---|
| | **TDV-1** | | **TDV-2** | | **TDV-3** | | **TDV-4** | |
| | Day 0 | Day 30 | Day 0 | Day 30 | Day 0 | Day 30 | Day 0 | Day 30 |
| Comparative Formulation 20 | 6.07 | 3.93 | 5.18 | 3.07 | 5.92 | 2.38 | 6.62 | 2.63 |
| Formulation acc. to Example 4 | 6.21 | 5.54 | 5.35 | 4.9 | 5.91 | 4.99 | * | 4.72 |
| * The value was above the detection limit of the assay. | | | | | | | | |

Comparative formulation 20 showed a significantly higher potency loss after 30 days compared to the formulation according to Example 4.

## Claims

1. A dengue vaccine formulation comprising:
A. a tetravalent dengue virus composition comprising:
(i) a live attenuated dengue virus serotype 1,
(ii) a live attenuated dengue virus serotype 2,
(iii) a live attenuated dengue virus serotype 3, and
(iv) a live attenuated dengue virus serotype 4; and
B. an excipient composition comprising:
a. trehalose,
b. poloxamer,
c. urea,
d. arginine hydrochloride,
e. tromethamine, and
f. human serum albumin,
wherein the dengue vaccine formulation comprises:
a. the trehalose at an amount of 20 to 34 mg/dose,
b. the poloxamer at an amount of 0.1 to 3.0 mg/dose,
c. the urea at an amount of 0.1 to 3.0 mg/dose,
d. the arginine hydrochloride at an amount of 0.1 to 2.0 mg/dose,
e. the tromethamine at an amount of 0.05 to 1.0 mg/dose, and
f. the human serum albumin at an amount of 0.05 to 0.5 mg/dose.

2. The dengue vaccine formulation according to claim 1, wherein the ratio of the amount of arginine hydrochloride to the amount of human serum albumin contained in the dengue vaccine formulation is 1:1 to 4:1.

3. The dengue vaccine formulation according to claim 1 or 2, wherein the ratio of the amount of urea to the amount of human serum albumin contained in the dengue vaccine formulation is 1:1 to 5:1 and/or wherein the ratio of the amount of urea to the amount of poloxamer contained in the dengue vaccine formulation is 1:1 to 2:1.

4. The dengue vaccine formulation according to any one of claims 1 to 3, wherein phosphate salts are contained in the dengue vaccine formulation at an amount of less than 0.3 mg/dose.

5. The dengue vaccine formulation according to any one of claims 1 to 4, wherein chloride salts are contained in the dengue vaccine formulation at an amount of less than 2.0 mg/dose.

6. The dengue vaccine formulation according to any one of claims 1 to 5, wherein hydrochloric acid is contained in the dengue vaccine formulation at an amount of 0.01 to 0.5 mg/dose.

7. The dengue vaccine formulation according to any one of claims 1 to 6, preferably in dry form, wherein the dengue vaccine formulation comprises based on the dry weight of the vaccine formulation:
a. 65 to 95 % w/w trehalose,
b. 0.05 to 5.0 % w/w poloxamer,
c. 0.1 to 6.0 % w/w urea,
d. 0.1 to 5.0 % w/w arginine hydrochloride,
e. 0.05 to 5.0 % w/w tromethamine, and
f. 0.05 to 3.0 % w/w human serum albumin.

8. The dengue vaccine formulation according to any one of claims 1 to 7, wherein a dose of the dengue vaccine formulation in dry form upon reconstitution with a pharmaceutically acceptable aqueous diluent to a volume of 0.5 mL comprises:
a. the trehalose in a concentration of from 40 to 80 g/L,
b. the poloxamer in a concentration of 0.1 to 5.0 g/L,
c. the urea in a concentration of 0.1 to 6.0 g/L,
d. the arginine hydrochloride in a concentration of 0.5 to 5.0 g/L,
e. the tromethamine in a concentration of 0.1 to 1.5 g/L, and
f. the human serum albumin in a concentration of from 0.1 to less than 1 g/L.

9. The dengue vaccine formulation according to any one of claims 1 to 8, wherein a dose of the dengue vaccine formulation in dry form upon reconstitution with a pharmaceutically acceptable aqueous diluent to a volume of 0.5 mL comprises chloride salts in a concentration of less than 5 g/L.

10. The dengue vaccine formulation according to any one of claims 1 to 9, wherein a dose of the dengue vaccine formulation in dry form upon reconstitution with a pharmaceutically acceptable aqueous diluent to a volume of 0.5 mL has an osmolality of 100 to 700 mOsmol/kg.

11. The dengue vaccine formulation according to any one of claims 1 to 10, wherein a dose of the dengue vaccine formulation in dry form upon reconstitution with a pharmaceutically acceptable aqueous diluent to a volume of 0.5 mL comprises:
(i) the dengue virus serotype 1 in an amount of at least 3.3 log10 pfu/0.5 mL,
(ii) the dengue virus serotype 2 in an amount of at least 2.7 log10 pfu/0.5 mL,
(iii) the dengue virus serotype 3 in an amount of at least 4.0 log10 pfu/0.5 mL, and
(iv) the dengue virus serotype 4 in an amount of at least 4.5 log10 pfu/0.5 mL.

12. The dengue vaccine formulation according to any one of claims 1 to 11, wherein the tetravalent dengue virus composition includes at least one non-chimeric dengue virus and at least one chimeric dengue virus.

13. The dengue vaccine formulation according to any one of claims 1 to 12, wherein the dengue virus serotype 1 is a chimeric dengue serotype 2/1 strain, the dengue virus serotype 2 is a non-chimeric dengue serotype 2 strain, the dengue virus serotype 3 is a chimeric dengue serotype 2/3 strain and the dengue virus serotype 4 is a chimeric dengue serotype 2/4 strain, preferably the dengue serotype 1 is TDV-1, the dengue serotype 2 is TDV-2, the dengue serotype 3 is TDV-3 and the dengue serotype 4 is TDV-4.

14. The dengue vaccine formulation according to any one of claims 1 to 13, wherein the tetravalent dengue virus composition is "TAK-003".

15. The dengue vaccine formulation according to any one of claims 1 to 14, which is a lyophilized dengue vaccine formulation.

16. A container, preferably a vial, comprising one or more than one dose of the dengue vaccine formulation according to any one of claims 1 to 15.

17. A container, preferably a vial comprising five doses of the dengue vaccine formulation according to any one of claims 1 to 15.

18. The container, preferably the vial, according to claim 17, wherein
a. the trehalose is contained at an amount of 100 to 170 mg,
b. the poloxamer is contained at an amount of 2.0 to 8.0 mg,
c. the urea is contained at an amount of 2.0 to 8.0 mg,
d. the arginine hydrochloride is contained at an amount of 1.5 to 7.5 mg,
e. the tromethamine is contained at an amount of 0.5 to 4.0 mg, and
f. the human serum albumin is contained at an amount of 0.1 to 2.5 mg.

19. A kit comprising more than one, preferably ten, container(s) according to any one of claims 16 to 18, and optionally one or more than one, preferably ten, container(s) comprising a pharmaceutically acceptable aqueous diluent for reconstitution.

20. A kit for preparing a dengue vaccine formulation in reconstituted form comprising:
a) a container, preferably a vial, comprising the dengue vaccine formulation according to any one of claims 1 to 15 in dry form, and
b) a container, preferably a vial, comprising a pharmaceutically acceptable aqueous diluent for reconstitution.

21. The kit according to claim 19 or 20, wherein the pharmaceutically acceptable aqueous diluent is selected from the group consisting of water for injection and an aqueous sodium chloride solution, preferably the pharmaceutically acceptable aqueous diluent is 37 mM aqueous sodium chloride solution.

22. A dose of a dengue vaccine formulation in aqueous form obtainable by reconstituting one to ten, preferably five, dose(s) of the dengue vaccine formulation according to claims 1 to 15 in dry form with an equal number of aliquots of a pharmaceutically acceptable aqueous diluent and removing the volume of a dose of the reconstituted dengue vaccine formulation.

23. The dose according to claim 22, wherein the aliquot has a volume of 0.4 to 0.8 mL/dose, preferably of 0.4 to 0.7 mL/dose, more preferably of 0.45 to 0.65 mL/dose.

24. A method of preparing a dengue vaccine formulation according to any one of claims 1 to 15 in dry form comprising the following steps:
(a) providing a dengue vaccine formulation in aqueous form comprising:
A. a tetravalent dengue virus composition comprising:
(i) a live attenuated dengue virus serotype 1,
(ii) a live attenuated dengue virus serotype 2,
(iii) a live attenuated dengue virus serotype 3, and
(iv) a live attenuated dengue virus serotype 4; and
B. an excipient composition comprising:
a. trehalose,
b. poloxamer,
c. urea,
d. arginine hydrochloride,
e. tromethamine, and
f. human serum albumin,
(b) freezing the dengue vaccine formulation in aqueous form in an apparatus with a shelf temperature of below -35 °C to form a frozen dengue vaccine formulation,
(c) subjecting the frozen dengue vaccine formulation to a primary drying step in an apparatus with a shelf temperature of from -31 to -19 °C, for example of from -31 to -21 °C, and at a pressure below 0.1 mbar to form a primary dried product, and
(d) subjecting the primary dried product to a secondary drying step in an apparatus with a shelf temperature of at least 15 °C, for example at least 20 °C, and at a pressure below 1 mbar to form a secondary dried product;
wherein the dengue vaccine formulation comprises:
a. the trehalose at an amount of 20 to 34 mg/dose,
b. the poloxamer at an amount of 0.1 to 3.0 mg/dose,
c. the urea at an amount of 0.1 to 3.0 mg/dose,
d. the arginine hydrochloride at an amount of 0.1 to 2.0 mg/dose,
e. the tromethamine at an amount of 0.05 to 1.0 mg/dose, and
f. the human serum albumin at an amount of 0.05 to 0.5 mg/dose.

25. The method according to claim 24, wherein the dengue vaccine formulation in aqueous form is subjected to the freezing step b) in a volume, which is less than the volume used for reconstituting the lyophilized dengue vaccine formulation, preferably the dengue vaccine formulation in aqueous form is subjected to the freezing step b) in containers, such as vials, each comprising one or more than one dose of the dengue vaccine formulation in a volume, which is about half of the volume used for reconstituting the one or more than one dose of the lyophilized dengue vaccine formulation.

26. The dengue vaccine formulation according to any one of claims 1 to 15 for use in a method of preventing dengue disease in a subject, wherein the method comprises administering a primary vaccination with only two administrations of a dose comprising the steps of:
administering a first dose of the dengue vaccine formulation to the subject, and
administering a second dose of the dengue vaccine formulation to the subject, preferably within 3 months of administration of the first dose,
and optionally administering a booster dose at least 12 months after administration of said second dose of the dengue vaccine formulation.

## Patentansprüche

1. Dengue-Impfstoffformulierung, umfassend:
A. eine tetravalente Dengue-Virus-Zusammensetzung, umfassend:
(i) einen Dengue-Virus-Serotyp 1 (lebend, attenuiert),
(ii) einen Dengue-Virus-Serotyp 2 (lebend, attenuiert),
(iii) einen Dengue-Virus-Serotyp 3 (lebend, attenuiert), und
(iv) einen Dengue-Virus-Serotyp 4 (lebend, attenuiert), und
B. eine Hilfsstoffzusammensetzung, umfassend:
a. Trehalose,
b. Poloxamer,
c. Harnstoff,
d. Argininhydrochlorid,
e. Tromethamin, und
f. Humanserumsalbumin,
wobei die Dengue-Impfstoffformulierung umfasst:
a. die Trehalose in einer Menge von 20 bis 34 mg/Dosis,
b. das Poloxamer in einer Menge von 0,1 bis 3,0 mg/Dosis,
c. den Harnstoff in einer Menge von 0,1 bis 3,0 mg/Dosis,
d. das Argininhydrochlorid in einer Menge von 0,1 bis 2,0 mg/Dosis,
e. das Tromethamin in einer Menge von 0,05 bis 1,0 mg/Dosis, und
f. das Humanserumsalbumin in einer Menge von 0,05 bis 0,5 mg/Dosis.

2. Dengue-Impfstoffformulierung nach Anspruch 1, wobei das Verhältnis der Menge an Argininhydrochlorid zu der Menge an humanem Serumalbumin, die in der Dengue-Impfstoffformulierung enthalten ist, 1:1 bis 4:1 ist.

3. Dengue-Impfstoffformulierung nach Anspruch 1 oder 2, wobei das Verhältnis der Menge an Harnstoff zu der Menge an humanem Serumalbumin, die in der Dengue-Impfstoffformulierung enthalten ist, 1:1 bis 5:1 ist und/oder wobei das Verhältnis der Menge an Harnstoff zu der Menge an Poloxamer, die in der Dengue-Impfstoffformulierung enthalten ist, 1:1 bis 2:1 ist.

4. Dengue-Impfstoffformulierung nach einem der Ansprüche 1 bis 3, wobei die Dengue-Impfstoffformulierung Phosphatsalze in einer Menge von weniger als 0,3 mg/Dosis enthält.

5. Dengue-Impfstoffformulierung nach einem der Ansprüche 1 bis 4, wobei die Dengue-Impfstoffformulierung Chloridsalze in einer Menge von weniger als 2,0 mg/Dosis enthält.

6. Dengue-Impfstoffformulierung nach einem der Ansprüche 1 bis 5, wobei die Dengue-Impfstoffformulierung Salzsäure in einer Menge von 0,01 bis 0,5 mg/Dosis enthält.

7. Dengue-Impfstoffformulierung nach einem der Ansprüche 1 bis 6, vorzugsweise in trockener Form, wobei die Dengue-Impfstoffformulierung bezogen auf das Trockengewicht der Impfstoffformulierung Folgendes umfasst:
a. 65-95 % (w/w) Trehalose,
b. 0,05-5,0 % (w/w) Poloxamer,
c. 0,1-6,0 % (w/w) Harnstoff,
d. 0,1-5,0 % (w/w) Argininhydrochlorid,
e. 0,05-5,0 % (w/w) Tromethamin und
f. 0,05-3,0 % (w/w) Humanserumsalbumin.

8. Dengue-Impfstoffformulierung nach einem der Ansprüche 1 bis 7, wobei eine Dosis der Dengue-Impfstoffformulierung in trockener Form nach Rekonstitution mit einem pharmazeutisch verträglichen wässrigen Verdünnungsmittel auf ein Volumen von 0,5 ml Folgendes umfasst:
a. die Trehalose in einer Konzentration von 40 bis 80 g/l,
b. das Poloxamer in einer Konzentration von 0,1 bis 5,0 g/l,
c. den Harnstoff in einer Konzentration von 0,1 bis 6,0 g/l,
d. das Argininhydrochlorid in einer Konzentration von 0,5 bis 5,0 g/l,
e. das Tromethamin in einer Konzentration von 0,1 bis 1,5 g/l, und
f. das Humanserumalbumin in einer Konzentration von 0,1 bis weniger als 1 g/l.

9. Dengue-Impfstoffformulierung nach einem der Ansprüche 1 bis **8,** wobei eine Dosis der Dengue-Impfstoffformulierung in trockener Form nach Rekonstitution mit einem pharmazeutisch verträglichen wässrigen Verdünnungsmittel auf ein Volumen von 0,5 ml Chloridsalze in einer Konzentration von weniger als 5 g/l umfasst.

10. Dengue-Impfstoffformulierung nach einem der Ansprüche 1 bis 9, wobei eine Dosis der Dengue-Impfstoffformulierung in trockener Form nach Rekonstitution mit einem pharmazeutisch verträglichen wässrigen Verdünnungsmittel auf ein Volumen von 0,5 ml eine Osmolalität von 100 bis 700 mOsmol/kg aufweist.

11. Dengue-Impfstoffformulierung nach einem der Ansprüche 1 bis 10, wobei eine Dosis der Dengue-Impfstoffformulierung in trockener Form nach Rekonstitution mit einem pharmazeutisch verträglichen wässrigen Verdünnungsmittel auf ein Volumen von 0,5 ml Folgendes umfasst:
(i) den Dengue-Virus-Serotyp 1 in einer Menge von mindestens 3,3 log10 pfu/0,5 ml,
(ii) den Dengue-Virus-Serotyp 2 in einer Menge von mindestens 2,7 log10 pfu/0,5 ml,
(iii) den Dengue-Virus-Serotyp 3 in einer Menge von mindestens 4,0 log10 pfu/0,5 ml, und
(iv) den Dengue-Virus-Serotyp 4 in einer Menge von mindestens 4,5 log10 pfu/0,5 ml.

12. Dengue-Impfstoffformulierung nach einem der Ansprüche 1 bis 11, wobei die tetravalente Dengue-Virus-Zusammensetzung mindestens ein nicht-chimäres Dengue-Virus und mindestens ein chimäres Dengue-Virus aufweist.

13. Dengue-Impfstoffformulierung nach einem der Ansprüche 1 bis 12, wobei der Dengue-Virus-Serotyp 1 ein chimärer Dengue-Serotyp-2/1-Stamm ist, der Dengue-Virus-Serotyp 2 ein nicht-chimärer Dengue-Serotyp-2-Stamm ist, der Dengue-Virus-Serotyp 3 ein chimärer Dengue-Serotyp-2/3-Stamm ist und der Dengue-Virus-Serotyp 4 ein chimärer Dengue-Serotyp-2/4-Stamm ist, wobei vorzugsweise der Dengue-Serotyp 1 TDV-1 ist, der Dengue-Serotyp 2 TDV-2 ist, der Dengue-Serotyp 3 TDV-3 ist und der Dengue-Serotyp 4 TDV-4 ist.

14. Dengue-Impfstoffformulierung nach einem der Ansprüche 1 bis 13, wobei die tetravalente Dengue-Virus-Zusammensetzung "TAK-003" ist.

15. Dengue-Impfstoffformulierung nach einem der Ansprüche 1 bis 14, die eine lyophilisierte Dengue-Impfstoffformulierung ist.

16. Behälter, vorzugsweise eine Ampulle, der eine oder mehr als eine Dosis der Dengue-Impfstoffformulierung nach einem der Ansprüche 1 bis 15 umfasst.

17. Behälter, vorzugsweise eine Ampulle, der fünf Dosen der Dengue-Impfstoffformulierung nach einem der Ansprüche 1 bis 15 umfasst.

18. Behälter, vorzugsweise die Ampulle, nach Anspruch 17, wobei
a. die Trehalose in einer Menge von 100 bis 170 mg enthalten ist,
b. das Poloxamer in einer Menge von 2,0 bis 8,0 mg enthalten ist,
c. der Harnstoff in einer Menge von 2,0 bis 8,0 mg enthalten ist,
d. das Argininhydrochlorid in einer Menge von 1,5 bis 7,5 mg enthalten ist,
e. das Tromethamin in einer Menge von 0,5 bis 4,0 mg enthalten ist und
f. das Humanserumalbumin in einer Menge von 0,1 bis 2,5 mg enthalten ist.

19. Kit, das mehr als einen, vorzugsweise zehn, Behälter nach einem der Ansprüche 16 bis 18 und gegebenenfalls einen oder mehr als einen, vorzugsweise zehn, Behälter umfasst, die ein pharmazeutisch verträgliches wässriges Verdünnungsmittel zur Rekonstitution umfassen.

20. Kit zum Herstellen einer Dengue-Impfstoffformulierung in rekonstituierter Form, umfassend:
a) einen Behälter, vorzugsweise eine Ampulle, der die Dengue-Impfstoffformulierung nach einem der Ansprüche 1 bis 15 in trockener Form umfasst, und
b) einen Behälter, vorzugsweise eine Ampulle, der ein pharmazeutisch verträgliches wässriges Verdünnungsmittel zur Rekonstitution umfasst.

21. Kit nach Anspruch 19 oder 20, wobei das pharmazeutisch verträgliche wässrige Verdünnungsmittel aus der Gruppe ausgewählt ist, die aus Wasser zur Injektion und einer wässrigen Natriumchloridlösung besteht, vorzugsweise das pharmazeutisch verträgliche wässrige Verdünnungsmittel eine 37 mM wässrige Natriumchloridlösung ist.

22. Dosis einer Dengue-Impfstoffformulierung in wässriger Form, die durch Rekonstitution einer bis zehn, vorzugsweise fünf, Dosen der Dengue-Impfstoffformulierung nach einem der Ansprüche 1 bis 15 in trockener Form mit einer gleichen Anzahl von Aliquoten eines pharmazeutisch verträglichen wässrigen Verdünnungsmittels sowie durch Entfernen des Volumens einer Dosis der rekonstituierten Dengue-Impfstoffformulierung erlangt werden kann.

23. Dosis nach Anspruch 22, wobei das Aliquot ein Volumen von 0,4 bis 0,8 ml/Dosis, vorzugsweise von 0,4 bis 0,7 ml/Dosis, noch bevorzugter von 0,45 bis 0,65 ml/Dosis aufweist.

24. Verfahren zum Herstellen einer Dengue-Impfstoffformulierung nach einem der Ansprüche 1 bis 15 in trockener Form, das die folgenden Schritte umfasst:
(a) Bereitstellen einer Dengue-Impfstoffformulierung in wässriger Form, umfassend:
A. eine tetravalente Dengue-Virus-Zusammensetzung, umfassend:
(i) einen Dengue-Virus-Serotyp 1 (lebend, attenuiert),
(ii) einen Dengue-Virus-Serotyp 2 (lebend, attenuiert),
(iii) einen Dengue-Virus-Serotyp 3 (lebend, attenuiert), und
(iv) einen Dengue-Virus-Serotyp 4 (lebend, attenuiert), und
B. eine Hilfsstoffzusammensetzung, umfassend:
a. Trehalose,
b. Poloxamer,
c. Harnstoff,
d. Argininhydrochlorid,
e. Tromethamin und
f. Humanserumsalbumin,
(b) Einfrieren der Dengue-Impfstoffformulierung in wässriger Form in einer Vorrichtung mit einer Lagertemperatur von unter -35 °C, um eine gefrorene Dengue-Impfstoffformulierung zu bilden,
(c) Unterziehen der gefrorenen Dengue-Impfstoffformulierung einem primären Trocknungsschritt in einer Vorrichtung mit einer Lagertemperatur von -31 °C bis -19 °C, beispielsweise von -31 °C bis -21 °C, und bei einem Druck unter 0,1 mbar, um ein primär getrocknetes Produkt zu bilden, und
(d) Unterziehen des primär getrockneten Produkts einem sekundären Trocknungsschritt in einer Vorrichtung mit einer Lagertemperatur von mindestens 15 °C, z. B. mindestens 20 °C, und bei einem Druck von weniger als 1 mbar, um ein sekundär getrocknetes Produkt zu bilden;
wobei die Dengue-Impfstoffformulierung umfasst:
a. die Trehalose in einer Menge von 20 bis 34 mg/Dosis,
b. das Poloxamer in einer Menge von 0,1 bis 3,0 mg/Dosis,
c. den Harnstoff in einer Menge von 0,1 bis 3,0 mg/Dosis,
d. das Argininhydrochlorid in einer Menge von 0,1 bis 2,0 mg/Dosis,
e. das Tromethamin in einer Menge von 0,05 bis 1,0 mg/Dosis, und
f. das Humanserumsalbumin in einer Menge von 0,05 bis 0,5 mg/Dosis.

25. Verfahren nach Anspruch 24, wobei die Dengue-Impfstoffformulierung in wässriger Form dem Gefrierschritt b) in einem Volumen unterzogen wird, das kleiner ist als das Volumen, das für die Rekonstitution der lyophilisierten Dengue-Impfstoffformulierung verwendet wird, vorzugsweise die Dengue-Impfstoffformulierung in wässriger Form dem Gefrierschritt b) in Behältern, wie etwa Ampullen, unterzogen wird, die jeweils eine oder mehr als eine Dosis der Dengue-Impfstoffformulierung in einem Volumen enthalten, das etwa die Hälfte des Volumens beträgt, das für die Rekonstitution einer oder mehr als einer Dosis der lyophilisierten Dengue-Impfstoffformulierung verwendet wird.

26. Dengue-Impfstoffformulierung nach einem der Ansprüche 1 bis 15 zur Verwendung in einem Verfahren zur Prävention einer Dengue-Erkrankung bei einem Probanden, wobei das Verfahren das Verabreichen einer primären Impfung mit nur zwei Verabreichungen einer Dosis umfasst, die die folgenden Schritte umfasst:
Verabreichen einer ersten Dosis der Dengue-Impfstoffformulierung an den Probanden, und
Verabreichen einer zweiten Dosis der Dengue-Impfstoffformulierung an den Probanden, vorzugsweise innerhalb von 3 Monaten nach Verabreichung der ersten Dosis,
und optional das Verabreichen einer Auffrischungsdosis mindestens 12 Monate nach Verabreichung der zweiten Dosis der Dengue-Impfstoffformulierung.

## Revendications

1. Formulation de vaccin contre la dengue comprenant :
A. une composition tétravalente de virus de la dengue comprenant :
(i) un virus de la dengue vivant atténué de sérotype 1,
(ii) un virus de la dengue vivant atténué de sérotype 2,
(iii) un virus de la dengue vivant atténué de sérotype 3, et
(iv) un virus de la dengue vivant atténué de sérotype 4 ; et
B. une composition d'excipients comprenant :
a. du tréhalose,
b. du poloxamère,
c. de l'urée,
d. du chlorhydrate d'arginine,
e. de la trométhamine, et
f. de l'albumine sérique humaine,
dans laquelle la formulation de vaccin contre la dengue comprend :
a. du tréhalose à une quantité de 20 à 34 mg/dose,
b. du poloxamère à une quantité de 0,1 à 3,0 mg/dose,
c. de l'urée à une quantité de 0,1 à 3,0 mg/dose,
d. du chlorhydrate d'arginine à une quantité de 0,1 à 2,0 mg/dose,
e. de la trométhamine à une quantité de 0,05 à 1,0 mg/dose, et
f. de l'albumine sérique humaine à une quantité de 0,05 à 0,5 mg/dose.

2. Formulation de vaccin contre la dengue selon la revendication 1, dans laquelle le rapport de la quantité de chlorhydrate d'arginine à la quantité d'albumine sérique humaine contenue dans la formulation de vaccin contre la dengue est de 1:1 à 4:1.

3. Formulation de vaccin contre la dengue selon la revendication 1 ou 2, dans laquelle le rapport de la quantité d'urée à la quantité d'albumine sérique humaine contenue dans la formulation de vaccin contre la dengue est de 1:1 à 5:1 et/ou dans laquelle le rapport de la quantité d'urée à la quantité de poloxamère contenue dans la formulation de vaccin contre la dengue est de 1:1 à 2:1.

4. Formulation de vaccin contre la dengue selon l'une quelconque des revendications 1 à 3, dans laquelle les sels de phosphate sont contenus dans la formulation de vaccin contre la dengue en une quantité inférieure à 0,3 mg/dose.

5. Formulation de vaccin contre la dengue selon l'une quelconque des revendications 1 à 4, dans laquelle les sels de chlorure sont contenus dans la formulation de vaccin contre la dengue en une quantité inférieure à 2,0 mg/dose.

6. Formulation de vaccin contre la dengue selon l'une quelconque des revendications 1 à 5, dans laquelle l'acide chlorhydrique est contenu dans la formulation de vaccin contre la dengue à une quantité de 0,01 à 0,5 mg/dose.

7. Formulation de vaccin contre la dengue selon l'une quelconque des revendications 1 à 6, de préférence sous forme sèche, dans laquelle la formulation de vaccin contre la dengue comprend, sur la base du poids sec de la formulation de vaccin :
a. 65 à 95 % p/p de tréhalose,
b. 0,05 à 5,0 % p/p de poloxamère,
c. 0,1 à 6,0 % p/p d'urée,
d. 0,1 à 5,0 % p/p de chlorhydrate d'arginine,
e. 0,05 à 5,0 % p/p de trométhamine, et
f. 0,05 à 3,0 % p/p de l'albumine sérique humaine.

8. Formulation de vaccin contre la dengue selon l'une quelconque des revendications 1 à 7, dans laquelle une dose de la formulation de vaccin contre la dengue sous forme sèche après reconstitution avec un diluant aqueux pharmaceutiquement acceptable jusqu'à un volume de 0,5 ml comprend :
a. du tréhalose à une concentration de 40 à 80 g/L,
b. du poloxamère à une concentration de 0,1 à 5,0 g/L,
c. de l'urée à une concentration de 0,1 à 6,0 g/L,
d. du chlorhydrate d'arginine à une concentration de 0,5 à 5,0 g/L,
e. de la trométhamine à une concentration de 0,1 à 1,5 g/L, et
f. de l'albumine sérique humaine à une concentration de 0,1 à moins de 1 g/L.

9. Formulation de vaccin contre la dengue selon l'une quelconque des revendications 1 à **8,** dans laquelle une dose de la formulation de vaccin contre la dengue sous forme sèche après reconstitution avec un diluant aqueux pharmaceutiquement acceptable jusqu'à un volume de 0,5 ml comprend des sels de chlorure à une concentration inférieure à 5 g/L.

10. Formulation de vaccin contre la dengue selon l'une quelconque des revendications 1 à 9, dans laquelle une dose de la formulation de vaccin contre la dengue sous forme sèche après reconstitution avec un diluant aqueux pharmaceutiquement acceptable jusqu'à un volume de 0,5 ml présente une osmolalité de 100 à 700 mOsmol/kg.

11. Formulation de vaccin contre la dengue selon l'une quelconque des revendications 1 à 10, dans laquelle une dose de la formulation de vaccin contre la dengue sous forme sèche après reconstitution avec un diluant aqueux pharmaceutiquement acceptable jusqu'à un volume de 0,5 ml comprend :
(i) le virus de la dengue de sérotype 1 en une quantité d'au moins 3,3 log10 pfu/0,5 ml,
(ii) le virus de la dengue de sérotype 2 en une quantité d'au moins 2,7 log10 pfu/0,5 ml,
(iii) le virus de la dengue de sérotype 3 en une quantité d'au moins 4,0 log10 pfu/0,5 ml, et
(iv) le virus de la dengue de sérotype 4 en une quantité d'au moins 4,5 log10 pfu/0,5 ml.

12. Formulation de vaccin contre la dengue selon l'une quelconque des revendications 1 à 11, dans laquelle la composition tétravalente de virus de la dengue comporte au moins un virus de la dengue non chimérique et au moins un virus de la dengue chimérique.

13. Formulation de vaccin contre la dengue selon l'une quelconque des revendications 1 à 12, dans laquelle le virus de la dengue de sérotype 1 est une souche chimérique de dengue de sérotype 2/1, le virus de la dengue de sérotype 2 est une souche non chimérique de dengue de sérotype 2, le virus de la dengue de sérotype 3 est une souche chimérique de dengue de sérotype 2/3 et le virus de la dengue de sérotype 4 est une souche chimérique de dengue de sérotype 2/4, de préférence la dengue de sérotype 1 est TDV-1, la dengue de sérotype 2 est TDV-2, la dengue de sérotype 3 est TDV-3 et la dengue de sérotype 4 est TDV-4.

14. Formulation de vaccin contre la dengue selon l'une quelconque des revendications 1 à 13, dans laquelle la composition tétravalente de virus de la dengue est « TAK-003 ».

15. Formulation de vaccin contre la dengue selon l'une quelconque des revendications 1 à 14, qui est une formulation de vaccin contre la dengue lyophilisée.

16. Récipient, de préférence un flacon, comprenant une ou plusieurs doses de la formulation de vaccin contre la dengue selon l'une quelconque des revendications 1 à 15.

17. Récipient, de préférence un flacon comprenant cinq doses de la formulation de vaccin contre la dengue selon l'une quelconque des revendications 1 à 15.

18. Récipient, de préférence le flacon, selon la revendication 17, dans lequel
a. le tréhalose est contenu à une quantité de 100 à 170 mg,
b. le poloxamère est contenu à une quantité de 2,0 à 8,0 mg,
c. l'urée est contenue à une quantité de 2,0 à 8,0 mg,
d. le chlorhydrate d'arginine est contenu à une quantité de 1,5 à 7,5 mg,
e. la trométhamine est contenue à une quantité de 0,5 à 4,0 mg, et
f. l'albumine sérique humaine est contenue à une quantité de 0,1 à 2,5 mg.

19. Kit comprenant plus d'un, de préférence dix, récipient(s) selon l'une quelconque des revendications 16 à 18, et éventuellement un ou plusieurs, de préférence dix, récipient(s) comprenant un diluant aqueux pharmaceutiquement acceptable pour reconstitution.

20. Kit de préparation d'une formulation de vaccin contre la dengue sous forme reconstituée comprenant :
a) un récipient, de préférence un flacon comprenant la formulation de vaccin contre la dengue selon l'une quelconque des revendications 1 à 15 sous forme sèche, et
b) un récipient, de préférence un flacon, comprenant un diluant aqueux pharmaceutiquement acceptable pour reconstitution.

21. Kit selon la revendication 19 ou 20, dans lequel le diluant aqueux pharmaceutiquement acceptable est choisi dans le groupe constitué par de l'eau pour injection et d'une solution aqueuse de chlorure de sodium, de préférence le diluant aqueux pharmaceutiquement acceptable est une solution aqueuse de chlorure de sodium à 37 mM.

22. Dose d'une formulation de vaccin contre la dengue sous forme aqueuse pouvant être obtenue en reconstituant une à dix, de préférence cinq, dose(s) de la formulation de vaccin contre la dengue selon les revendications 1 à 15 sous forme sèche avec un nombre égal d'aliquotes d'un diluant aqueux pharmaceutiquement acceptable et en retirant le volume d'une dose de la formulation de vaccin contre la dengue reconstituée.

23. Dose selon la revendication 22, dans laquelle l'aliquote a un volume de 0,4 à 0,8 ml/dose, de préférence de 0,4 à 0,7 ml/dose, de manière davantage préférée de 0,45 à 0,65 ml/dose.

24. Procédé de préparation d'une formulation de vaccin contre la dengue selon l'une quelconque des revendications 1 à 15 sous forme sèche comprenant les étapes suivantes :
(a) la fourniture d'une formulation de vaccin contre la dengue sous forme aqueuse comprenant :
A. une composition tétravalente de virus de la dengue comprenant :
(i) un virus de la dengue vivant atténué de sérotype 1,
(ii) un virus de la dengue vivant atténué de sérotype 2,
(iii) un virus de la dengue vivant atténué de sérotype 3, et
(iv) un virus de la dengue vivant atténué de sérotype 4 ; et
B. une composition d'excipients comprenant :
a. du tréhalose,
b. du poloxamère,
c. de l'urée,
d. du chlorhydrate d'arginine,
e. de la trométhamine, et
f. de l'albumine sérique humaine,
(b) la congélation de la formulation de vaccin contre la dengue sous forme aqueuse dans un appareil dont la température de conservation est inférieure à -35 °C pour former une formulation congelée de vaccin contre la dengue,
(c) la soumission de la formulation congelée de vaccin contre la dengue à une étape de séchage primaire dans un appareil dont la température de conservation est comprise entre -31 et -19 °C, par exemple entre -31 et -21 °C, et à une pression inférieure à 0,1 mbar pour former un produit séché primaire, et
(d) la soumission du produit séché primaire à une étape de séchage secondaire dans un appareil avec une température de conservation d'au moins 15 °C, par exemple d'au moins 20 °C, et à une pression inférieure à 1 mbar pour former un produit séché secondaire ;
dans laquelle la formulation de vaccin contre la dengue comprend :
a. du tréhalose à une quantité de 20 à 34 mg/dose,
b. du poloxamère à une quantité de 0,1 à 3,0 mg/dose,
c. de l'urée à une quantité de 0,1 à 3,0 mg/dose,
d. du chlorhydrate d'arginine à une quantité de 0,1 à 2,0 mg/dose,
e. de la trométhamine à une quantité de 0,05 à 1,0 mg/dose, et
f. de l'albumine sérique humaine à une quantité de 0,05 à 0,5 mg/dose.

25. Procédé selon la revendication 24, dans lequel la formulation de vaccin contre la dengue sous forme aqueuse est soumise à l'étape de congélation b) dans un volume qui est inférieur au volume utilisé pour reconstituer la formulation de vaccin contre la dengue lyophilisée, de préférence la formulation de vaccin contre la dengue sous forme aqueuse est soumise à l'étape de congélation b) dans des récipients, tels que des flacons, comprenant chacun une ou plusieurs doses de la formulation de vaccin contre la dengue dans un volume qui est environ la moitié du volume utilisé pour reconstituer les une ou plusieurs doses de la formulation de vaccin contre la dengue lyophilisée.

26. Formulation de vaccin contre la dengue selon l'une quelconque des revendications 1 à 15, destinée à être utilisée dans un procédé de prévention de la dengue chez un sujet, dans lequel le procédé comprend l'administration d'une vaccination primaire avec seulement deux administrations d'une dose comprenant les étapes :
d'administration d'une première dose de la formulation de vaccin contre la dengue chez le sujet, et
d'administration d'une seconde dose de la formulation de vaccin contre la dengue chez le sujet, de préférence dans les 3 mois suivant l'administration de la première dose,
et éventuellement d'administration d'une dose de rappel au moins 12 mois après l'administration de ladite seconde dose de la formulation de vaccin contre la dengue.
